# EUROPÄISCHE PATENTSCHRIFT

(11) **EP 1 481 970 B1**
(45) Veröffentlichungstag und Bekanntmachung des Hinweises auf die Patenterteilung: **22.03.2006**
(21) Anmeldenummer: 04013876.0
(22) Anmeldetag: 05.09.2000
(51) Int. Cl.: C07D 231/32, C07D 231/34, C07D 231/36, C07D 491/04, C07D 417/12, C07D 403/12, C07D 409/12, C07D 413/12, C07D 487/04, C07D 207/38, C07D 491/10, C07D 209/96, C07D 307/60, C07D 307/94, C07D 493/10, C07D 333/32, C07D 333/50, C07D 495/10, C07D 303/32, C07D 311/96, C07C 69/013, C07D 309/38, C07D 309/32, C07D 279/06, C07D 265/02, C07D 237/04, C07D 211/86, C07D 335/02, C07C 323/22, A01N 43/90

(54) **Neue Herbizide**
Novel herbicides
Nouveaux herbicides

(30) Priorität: 07.09.1999 CH 164299
(43) Veröffentlichungstag der Anmeldung: 01.12.2004
(62) Teilanmeldung aus: 00965923.6
(73) Patentinhaber: Syngenta Participations AG, 4058 Basel (CH)
(72) Erfinder: Maetzke, Thomas, 4142 Münchenstein (CH); Stoller, André, 4058 Basel (CH); Wendeborn, Sebastian Volker, 4058 Basel (CH); Szczepanski, Henry, 4323 Wallbach (CH)
(74) Vertreter: Hölscher, Ingo

(56) Entgegenhaltungen:
- EP-A- 0 508 126
- WO-A-00/78712
- WO-A-96/25395

## Beschreibung

Die vorliegende Erfindung betrifft neue, herbizid wirksame durch eine Phenylgruppe substituierte Heterocyclen, Verfahren zu ihrer Herstellung, Mittel, die diese Verbindungen enthalten, sowie ihre Verwendung zum Bekämpfen von Unkräutern, vor allem in Nutzpflanzenkulturen oder zum Hemmen des Pflanzenwachstums.

3-Hydroxy-4-aryl-5-oxo-pyrazolin-Derivate mit herbizider Wirkung sind beispielsweise in EP-A-0 508 126, WO 96/25395 und WO 96/21652 beschrieben.

Es wurden nun neue durch eine Phenylgruppe substituierte Heterocyclen mit herbiziden und wuchshemmenden Eigenschaften gefunden.

Gegenstand der vorliegenden Erfindung sind somit Verbindungen der Formel I 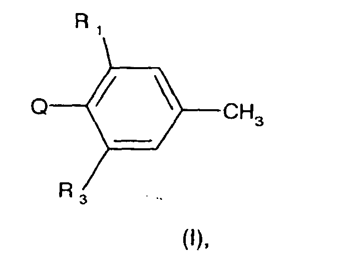 worin
R₁ und R₃ unabhängig voneinander Ethyl, Halogenethyl, Ethinyl, C₁-C₂-Alkoxy, C₁-C₂₋Halogenalkoxy oder C₁-C₂-Alkylcarbonyl bedeuten;
Q eine Gruppe 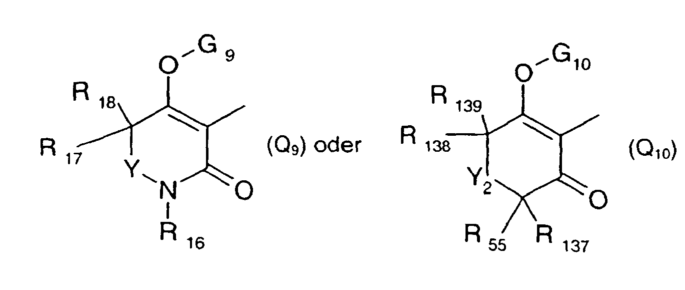 bedeutet;
R₁₆ C₁-C₁₀-Alkyl, C₂-C₁₀-Alkenyl, C₂-C₁₀-Alkinyl, C₁-C₁₀-Halogenalkyl, C₂-C₁₀-Alkoxyalkyl, C₃₋C₁₀-Alkenyloxyalkyl, C₃-C₁₀-Alkinyloxyalkyl, C₂-C₁₀-Alkylthiolkyl, C₂-C₁₀-Alkylsulfinylalkyl, C₂₋C₁₀-Alkylsulfonylalkyl, C₃-C₁₀-Cycloalkyl, Aryl oder Heteroaryl;
R₁₇ C₁-C₁₀-Alkyl, C₂-C₁₀-Alkenyl, C₂-C₁₀-Alkinyl, C₁-C₁₀-Halogenalkyl, C₂-C₁₀-Alkoxyalkyl, C₃₋C₁₀-Alkenyloxyalkyl; C₃-C₁₀-Alkinyloxyalkyl, C₂-C₁₀-Alkylthioalkyl, C₂-C₁₀-Alkylsulfinylalkyl, C₂₋C₁₀-Alkylsulfonylalkyl, C₂-C₁₀-Alkylcarbonyl-alkyl, C₃-C₁₀-Cycloalkyl, Aryl oder Heteroaryl bedeuten;
R₁₈ Wasserstoff, C₂-C₁₀-Alkenyl, C₂-C₁₀-Alkinyl, C₁-C₁₀-Alkyl oder C₁-C₁₀-Alkoxyalkyl bedeutet; oder
R₁₇ und R₁₈ bilden gemeinsam mit den Atomen, an die sie gebunden sind einen 3- bis 7-gliedrigen Cyclus, der ein oder zwei Heteroatome ausgewählt aus Stickstoff, Sauerstoff und Schwefel enthalten kann;
Y Sauerstoff oder C-R₁₉ bedeutet,
R₁₉ C₁-C₁₀-Alkyl, C₂-C₁₀-Alkenyl, C₂-C₁₀-Alkinyl, C₁-C₁₀-Halogenalkyl, Phenyl oder Heteroaryl bedeutet; oder
R₁₈ und R₁₉ bilden gemeinsam mit dem Atom, an das sie gebunden sind, einen gesättigten 5- bis 7- gliedrigen Cyclus, der ein oder zwei Heteroatome ausgewählt aus Stickstoff, Sauerstoff und Schwefel enthalten kann;
G₉ und G₁₀ unabhängig voneinander Wasserstoff, -C(X₁)-R₂₀, -C(X₂)-X₃-R₂₁, -C(X₄)-N(R₂₂)-R₂₃, -SO₂-R₂₄, ein Alkali-, Erdalkali-, Sulfonium- oder Ammoniumkation, -P(X₅)(R₂₅)-R₂₆ oder -CH₂-X₆-R₂₇ bedeuten;
X₁, X₂, X₃, X₄ , X₅ und X₆ unabhängig voneinander Sauerstoff oder Schwefel bedeuten;
R₂₀, R₂₁, R₂₂ und R₂₃ unabhängig voneinander Wasserstoff, C₁-C₁₀-Alkyl, C₂-C₁₀-Alkenyl, C₂-C₁₀-Alkinyl, C₁-C₁₀-Halogenalkyl, C₁-C₁₀-Cyanoalkyl, C₁-C₁₀-Nitroalkyl, C₁-C₁₀-Aminoalkyl, C₁-C₅-Alkylamino-C₁-C₅-alkyl, C₂-C₈-Dialkylamino-C₁-C₅-alkyl, C₃-C₇-Cycloalkyl-C₁-C₅-alkyl, C₂-C₁₀-Alkoxy-alkyl, C₄-C₁₀-Alkenyloxy-alkyl, C₄-C₁₀-Alkinyloxy-alkyl, C₂-C₁₀-Alkylthio-alkyl, C₁-C₅-Alkysulfoxyl-C₁-C₅-alkyl, C₁-C₅-Alkylsulfonyl-C₁-C₅-alkyl, C₂-C₈-Alkylideneamino-oxy-C₁-C₅-alkyl, C₁-C₅-Alkylcarbonyl-C₁-C₅-alkyl, C₁-C₅-Alkoxycarbonyl-C₁-C₅-alkyl, C₁-C₅-Aminocarbonyl-C₁-C₅-alkyl, C₂-C₈-Dialkylamino-carbonyl-C₁-C₅-alkyl, C₁-C₅-Alkylcarbonylamino-C₁₋C₅-alkyl, C₁-C₅-Alkylcarbonyl-(C₂-C₅-alkyl)-aminoalkyl, C₃-C₆-Trialkylsilyl-C₁-C₅-alkyl, Phenyl-C₁-C₅-alkyl, Heteroaryl-C₁-C₅-alkyl, Phenoxy- C₁-C₅-alkyl, Heteroaryloxy-C₁-C₅-alkyl, C₂-C₅₋Alkenyl, C₂-C₅-Halogenalkenyl, C₃-C₈-Cycloalkyl, Phenyl, oder durch C₁-C₃-Alkyl, C₁-C₃₋Halogenalkyl, C₁-C₃-Alkoxy, C₁-C₃-Halogenalkoxy, Halogen, Cyano oder Nitro substituiertes Phenyl, oder Heteroaryl oder Heteroarylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Heteroaryl oder Heteroarylamino, Diheteroarylamino oder durch C₁-C₃-Alkyl, C₁-C₃-Halogenalkyl, C₁₋C₃-Alkoxy, C₁-C₃-Halogenalkoxy, Halogen, Cyano oder Nitro substituiertes Diheteroarylamino, Phenylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenylamino, Diphenylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Diphenylamino, oder C₃-C₇-Cycloalkylamino, Di-C₃-C₇₋cycloalkylamino oder C₃-C₇-Cycloalkoxy bedeuten;
R₂₄, R₂₅ und R₂₆ Wasserstoff, C₁-C₁₀-Alkyl, C₂-C₁₀-Alkenyl, C₂-C₁₀-Alkinyl, C₁-C₁₀₋Halogenalkyl, C₁-C₁₀-Cyanoalkyl, C₁-C₁₀-Nitroalkyl, C₁- C₁₀-Aminoalkyl, C₁-C₅-Alkylamino-C₁₋C₅-alkyl, C₂-C₈-Dialkylamino- C₁-C₅-alkyl, C₃-C₇₋Cycloalkyl-C₁-C₅-alkyl, C₂-C₁₀-Alkoxy-alkyl, C₄-C₁₀-Alkenyloxy-alkyl, C₄-C₁₀-Alkinyloxy-alkyl, C₂-C₁₀-Alkylthio-alkyl, C₁-C₅-Alkysulfoxyl-C₁-C₅-alkyl, C₁-C₅-Alkylsutfonyl-C₁-C₅-alkyl, C₂-C₈-Alkylideneamino-oxy-C₁-C₅-alkyl, C₁-C₅₋Alkylcarbonyl-C₁-C₅-alkyl, C₁-C₅-Alkoxycarbonyl-C₁-C₅-alkyl, C₁-C₅-Amino-carbonyl-C₁-C₅₋alkyl, C₂-C₈-Dialkylamino-carbonyl-C₁-C₅-alkyl, C₁-C₅-Alkylcarbonylamino-C₁-C₅-alkyl, C₁-C₅₋Alkylcarbonyl-(C₂-C₅-alkyl)-aminoalkyl, C₃-C₆-Trialkylsilyl-C₁-C₅-alkyl, Phenyl-C₁-C₅-alkyl, Heteroaryl-C₁-C₅-alkyl, Phenoxy- C₁-C₅-alkyl, Heteroaryloxy- C₁-C₅-alkyl, C₂-C₅-Alkenyl, C₂₋C₅-Halogenalkenyl, C₃-C₈-Cycloalkyl, Phenyl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenyl, oder Heteroaryl oder Heteroarylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Heteroaryl oder Heteroarylamino, Diheteroarylamino, oder durch C₁-C₃-Alkyl, C₁-C₃-Halogenalkyl, C₁-C₃₋Alkoxy, C₁-C₃-Halogenalkoxy, Halogen, Cyano oder Nitro substituiertes Diheteroarylamino, Phenylamino, oder durch C₁-C₃-Alkyl, C₁-C₃-Halogenalkyl, C₁-C₃-Alkoxy, C₁-C₃₋Halogenalkoxy, Halogen, Cyano oder Nitro substituiertes Phenylamino, Diphenylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Diphenylamino, oder C₃-C₇-Cycloalkylamino, Di-C₃-C₇₋cycloalkylamino, C₃-C₇-Cycloalkoxy, C₁-C₁₀-Alkoxy, C₁-C₁₀-Halogenalkoxy, C₁-C₅₋Alkylamino, C₂-C₈-Dialkylamino, Benzyloxy oder Phenoxy, wobei die Benzyl- und Phenylgruppen ihrerseits durch C₁-C₃-Alkyl, C₁-C₃-Halogenalkyl, C₁-C₃-Alkoxy, C₁-C₃₋Halogenalkoxy, Halogen, Cyano oder Nitro substituiert sein können, bedeuten;
R₂₇ C₁-C₁₀-Alkyl, C₂-C₁₀-Alkenyl, C₂-C₁₀-Alkinyl, C₁-C₁₀-Halogenalkyl, C₁-C₁₀-Cyanoalkyl, C₁₋C₁₀-Nitroalkyl, C₁-C₁₀-Aminoalkyl, C₁-C₅-Alkylamino-C₁-C₅-alkyl, C₂-C₈-Dialkylamino-C₁-C₅₋alkyl, C₃-C₇Cycloalkyl-C₁-C₅-alkyl, C₂-C₁₀-Alkoxy-alkyl, C₄-C₁₀-Alkenyloxy-alkyl, C₄-C₁₀₋Alkinyloxy-alkyl, C₂-C₁₀-Alkylthio-alkyl, C₁-C₅-Alkylsulfoxyl- C₁-C₅-alkyl, C₁-C₅-Alkylsulfonyl-C₁-C₅-alkyl, C₂-C₈-Alkylideneamino-oxy-C₁-C₅-alkyl, C₁-C₅-Alkylcarbonyl-C₁-C₅-alky), C₁-C₅₋Alkoxycarbonyl-C₁-C₅-alkyl, C₁-C₅-Amino-carbonyl-C₁-C₅-alkyl, C₂-C₈-Dialkylamino-carbonyl-C₁-C₅-alkyl, C₁-C₅-Alkylcarbonylamino-C₁-C₅-alky), C₁-C₅-Alkylcarbonyl-(C₂-C₅-alkyl)-aminoalkyl, C₃-C₆-Trialkylsilyl-C₁-C₅-alkyl, Phenyl-C₁-C₅-alkyl, Heteroaryl-C₁-C₅-alkyl, Phenoxy-C₁-C₅-alkyl, Heteroaryloxy-C₁-C₅-alkyl, C₂-C₅-Alkenyl, C₂-C₅-Halogenalkenyl, C₃-C₈₋Cycloalkyl, Phenyl, oder durch C₁-C₃-Alkyl, C₁-C₃-Halogenalkyl, C₁-C₃-Alkoxy, C₁-C₃₋Halogenalkoxy, Halogen, Cyano oder Nitro substituiertes Phenyl, oder Heteroaryl, oder Heteroarylamino, oder durch C₁-C₃-Alkyl, C₁-C₃-Halogenalkyl, C₁-C₃-Alkoxy, C₁-C₃₋Halogenalkoxy, Halogen, Cyano oder Nitro substituiertes Heteroaryl oder Heteroarylamino, Diheteroarylamino, durch C₁-C₃-Alkyl, C₁-C₃-Halogenalkyl, C₁-C₃-Alkoxy, C₁-C₃₋Halogenalkoxy, Halogen, Cyano oder Nitro substituiertes Diheteroarylamino, oder Phenylamino, durch C₁-C₃-Alkyl, C₁-C₃-Halogenalkyl, C₁-C₃-Alkoxy, C₁-C₃-Halogenalkoxy, Halogen, Cyano oder Nitro substituiertes Phenylamino, Diphenylamino, durch C₁-C₃-Alkyl, C₁-C₃-Halogenalkyl, C₁-C₃-Alkoxy, C₁-C₃-Halogenalkoxy, Halogen, Cyano oder Nitro substituiertes Diphenylamino, C₃-C₇-Cycloalkylamino, Di-C₃-C₇-cycloalkylamino, C₃-C₇₋Cycloalkoxy oder C₁-C₁₀-Alkylcarbonyl bedeutet;
Y₂ Sauerstoff oder Schwefel bedeutet,
R₅₅ C₁-C₁₀-Alkyl, C₂-C₁₀-Alkenyl, C₂-C₁₀-Alkinyl, C₁-C₁₀-Halogenalkyl, C₂-C₁₀-Alkoxyalkyl, C₃₋C₁₀-Alkenyloxyalkyl, C₃-C₁₀-Alkinyloxyalkyl, C₂-C₁₀-Alkylthioalkyl, C₂-C₁₀-Alkylsulfinylalkyl, C₂₋C₁₀-Alkylsulfonylalkyl, C₂-C₁₀-Alkylcarbonyl-alkyl, C₃-C₁₀-Cycloalkyl, Aryl oder Heteroaryl bedeutet;
R₁₃₇ Wasserstoff, C₁-C₁₀-Alkyl C₂-C₁₀-Alkenyl, C₂-C₁₀-Alkinyl, oder C₁-C₁₀-Alkoxyalkyl bedeutet; oder
R₅₅ und R₁₃₇ bilden gemeinsam mit den Atomen, an die sie gebunden sind, einen 3- bis 7-gliedrigen Cyclus, der ein oder zwei Heteroatome ausgewählt aus Stickstoff, Sauerstoff und Schwefel enthalten kann; und
R₁₃₈ und R₁₃₉ unabhängig voneinander Wasserstoff, C₁-C₁₀-Alkyl, C₂-C₁₀-Alkenyl, C₂-C₁₀₋Alkinyl, C₂-C₁₀)-Alkoxyalkyl sind;
sowie die Verbindung Nr. 21.115,
sowie agronomisch verträgliche Salze, Isomere und Enantiomere dieser Verbindungen.

Die in den Substituentendefinitionen vorkommenden Alkylgruppen können geradkettig oder verzweigt sein und stehen beispielsweise für Methyl, Ethyl, n-Propyl, iso-Propyl, n-Butyl, sek.-Butyl, iso-Butyl, tert.-Butyl sowie die Isomeren Pentyle, Hexyle, Heptyle, Octyle, Nonyle und Decyle. Halogenalkyl ist beispielsweise Fluormethyl, Difluormethyl, Trifluormethyl, Chlormethyl, Dichlormethyl, Trichlormethyl, 2,2,2-Trifluorethyl, 2-Fluorethyl, 2-Chlorethyl, Pentafluorethyl, 1,1-Difluor-2,2,2-trichlorethyl, 2,2,3,3-Tetrafluorethyl und 2,2,2-Trichlorethyl; vorzugsweise Trichlormethyl, Difluorchlormethyl, Difluormethyl, Trifluormethyl und Dichlorfluormethyl. Alkoxyalkyl ist beispielsweise Methoxymethyl, Ethoxymethyl, Propoxyethyl, i-Propoxyethyl, n-Butoxymethyl, iso-Butoxyn-butyl, sek.-Butoxymethyl und tert.-Butoxy-i-propyl, vorzugsweise Methoxymethyl und Ethoxymethyl. Alkoxy-, Alkenyl-, Alkinyl-, Alkoxyalkyl-, Alkylthio-, Alkylsulfonyl-, Alkylaminocarbonyl-, Dialkylaminocarbonyl-Alkylaminoalkyl, Phenylalkyl-, Nitroalkyl-, Aminoalkyl- und N-Alkoxycarbonyl-N-alkylaminoalkylgruppen leiten sich von den genannten Alkylresten ab. Die Alkenyl- und Alkinylgruppen können ein- oder mehrfach ungesättigt sein. Unter Alkenyl ist beispielsweise Vinyl, Allyl, Methallyl, 1-Methylvinyl oder But-2-en-1-yl zu verstehen. Alkinyl bedeutet beispielsweise Ethinyl, Propargyl, But-2-in-1-yl, 2-Methylbutin-2-yl oder But-3-in-2-yl. Alkinyl bedeutet beispielsweise Ethinyl, Propargyl, But-2-in-1-yl, 2-Methylbutin-2-yl oder But-3-in-2-yl. Halogenalkylgruppen haben vorzugsweise eine Kettenlänge von 1 bis 4 Kohlenstoffatomen. Halogenalkyl ist beispielsweise Fluormethyl, Difluormethyl, Trifluormethyl, Chlormethyl, Dichlormethyl, Trichlormethyl, 2,2,2-Trifluorethyl, 2-Fluorethyl, 2-Chlorethyl,Pentafluorethyl, 1,1-Difluor-2,2,2-trichlorethyl, 2,2,3,3-Tetrafluorethyl und 2,2,2-Trichlorethyl; vorzugsweise Trichlormethyl, Difluorchlormethyl, Difluormethyl, Trifluormethyl und Dichlorfluormethyl. Als Halogenalkenyl kommen ein- oder mehrfach durch Halogen substituierte Alkenylgruppen in Betracht, wobei Halogen Fluor, Chlor, Brom und Jod und insbesondere Fluor und Chlor bedeutet, beispielsweise 2,2-Difluor-1-methylvinyl, 3-Fluorpropenyl, 3-Chlorpropenyl, 3-Brompropenyl, 2,3,3-Trifluorpropenyl, 2,3,3-Trichlorpropenyl und 4,4,4-Trifluor-but-2-en-1-yl. Unter den durch Halogen 1-, 2- oder 3-fach substituierten C₂-C₆-Alkenylgruppen sind diejenigen bevorzugt, die eine Kettenlänge von 3 bis 5 Kohlenstoffatomen besitzen. Alkoxygruppen haben vorzugsweise eine Kettenlänge von 1 bis 6 Kohlenstoffatomen. Alkoxy ist beispielsweise Methoxy, Ethoxy, Propoxy, i-Propoxy, n-Butoxy, iso-Butoxy, sek.-Butoxy und tert.-Butoxy sowie die Isomeren Pentyloxy und Hexyloxy; vorzugsweise Methoxy und Ethoxy. Alkylcarbonyl steht vorzugsweise für Acetyl oder Propionyl. Alkoxycarbonyl bedeutet beispielsweise Methoxycarbonyl, Ethoxycarbonyl, Propoxycarbonyl, iso-Propoxycarbonyl, n-Butoxycarbonyl, iso-Butoxycarbonyl, sek.-Butoxycarbonyl oder tert.-Butoxycarbonyl; vorzugsweise Methoxycarbonyl oder Ethoxycarbonyl. Alkylthiogruppen haben vorzugsweise eine Kettenlänge von 1 bis 4 Kohlenstoffatomen. Alkylthio ist beispielsweise Methylthio, Ethylthio, Propylthio, iso-Propylthio, n-Butylthio, iso-Butylthio, sek.-Butylthio oder tert.-Butylthio, vorzugsweise Methylthio und Ethylthio. Alkylsulfinyl ist beispielsweise Methylsulfinyl, Ethylsulfinyl, Propylsulfinyl, iso-Propylsulfinyl, n-Butylsulfinyl, iso-Butylsulfinyl, sek.-Butylsulfinyl, tert.-Butylsulfinyl; vorzugsweise Methylsulfinyl und Ethylsulfinyl. Alkylsulfonyl steht beispielsweise für Methylsulfonyl, Ethylsulfonyl, Propylsulfonyl, iso-Propylsulfonyl, n-Butylsulfonyl, iso-Butylsulfonyl, sek.-Butylsulfonyl oder tert.-Butylsulfonyl; vorzugsweise für Methylsulfonyl oder Ethylsulfonyl. Alkylamino ist beispielsweise Methylamino, Ethylamino, n-Propylamino, isoPropylamino oder die isomeren Butylamine. Dialkylamino steht beispielsweise für Dimethylamino, Methylethylamino, Diethylamino,
n-Propylmethylamino, Di-butylamino und Di-Isopropylamino. Alkoxyalkylgruppen haben vorzugsweise 1 bis 6 Kohlenstoffatome. Alkoxyalkyl bedeutet beispielsweise Methoxymethyl, Methoxyethyl, Ethoxymethyl, Ethoxyethyl, n-Propoxymethyl, n-Propoxyethyl, iso-Propoxymethyl oder iso-Propoxyethyl. Alkylthioalkyl bedeutet beispielsweise Methylthiomethyl, Methylthioethyl, Ethylthiomethyl, Ethylthioethyl, n-Propylthiomethyl, n-Propylthioethyl, iso-Propylthiomethyl, iso-Propylthioethyl, Butylthiomethyl, Butylthioethyl oder Butylthiobutyl. Phenyl, kann substituiert vorliegen. Die Substituenten können dann in ortho-, meta- und/oder para-Stellung stehen. Bevorzugte Substituentenstellungen sind die ortho- und para-Positionen zur Ringverknüpfungsstelle.

Aryl steht beispielsweise für Phenyl oder Naphtyl. Diese Gruppen können auch substituiert sein. Phenyl, auch als Teil eines Substitenten wie Phenylalkyl, kann beispielsweise- wenn in den Definitionen nicht anders angegeben, durch Halogen, Nitro, Cyano, C₁-C₄-Alkyl, C₁-C₄₋Alkoxy, C₁-C₄-Alkylthio, C₁-C₄-Alkylsulfoxy, C₁-C₄-Alkylsulfonyl, Carboxyl, C₁-C₄₋Alkoxycarbonyl, Amino, C₁-C₄-Alkylamino, C₁-C₄-Dialkylamino oder C₁-C₄₋Alkylcarbonylamino substituiert sein.

Heteroarylgruppen sind üblicherweise aromatische Heterocyclen, die vorzugsweise 1 bis 3 Heteroatome wie Stickstoff, Schwefel und Sauerstoff enthalten. Beispiele für geeignete Heterocyclen und Heteroaromaten sind: Pyrrolidin, Piperidin, Pyran, Dioxan, Azetidin, Oxetan, Pyridin, Pyrimidin, Triazin, Thiazol, Thiadiazol, Imidazol, Oxazol, Isoxazol sowie Pyrazin, Furan, Morpholin, Piperazin, Pyrazol, Benzoxazol, Benzthiazol, Chinoxalin und Chinolin. Diese Heterocyclen und Heteroaromaten können weiter substituiert sein, beispielsweise mit Halogen, Alkyl, Alkoxy, Haloalkyl, Haloalkoxy, Nitro, Cyano, Thioalkyl, Alkylamino oder Phenyl.

Unter den 3- bis 7- gliedrigen Cyclen sind im Rahmen der vorliegenden Erfindung Ringsysteme zu verstehen, die zusätzlich zu den gegebenenfalls im Ring der Substituenten Q bereits vorhandenen Heteroatomen neben den Kohlenstoffatomen ein oder mehrere Heteroatome wie Stickstoff, Sauerstoff und/oder Schwefel enthalten können. Sie können gesättigt oder ungesättigt sein. Die ungesättigte Bindung kann beispielsweise bei der Gruppe Q₂ durch die Substituenten R₆ und R₇ gebildet werden. Bevorzugt enthalten solche Ringsysteme 5 bis 7 Ringatome.
3- bis 7- gliedrige Cyclen einschließlich die Cycloalkyle wie z.B. Cyclopropyl, Cyclobutyl, Cyclopentyl, Cyclohexyl, Cycloheptyl oder Cyclooctyl können auch substituiert sein. Geeignete Substituenten sind Halogen, Hydroxy, Nitro, Cyano, C₁-C₄-Alkylcarbonyl, C₁-C₄₋Alkoxycarbonyl, C₁-C₄-Alkyl, C₁-C₄-Halogenalkyl, Keto, C₂-C₄-Alkenyloxyimino, C₁-C₄-Alkoxy, C₁-C₄-Alkoxyalkoxy, C₁-C₄-Alkylthio, oder eine der folgenden 3 Gruppen 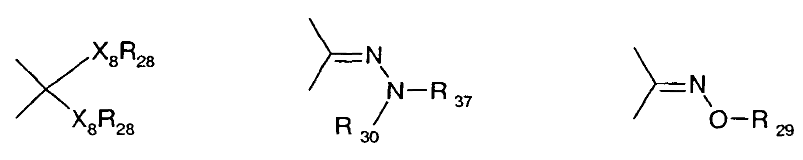 worin X₈ Schwefel oder Sauerstoff bedeutet, R₂₈ C₁-C₄-Alkoxyl oder beide R₂₈ bilden mit der -X₈-C-X₈-Brücke, an die sie gebunden sind, einen 5- oder 6-gliedrigen Ring, der mit Methyl, Ethyl, Methoxy oder einer Ketogruppe substituiert sein kann,
R₂₉ C₁-C₄-Alkyl, C₁-C₄-Halogenalkyl, C₂-C₄-Alkenyl oder C₂-C₄-Halogenalkenyl bedeutet,
R₃₀ und R₃₇ unabhängig voneinander C₁-C₄-Alkyl, Phenyl, C₂-C₄-Alkenyl, oder R₃₀ und R₃₇ bilden zusammen mit dem Stickstoffatom, an das sie gebunden sind, einen 5- bis 6 gliedrigen Ring, der ein Heteroatom ausgewählt aus Stickstoff, Sauerstoff und Schwefel enthalten kann.

ln den Substituentendefinitionen bedeutet die Zahl der Kohlenstoffatome die Gesamtzahl der Kohlenstoffatome in den Alkyl-, Alkenyl- und Alkinylgruppen sowie den davon abgeleiteten Gruppen wie z.B. Halogenalkyl oder Alkenyloxy. C₂-C₃-Alkoxyalkyl umfaßt daher Methoxymethyl, Metoxyethyl und Ethoxymethyl. C₃-Alkoxycarbonylalkyl umfaßt Methoxycarbonylethyl und Ethoxycarbonylmethyl.

Alkali-, Erdalkali- oder Ammoniumkationen für die Substituenten G₉ und G₁₀ sind beispielsweise die Kationen von Natrium, Kalium, Magnesium, Kalzium und Ammonium. Bevorzugte Sulfoniumkationen sind insbesondere Trialkylsulfoniumkationen, worin die Alkylgruppen vorzugsweise je 1 bis 4 Kohlenstoffatome enthalten.

Die Verbindungen der Formel I können, auch in Abhängigkeit von der Art der Substituenten, als geometrische, und/oder optische Isomere und Isomerengemische sowie als Tautomere und Tautomerengemische vorliegen. Diese Verbindungen der Formel I bilden ebenfalls einen Gegenstand der vorliegenden Erfindung.

Die Erfindung umfaßt ebenfalls die Salze, die die Verbindungen der Formel 1 vorzugsweise mit Aminen, Alkali- und Erdalkalimetallbasen oder quaternären Ammoniumbasen bilden können. Geeignete Salzbildner sind beispielsweise in WO 98/41089 beschrieben.

Die Erfindung umfaßt ebenfalls die Salze, die die Verbindungen der Formel I mit Aminen, Alkali- und Erdalkalimetallbasen oder quaternären Ammoniumbasen bilden können. Unter den Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Als Beispiele für zur Ammoniumsalzbildung geeignete Amine kommen sowohl Ammoniak wie auch primäre, sekundäre und tertiäre C₁-C₁₈-Alkylamine, C₁-C₄-Hydroxyalkylamine und C₂-C₄-Alkoxyalkylamine in Betracht, beispielsweise Methylamin, Ethylamin, n-Propylamin, iso-Propylamin, die vier isomeren Butylamine, n-Amylamin, iso-Amylamin, Hexylamin, Heptylamin, Octylamin, Nonylamin, Decylamin, Pentadecylamin, Hexadecylamin, Heptadecylamin, Octadecylamin, Methyl-ethylamin, Methyl-iso-propylamin, Methylhexylamin, Methyl-nonylamin, Methyl-pentadecylamin, Methyl-octadecylamin, Ethylbutylamin, Ethyl-heptylamin, Ethyl-octylamin, Hexyl-heptylamin, Hexyl-octylamin, Dimethylamin, Diethylamin, Di-n-propylamin, Di-iso-propylamin, Di-n-butylamin, Di-n-amylamin, Di-iso-amylamin, Dihexylamin, Diheptylamin, Dioctylamin, Ethanolamin, n-Propanolamin, iso-Propanolamin, N,N-Diethanolamin, N-Ethylpropanolamin, N-Butylethanolamin, Allylamin, n-Butenyl-2-amin, n-Pentenyl-2-amin, 2,3-Dimethylbutenyl-2-amin, Di-butenyl-2-amin, n-Hexenyl-2-amin, Propylendiamin, Trimethylamin, Triethylamin, Tri-n-propylamin, Tri-iso-propylamin, Tri-n-butylamin, Tri-iso-butylamin, Tri-sek.-butylamin, Tri-n-amylamin, Methoxyethylamin und Ethoxyethylamin; heterocyclische Amine wie z.B. Pyridin, Chinolin, iso-Chinolin, Morpholin, Piperidin, Pyrrolidin, Indolin, Chinuclidin und Azepin; primäre Arylamine wie z.B. Aniline, Methoxyaniline, Ethoxyaniline, o,m,p-Toluidine, Phenylendiamine, Benzidine, Naphthylamine und o,m,p-Chloraniline; insbesondere aber Triethylamin, iso-Propylamin und Di-iso-propylamin.

Bevorzugte quarternäre Ammoniumbasen, die zur Salzbildung geeignet sind, entsprechen z.B. der Formel [N(Rₐ R_{b}R_{c}R_{d} )]OH, worin Rₐ, R_{b}, R_{c} und R_{d} unabhängig voneinander C₁-C₄ Alkyl bedeuten. Andere geeignete Tetraalkylammoniumbasen mit anderen Anionen können beispielsweise durch Anionenaustauschreaktionen erhalten werden.

Ferner sind diejenigen Verbindungen der Formel I bevorzugt, worin
R₁₆ C₁-C₆-Alkyl, C₁-C₆-Halogenalkyl, C₂-C₆-Alkoxyalkyl, C₄-C₆-Alkenyloxyalkyl, C₄-C₆₋Alkinyloxyalkyl, C₂-C₆-Alkylthiolkyl, C₂-C₆-Alkylsulfinylalkyl, C₂-C₆- Alkylsulfonylalkyl, C₃-C₈-Cyclolalkyl, Aryl oder Heteroaryl;
R₁₇ C₁-C₆-Alkyl, C₁-C₆-Halogenalkyl, C₂-C₆-Alkoxyalkyl, C₄-C₆-Alkenyloxyalkyl, C₄-C₆₋Alkinyloxyalkyl, C₂-C₆-Alkylthialkyl, C₂-C₆-Alkylsulfinylalkyl, C₂-C₆-Alkylsulfonylalkyl, C₃-C₆₋Alkylcarbonyl-alkyl, C₃-C₈-Cyclolalkyl, Aryl oder Heteroaryl bedeuten;
R₁₈ Wasserstoff, C₁-C₆-Alkyl oder C₂-C₆-Alkoxalkyl; oder
R₁₇ und R₁₈ bilden gemeinsam mit den Atomen, an die sie gebunden sind, einen 3- bis 7-gliedrigen Cyclus, der ein oder zwei Heteroatome ausgewählt aus Stickstoff, Sauerstoff und Schwefel enthalten kann,
R₁₉ C₁-C₆-Alkyl, C₁-C₆-Halogenalkyl, Phenyl, oder Heteroaryl bedeutet; oder
R₁₈ und R₁₉ bilden gemeinsam mit dem Atom, an das sie gebunden sind, einen gesättigten 5- bis 7- gliedrigen Cyclus, der ein oder zwei Heteroatome ausgewählt aus Stickstoff, Sauerstoff und Schwefel enthalten kann;
R₂₀, R₂₁, R₂₂, R₂₃, und R₂₇ unabhängig voneinander Wasserstoff,
C₁-C₈-Alkyl, C₁-C₈-Halogenalkyl, C₁-C₈-Cyanoalkyl, C₁-C₈-Nitroalkyl, C₁-C₈-Aminoalkyl, C₁₋C₅-Alkylamino-C₁-C₂-alkyl, C₂-C₆-Dialkylamino-C₁-C₂-alkyl, C₃-C₇-Cycloalkyl-C₁-C₂-alkyl, C₂₋C₈-Alkoxy-alkyl, C₄- C₈-Alkenyloxy-alkyl, C₄-C₈-Alkinyloxy-alkyl, C₂-C₈-Alkylthio-alkyl, C₁-C₂₋Alkysulfoxyl-C₁C₂-alkyl, C₁-C₂-Alkylsulfonyl-C₁-C₂-alkyl, C₂-C₈-Alkylideneamino-oxy-C₁-C₂₋alkyl, C₁-C₅-Alkylcarbonyl-C₁-C₂-alkyl, C₁-C₅-Alkoxycarbonyl-C₁-C₂-alkyl, C₁-C₅-Aminocarbonyl-C₁-C₂-alkyl, C₂-C₈-Dialkylamino-carbonyl-C₁-C₂-alkyl, C₁-C₅-Alkylcarbonylamino-C₁₋C₂-alkyl, C₁-C₂-Alkylcarbonyl-N-C₁-C₃-alkyl-C₁-C₂-aminoalkyl, C₃-C₆-Trialkylsilyl-C₁-C₃-alkyl, Phenyl-C₁-C₂-alkyl, Heteroaryl-C₁-C₂-alkyl, Phenoxy-C₁-C₂-alkyl, Heteroaryloxy-C₁-C₂-alkyl, C₂-C₅-Alkenyl, C₂-C₅-Halogenalkenyl, C₃-C₈-Cycloalkyl, Phenyl oder Heteroaryl bedeuten;
R₂₄, R₂₅ und R₂₆ unabhängig voneinander Wasserstoff, C₁-C₈-Alkyl, C₁-C₈-Halogenalkyl, C₁₋C₈-Cyanoalkyl, C₁- C₈-Nitroalkyl, C₁- C₈-Aminoalkyl, C₁-C₅-Alkylamino-C₁-C₂-alkyl, C₂-C₆₋Dialkylamino-C₁-C₂-alkyl, C₃-C₇-Cycloalkyl-C₁-C₂-alkyl, C₂-C₈-Alkoxy-alkyl, C₄- C₈-Alkenyloxyalkyl, C₄-C₈-Alkinyloxy-alkyl, C₂-C₈-Alkylthio-alkyl, C₁-C₂-Alkysulfoxyl-C₁-C₂-alkyl, C₁-C₂₋Alkylsulfonyl-C₁-C₂-alkyl, C₂-C₈-Alkylideneamino-oxy-C₁-C₂-alkyl, C₁-C₅-Alkylcarbonyl-C₁-C₂₋alkyl, C₁-C₅-Alkoxycarbonyl-C₁-C₂-alkyl, C₁-C₅-Amino-carbonyl-C₁-C₂-alkyl, C₂-C₈₋Dialkylamino-carbonyl-C₁-C₂-alkyl, C₁-C₅-Alkylcarbonylamino-C₁-C₂-alkyl, C₁-C₂₋Alkylcarbonyl-N-C₁-C₃-alkyl-C₁-C₂-aminoalkyl, C₃-C₆-Trialkylsilyl-C₁-C₃-alkyl, Phenyl-C₁-C₂₋alkyl, Heteroaryl-C₁-C₂-alkyl, Phenoxy-C₁-C₂-alkyl, Heteroaryloxy-C₁-C₂-alkyl,
C₂-C₅-Alkenyl, C₂-C₅-Halogenalkenyl, C₃-C₈-Cycloalkyl, Phenyl, Heteroaryl, C₁-C₆-Alkoxy, C₁-C₆-Halogenalkoxy, C₁-C₃-Alkylamino, C₂-C₆-Dialkylamino sowie Benzyloxy oder Phenoxy, wobei die Benzyl- und Phenylgruppen ihrerseits durch C₁-C₃-Alkyl, C₁-C₃-Halogenalkyl, C₁₋C₃-Alkoxy, C₁-C₃-Halogenalkoxy, Halogen, Cyano oder Nitro substituiert sein können, bedeuten; und
R₂₇ C₁-C₈-Alkyl, C₁-C₈-Halogenalkyl, C₁- C₈-Cyanoalkyl, C₁-C₈-Nitroalkyl, C₁-C₈-Aminoalkyl, C₁-C₅-Alkylamino-C₁-C₂-alkyl, C₂-C₆-Dialkylamino-C₁-C₂-alkyl, C₃-C₇-Cycloalkyl-C₁-C₂-alkyl, C₂-C₈-Alkoxy-alkyl, C₄-C₈-Alkenyloxy-alkyl, C₄-C₈-Alkinyloxy-alkyl, C₂-C₈-Alkylthio-alkyl, C₁₋C₂-Alkysulfoxyl-C₁-C₂-alkyl, C₁-C₂-Alkylsulfonyl-C₁-C₂-alkyl, C₂-C₈-Alkylideneamino-oxy-C₁₋C₂-alkyl, C₁-C₅-Alkylcarbonyl-C₁-C₂-alkyl, C₁-C₅-Alkoxycarbonyl-C₁-C₂-alkyl, C₁-C₅-Aminocarbonyl-C₁-C₂-alkyl, C₂-C₈-Dialkylamino-carbonyl-C₁-C₂-alkyl, C₁-C₅-Alkylcarbonylamino-C₁₋C₂-alkyl, C₁-C₂-Alkylcarbonyl-N-C₁-C₃-alkyl-C₁-C₂-aminoalkyl, C₃-C₆-Trialkylsilyl-C₁-C₃-alkyl, Phenyl-C₁-C₂-alkyl, Heteroaryl-C₁-C₂-alkyl, Phenoxy-C₁-C₂-alkyl, Heteroaryloxy-C₁-C₂-alkyl, C₂-C₅-Alkenyl, C₂-C₅-Halogenalkenyl, C₃-C₈-Cycloalkyl, Phenyl, Heteroaryl, C₁-C₆-Alkoxy, C₁-C₆-Halogenalkoxy, C₁-C₈-Alkylcarbonyl, C₁-C₃-Alkylamino, C₂-C₆-Dialkylamino sowie Benzyloxy oder Phenoxy, wobei die Benzyl- und Phenylgruppen ihrerseits durch C₁-C₃-Alkyl, C₁-C₃-Halogenalkyl, C₁-C₃-Alkoxy, C₁-C₃-Halogenalkoxy, Halogen, Cyano oder Nitro substituiert sein können, bedeutet.

Besonders bevorzugte Verbindungen der Formel I sind dadurch gekennzeichnet, daß
R₁ und R₃ unabhängig voneinander Ethyl, Halogenethyl, Ethinyl, C₁-C₂-Alkoxy, C₁-C₂₋Halogenalkoxy oder C₁-C₂-Alkylcarbonyl bedeuten;
R₁₆ C₁-C₆-Alkyl, C₁-C₆-Halogenalkyl, C₂-C₆-Alkoxyalkyl, C₃-C₈-Cyclolalkyl, Aryl oder Heteroaryl bedeutet;
R₁₇ C₁-C₆-Alkyl, C₁-C₆-Halogenalkyl, C₃-C₈-Cyclolalkyl, Aryl oder Heteroaryl;
R₁₈ Wasserstoff, C₁-C₆-Alkyl oder C₂-C₆-Alkoxalkyl bedeutet; oder
R₁₇und R₁₈ bilden gemeinsam mit den Atomen, an die sie gebunden sind, einen 3- bis 7-gliedrigen Cyclus, der ein oder zwei Heteroatome ausgewählt aus Stickstoff, Sauerstoff und Schwefel enthalten kann,
R₁₉ C₁-C₆-Alkyl oder C₁-C₆-Halogenalkyl bedeutet; oder
R₁₈ und R₁₉ bilden gemeinsam mit dem Atom, an das sie gebunden sind, einen gesättigten 5- bis 7- gliedrigen Cyclus, der ein oder zwei Heteroatome ausgewählt aus Stickstoff, Sauerstoff und Schwefel enthalten kann;
R₂₀, R₂₁ , R₂₂ und R₂₃ unabhängig voneinander Wasserstoff, C₁-C₈-Alkyl, C₁-C₈-Halogenalkyl, C₃-C₇-Cycloalkyl-C₁-C₂-alkyl, C₂-C₈-Alkoxy-alkyl, Phenyl-C₁-C₂-alkyl, Heteroaryl-C₁-C₂-alkyl, Phenoxy-C₁-C₂-alkyl, Heteroaryloxy-C₁-C₂-alkyl, C₂-C₅-Alkenyl, C₂-C₅-Halogenalkenyl, C₃-C₈₋Cycloalkyl, Phenyl oder Heteroaryl bedeuten;
R₂₄, R₂₅ und R₂₆ unabhängig voneinander Wasserstoff, C₁-C₈-Alkyl, C₁-C₈-Halogenalkyl, C₃₋C₇-Cycloalkyl-C₁-C₂-alkyl, C₂-C₈-Alkoxy-alkyl, Phenyl-C₁-C₂-alkyl, Heteroaryl-C₁-C₂-alkyl, Phenoxy-C₁-C₂-alkyl, Heteroaryloxy-C₁-C₂-alkyl, C₂-C₅-Alkenyl, C₂-C₅-Halogenalkenyl, C₃-C₈₋Cycloalkyl, Phenyl, Heteroaryl, C₁-C₆-Alkoxy, C₁-C₃-Alkylamino oder C₂-C₆-Dialkylamino bedeuten; und
R₂₇ C₁-C₈-Alkyl, C₁-C₈-Halogenalkyl, C₃-C₇-Cycloalkyl-C₁-C₂-alkyl, C₂-C₈-Alkoxy-alkyl, Phenyl-C₁-C₂-alkyl, Heteroaryl-C₁-C₂-alkyl, Phenoxy-C₁-C₂-alkyl, Heteroaryloxy-C₁-C₂-alkyl, C₂-C₅-Alkenyl, C₂-C₅-Halogenalkenyl, C₃-C₈-Cycloalkyl, Phenyl, Heteroaryl, C₁-C₆-Alkoxy, C₁₋C₃-Alkylamino, C₂-C₆-Dialkylamino oder C₁-C₈-Alkylcarbonyl bedeuten.

Die Verbindungen der Formel I können hergestellt werden, indem man eine Verbindung der Formel XXX

Q-H (XXX)

worin Q für Q₉ oder Q₁₀ steht, wobei deren Substituenten mit Ausnahme von G₉ und G₁₀ die oben angegebene Bedeutung haben und G₉ und G₁₀ Wasserstoff bedeutet, mit einer Verbindung der Formel XXXI 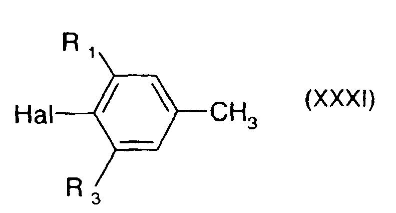 worin R₁ und R₃ die unter Formel I angegebene Bedeutung haben und Hal für Chlor, Brom oder Jod steht, in Gegenwart eines inerten Lösungsmittels, einer Base und eines Palladiumkatalysators bei Temperaturen von 30 bis 250 °C umsetzt. Die Reaktion wird vorzugsweise unter Inertgasatmosphäre durchgeführt.

Es hat sich überraschenderweise gezeigt, dass die Herstellung von Verbindungen der Formel I, worin R₁ und R₃ für Ethyl stehen, mit diesem Verfahren ganz besonders vorteilhaft ist.

Die Verbindungen der Formel XXX sind bekannt oder nach bekannten Verfahren, wie beispielsweise in J. Chem. Soc. Perkin Trans. 1 (1987), (4), 877-884 beschrieben, herstellbar. Die Verbindungen der Formel XXXI können beispielsweise nach bekannten Methoden wie z.B. Sandmeyer-Reaktion aus den entsprechenden Anilinen der Formel XXXII 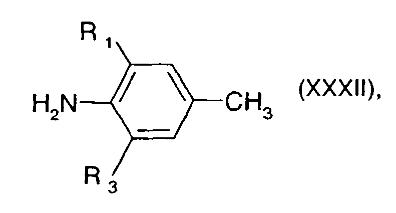 worin R₁ und R₃ die unter Formel I angegebenen Bedeutungen haben, über die Diazoniumsalze hergestellt werden. Derartige Reaktionen sind beispielsweise in Vogel's Textbook of Practical Organic Chemistry, 5*th* Edition, B.S. Furniss, A.J. Hannaford, P.W.G. Smith, A.R. Tatchell; Longman Scientific & Technical 1989, Seite 923 beschrieben. Die Verbindungen der Formel XXXII sind bekannt, teilweise kommerziell erhältlich oder können analog zu bekannten hergestellt werden.

Geeignet für diese Reaktion sind Basen, wie Tri-Alkalimetallphosphate, Alkali- und Erdalkalimetallhydride, Alkali- und Erdalkalimetallamide oder Alkalimetallalkoholate, beispielsweise Tri-Kaliumphosphat, Natriumhydrid, Lithiumdiisopropylamid (LDA), Na-tert.-Butylat oder K-tert.Butylat. Besonders bevorzugt sind Na-tert.-Butylat, K-tert.Butylat sowie Trikaliumphosphat.

Geeignete Lösungsmittel sind beispielsweise aromatische Kohlenwasserstoffe wie z.B. Xylol oder Toluol, Ether wie Tetrahydrofuran, Dioxan oder Ethylenglykoldimethylether, Dimethylsulfoxid oder tertiäre Amide wie Dimethylformamid , N-Methylpyrrolidinon oder Dimethylacetamid oder acyclische Harnstoffe wie N,N'-Dimethylpropylenharnstoff.

Die für die C-C-Verknüpfungsreaktion einer Verbindung der Formel XXX mit einer Verbindung der Formel XXXI in Betracht kommenden Palladiumkatalysatoren sind generell Palladium(II)- oder Palladium(0)-Komplexe wie z.B. Palladium(II)-dihalogenide, Palladium(II)-acetat, Palladium(II)-sulfat, Bis-(triphenylphosphin)-palladium(II)-dichlorid, Bis-(tricyclopentylphosphin)-palladium(II)-dichlorid, Bis-(tricyclohexylphosphin)-palladium(II)-dichlorid, Bis-(dibenzylidenaceton)-palladium(0) oder Tetrakis-(triphenylphosphin)-palladium(0). Der Palladiumkatalysator kann auch aus Palladium(II)- oder Palladium(0)-Verbindungen durch Komplexierung mit den gewünschten Liganden *'in situ'* hergestellt werden, indem z.B. das zu komplexierende Palladium(II)-Salz beispielsweise Palladium(II)-dichlorid (PdCl₂) oder Palladium(II)-acetat (Pd(OAc)₂) zusammen mit dem gewünschten Liganden beispielsweise Triphenylphosphin (PPh₃), tricyclopentylphosphin oder Tricyclohexylphosphin zusammen mit dem gewählten Lösungsmittel, eine Verbindung der Formel XXXI, eine Verbindung der Formel XXX und Base vorgelegt wird. Bidendate Liganden kommen auch in Frage, wie zum Beispiel 1,1'-Bis-(Diphenylphosphino)ferrocen oder 1,2-Bis-(Diphenylphosphino)ethan. Durch Erwärmung des Reaktionsmediums bildet sich dann 'in situ' der für die C-C-Kopplungsreaktion gewünschte Palladium(II)- bzw. Palladium(0)-Komplex, welcher dann die C-C-Kopplungsreaktion startet. Die Palladiumkatalysatoren werden in einer Menge von 0,001 - 50 Mol%, bevorzugt in einer Menge von 0,1 - 15 Mol% bezogen auf die Verbindung der Formel XXXI eingesetzt.

Die Reaktionstemperaturen werden in Abhängigkeit des verwendeten Lösungsmittels und gegebenenfalls des Drucks gewählt. Vorzugsweise wird die Reaktion bei Atmosphärendruck durchgeführt.

Verbindungen der Formel I in denen Q für Q₉ steht, können analog JP 11152273 A (Priorität: 19.11.1997 JP 318614), Verbindungen der Formel 1, worin Q für Q₁₀ steht, können nach J. Org. Chem. (1979), 44(26), 4906-4912 oder
J. Org. Chem. (1977), 42(7), 1163-1169 oder analog hergestellt werden.

Die Umsetzungen zu Verbindungen der Formel I werden vorteilhafterweise in aprotischen, inerten organischen Lösungsmitteln vorgenommen. Solche Lösungsmittel sind Kohlenwasserstoffe wie Benzol, Toluol, Xylol oder Cyclohexan, chlorierte Kohlenwasserstoffe wie Dichlormethan, Trichlormethan, Tetrachlormethan oder Chlorbenzol, Ether wie Diethylether, Ethylenglykoldimethylether, Diethylenglykoldimethylether, Tetrahydrofuran oder Dioxan, Nitrile wie Acetonitril oder Propionitril, Amide wie N,N-Dimethylformamid, Diethylformamid oder N-Methylpyrrolidinon. Die Reaktionstemperaturen liegen vorzugsweise zwischen -20°C und +120°C. Die Umsetzungen verlaufen im allgemeinen leicht exotherm und können in der Regel bei Raumtemperatur durchgeführt werden. Zum Abkürzen der Reaktionszeit oder auch zum Einleiten der Umsetzung kann gegebenenfalls für kurze Zeit bis zum Siedepunkt des Reaktionsgemisches aufgewärmt werden. Die Reaktionszeiten können ebenfalls durch Zugabe einiger Tropfen Base als Reaktionskatalysator verkürzt werden. Als Basen sind insbesondere tertiäre Amine wie Trimethylamin, Triethylamin, Chinuclidin, 1,4-Diazabicyclo-[2.2.2]octan, 1,5-Diazabicyclo[4.3.0]non-5-en oder 1,5-Diazabicyclo[5.4.0]undec-7-en geeignet. Als Basen können aber auch anorganische Basen wie Hydride wie Natrium- oder Calciumhydrid, Hydroxide wie Natrium- oder Kaliumhydroxid, Carbonate wie Natrium- und Kaliumcarbonat oder Hydrogencarbonate wie Kalium- und Natriumhydrogencarbonat verwendet werden.
Die Verbindungen der Formel I können auf übliche Weise durch Einengen und/oder Verdampfen des Lösungsmittels isoliert und durch Umkristallisieren oder Zerreiben des festen Rückstandes in Lösungsmitteln, in denen sie sich nicht gut lösen, wie Ether, aromatischen Kohlenwasserstoffe oder chlorierten Kohlenwasserstoffe, gereinigt werden.

Für die erfindungsgemäße Verwendung der Verbindungen der Formel I oder diese enthaltende Mittel kommen alle in der Landwirtschaft üblichen Applikationsmethoden wie z.B. preemergente Applikation, postemergente Applikation und Saatbeizung, sowie verschiedene Methoden und Techniken in Betracht, wie beispielsweise die kontrollierte Wirkstoffabgabe. Dazu wird der Wirkstoff in Lösung auf mineralische Granulatträger oder polymerisierte Granulate (Harnstoff/Formaldehyd) aufgezogen und getrocknet. Gegebenenfalls kann zusätzlich ein Überzug aufgebracht werden (Umhüllungsgranulate), der es erlaubt, den Wirkstoff über einen bestimmten Zeitraum dosiert abzugeben.

Die Verbindungen der Formel I können in unveränderter Form, d.h. wie sie in der Synthese anfallen, als Herbizide eingesetzt werden. Vorzugsweise verarbeitet man sie aber auf übliche Weise mit den in der Formulierungstechnik gebräuchlichen Hilfsmitteln z.B. zu emulgierbaren Konzentraten, direkt versprühbaren oder verdünnbaren Lösungen, verdünnten Emulsionen, Spritzpulvern, löslichen Pulvern, Stäubemitteln, Granulaten oder Mikrokapseln. Solche Formulierungen sind beispielsweise in der WO 97/34485 auf den Seiten 9 bis 13 beschrieben. Die Anwendungsverfahren wie Versprühen, Vernebeln, Verstäuben, Benetzen, Verstreuen oder Gießen werden gleich wie die Art der Mittel, den angestrebten Zielen und den gegebenen Verhältnissen entsprechend gewählt.

Die Formulierungen, d.h. die den Wirkstoff der Formel I bzw. mindestens einen Wirkstoff der Formel I und in der Regel einen oder mehrere feste oder flüssige Formulierungshilfsmittel enthaltenden Mittel, Zubereitungen oder Zusammensetzungen werden in bekannter Weise hergestellt, z.B. durch inniges Vermischen und/oder Vermahlen der Wirkstoffe mit den Formulierungshilfsmitteln wie z.B. Lösungsmittel oder festen Trägerstoffe. Ferner können zusätzlich oberflächenaktive Verbindungen (Tenside) bei der Herstellung der Formulierungen verwendet werden. Beispiele für Lösungsmittel und feste Trägerstoffe sind z.B. in der WO 97/34485 auf der Seite 6 angegeben.

Als oberflächenaktive Verbindungen kommen je nach der Art des zu formulierenden Wirkstoffes der Formel I nichtionogene, kation- und/oder anionaktive Tenside und Tensidgemische mit guten Emulgier-, Dispergier- und Netzeigenschaften in Betracht. Beispiele für geeignete anionische, nichtionische und kationische Tenside sind beispielsweise in der WO 97/34485 auf den Seiten 7 und 8 aufgezählt. Ferner sind auch die in der Formulierungstechnik gebräuchlichen Tenside, die u.a. in "Mc Cutcheon's Detergents and Emulsifiers Annual" MC Publishing Corp., Ridgewood New Jersey, 1981, Stache, H., "Tensid-Taschenbuch", Carl Hanser Verlag, München/Wien, 1981 und M. und J. Ash, "Encyclopedia of Surfactants", Vol I-III, Chemical Publishing Co., New York, 1980-81 beschrieben sind, zur Herstellung der erfindungsgemäßen herbiziden Mittel geeignet.

Erfindungsgemäße herbizide und den Pflanzenwuchs hemmende Mittel mit einem herbizid wirksamen Gehalt an Verbindung der Formel I können durch Zugabe von Sprühtank-Adjuvantien in ihrer Wirksamkeit gesteigert werden.
Diese Adjuvantien können beispielsweise sein: nichtionische Tenside, Mischungen von nichtionischen Tensiden, Mischungen von anionischen Tensiden mit nichtionischen Tensiden, kationische Tenside, silizium-organische Tenside, Mineralölderivate mit und ohne Tenside , Pflanzenölderivate mit und ohne Tensidzusatz, alkylierte Derivate von Ölen pflanzlichen oder mineralischen Ursprungs mit und ohne Tenside, Fischöle und andere tierische Öle tierischer Natur sowie deren Alkylderivate mit und ohne Tenside, natürlich vorkommende höhere Fettsäuren, vorzugsweise mit 8 bis 28 Kohlenstoffatomen, und deren Alkylesterderivate, organische Säuren enthaltend ein aromatisches Ringsystem und einen oder mehrere Carbonsäurerester, sowie deren Alkylderivaten, ferner Suspensionen von Polymeren des Vinylacetats oder Copolymeren von Vinylacetat-Acrylsäureestern. Mischungen einzelner Adjuvantien untereinander sowie in Kombination mit organischen Lösungsmitteln können zu einer weiteren Steigerung der Wirkung führen.

Als nichtionische Tenside kommen beispielsweise Polyglykoletherderivate von aliphatischen oder cycloaliphatischen Alkoholen, gesättigten oder ungesättigten Fettsäuren und Alkylphenolen in Frage, vorzugsweise die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, vorzugsweise 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit vorzugsweise 1 bis 10 Kohlenstoffatomen in der Alkylkette. Die genannten Verbindungen enthalten üblicherweise pro Propylenglykol-Einheit 1 bis 5 Ethylenglykoleinheiten.

Als weitere Beispiele nichtionischer Tenside seien auch Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei anionischen Tensiden werden vor allem Alkylsulfate, Alkylsulfonate, Alkylarylsulfonate, alkylierte Phosphorsäuren bevorzugt sowie deren ethoxylierte Derivate. Die Alkylreste enthalten üblicherweise 8 bis 24 Kohlenstoffatome.

Bevorzugte nicht-ionische Tenside sind unter den folgenden Handelsnamen bekannt:

Polyoxyethylen Cocoalkylamin (z.B. AMIET® 105 (Kao Co.)), Polyoxyethylen Oleylamin (z.B. AMIET® 415 (Kao Co.)), Nonylphenolpolyethoxyethanole, Polyoxyethylen Stearylamin (z.B. AMIET® 320 (Kao Co.)), N-polyethoxyethylamines (z.B. GENAMIN® (Hoechst AG)), N,N,N',N'-Tetra(Polyethoxypolypropoxyethyl)ethylen-diamine (z.B. TERRONIL® und TETRONIC® (BASF Wyandotte Corp.)), BRIJ® (Atlas Chemicals), ETHYLAN® CD und ETHYLAN® D (Diamond Shamrock), GENAPOL® C, GENAPOL® O, GENAPOL® S und GENAPOL® X080 (Hoechst AG), EMULGEN® 104P, EMULGEN® 109P und EMULGEN® 408 (Kao Co.); DISTY® 125 (Geronazzo), SOPROPHOR® CY 18 (Rhone Poulenc S.A.); NONISOL® (Ciba-Geigy), MRYJ® (ICI); TWEEN® (ICI); EMULSOGEN® (Hoechst AG); AMIDOX® (Stephan Chemical Co.), ETHOMID® (Armak Co.); PLURONIC® (BASF Wyandotte Corp.), SOPROPHOR® 461 P (Rhöne Poulenc S.A.), SOPROPHOR® 496/P (Rhone Poulenc S.A.), ANTAROX FM-63 (Rhone Poulenc S.A.), SLYGARD 309 (Dow Corning), SILWET 408, SILWET L-7607N (Osi-Specialities).

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die verwendeten Öle sind entweder von mineralischer oder natürlicher Herkunft. Die natürlichen Öle können zudemnoch von tierischem oder pflanzlichen Ursprung sein. Bei tierischen Ölen werden vor allem Derivate von Rindertalg bevorzugt, aber auch Fischöle (z.B. Sardinenöl) und deren Derivate werden verwendet. Pflanzliche Öle sind meist Saatöle verschiedener Herkunft. Als Beispiele für besonders verwendete Pflanzenöle können Kokos-, Raps- oder Sonnenblumenöle sowie deren Derivate genannt werden.
In dem erfindungsgemäßen Mittel betragen die Aufwandmengen an Öladditiv in der Regel zwischen 0,01 und 2 % in Bezug auf die Spritzbrühe. Beispielsweise kann das Öladditv nach Herstellung der Spritzbrühe in der gewünschten Konzentration in den Sprühtank gegeben werden.

Im erfindungsgemäßen Mittel bevorzugte Öladditive enthalten ein Öl pflanzlichen Ursprungs wie beispielsweise Rapsöl oder Sonnenblumenöl, Alkylester von Ölen pflanzlichen Ursprungs wie beispielsweise die Methylderivate, oder Mineralöle.

Besonders bevorzugte Öladditive enthalten Alkylester von höheren Fettsäuren (C₈-C₂₂)-insbesondere die Methylderivate von C₁₂-C₁₈ Fettsäuren, beispielsweise die Methylester der Laurinsäure, Palmitinsäure und Ölsäure. Diese Ester sind bekannt als Methyllaurat (CAS-111-82-0), Methylpalmitat (CAS-112-39-0) und Methyloleat (CAS-112-62-9).

Das Ausbringen und die Wirkung der Öladditive kann durch deren Kombination mit oberflächenaktiven Substanzen wie nichtionische-, anionische oder kationische Tenside verbessert werden. Beispiele für geeignete anionische, nichtionische und kationische Tenside sind in der WO 97/auf den Seiten 7 und 8 aufgezählt.

Bevorzugte oberflächenaktive Substanzen sind anionische Tenside vom Typ der Dodecylbenzylsulfonate, insbesondere die Calciumsalze davon sowie nichtionische Tenside vom Typ der Fettalkoholethoxylate. lnsbeondere bevorzugt sind ethoxylierte C₁₂-C₂₂₋Fettalkohole mit einem Ethoxylierungsgrad zwischen 5 und 40. Beispiele für kommerziell erhältliche, bevorzugte Tenside sind die Genapol Typen (Clariant AG, Muttenz, Schweiz). Die Konzentration der oberflächenaktiven Substanzen in Bezug auf das gesamte Additiv beträgt im allgemeinen zwischen 1 und 30 Gew.%.

Beispiele für Öladditive, die aus Mischungen von Ölen bzw. Mineralölen oder deren Derivaten mit Tensiden bestehen, sind Edenor ME SU®, Emery 2231® (Henkel Tochtergesellschaft Cognis GMBH, DE), Turbocharge® (Zeneca Agro, Stoney Creek, Ontario , CA) oder, besonders bevorzugt, Actipron® (BP Oil UK Limited, GB).

Ferner kann die Zugabe eines organischen Lösungsmittels zu dem Öladditiv/Tensidgemisch eine weitere Steigerung der Wirkung bewirken. Geeignete Lösungsmittel sind beispielsweise Solvesso® (ESSO) oder Aromatic Solvent® (Exxon Corporation) Typen.

Die Konzentration derartiger Lösungsmittel kann von 10 bis 80 Gew.% des Gesamtgewichtes betragen.

Derartige Öladditive, die beispielsweise auch in US-A-4,834,908 beschrieben sind, sind für das erfindungsgemäße Mittel besonders bevorzugt. Ein ganz besonders bevorzugtes Öladditiv ist unter dem Namen MERGE® bekannt, kann von der BASF Corporation bezogen werden und ist beispielsweise in US-A-4,834,908 in col. 5, als Example COC-1 im wesentlichen beschrieben. Ein weiteres erfindungsgemäß bevorzugtes Öladditiv ist SCORE® (Novartis Crop Protection Canada.)

In der Formulier- und Adjuvanttechnik gebräuchliche Tenside, Öle, insbesondere Pflanzenöle, Derivate davon wie alkylierte Fettsäuren und Mischungen davon, z.B. mit vorzugsweise anionischen Tensiden wie alkylierten Phosphorsäuren, Alkylsulfate und Alkylarylsulfonaten sowie höheren Fettsäuren, die auch in den erfindungsgemäßen Mitteln und Sprühtanklösungen davon verwendet werden können, sind u.a. in "Mc Cutcheon's Detergents and Emulsifiers Annual" MC Publishing Corp., Ridgewood New Jersey, 1998, Stache, H., "Tensid-Taschenbuch", Carl Hanser Verlag, München/Wien, 1990, M. und J. Ash, "Encyclopedia of Surfactants", Vol I-IV, Chemical Publishing Co., New York, 1981-89, G. Kapusta, "A Compendium of Herbicide Adjuvants", Southern lllinois Univ. , 1998, L. Thomson Harvey, "A Guide to Agricultural Spray Adjuvants Used in the United States", Thomson Pubns., 1992 beschrieben.

Die herbiziden Formulierungen enthalten in der Regel 0,1 bis 99 Gew%, insbesondere 0,1 bis 95 Gew.-% Herbizid, 1 bis 99,9 Gew.-%, insbesondere 5 bis 99,8 Gew.-%, eines festen oder flüssigen Formulierungshilfsstoffes und 0 bis 25 Gew.-%, insbesondere 0,1 bis 25 Gew.-%, eines Tensides. Während als Handelsware eher konzentrierte Mittel bevorzugt werden, verwendet der Endverbraucher in der Regel verdünnte Mittel. Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe enthalten.

Die Wirkstoffe der Formel I werden in der Regel auf die Pflanze oder deren Lebensraum mit Aufwandmengen von 0,001 bis 4 kg/ha, insbesondere 0,005 bis 2 kg/ha eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Die Verbindungen der Formel I zeichnen sich durch herbizide und wuchshemmende Eigenschaften aus, die sie zum Einsatz in Kulturen von Nutzpflanzen, insbesondere in Getreide, Baumwolle, Soja, Zuckerrüben, Zuckerrohr, Plantagen, Raps, Mais und Reis sowie zur nicht-selektiven Unkrautkontrolle befähigen. Unter Kulturen sind auch solche zu verstehen, die durch konventionelle züchterische oder gentechnologische Methoden gegen Herbizide bzw. Herbizidklassen tolerant gemacht worden sind. Diese sind z.B. IMI Maize, Poast Protected Maize (Sethoxydim-Toleranz), Liberty Link Maize, B.t./Liberty Link Maize, IMI/Liberty Link Maize, IMI/Liberty Link /B.t. Maize, Roundup Ready Maize und Roundup Ready/B.t. Maize.

Bei den zu bekämpfenden Unkräutern kann es sich sowohl um mono- als auch um dikotyle Unkräuter handeln, wie zum Beispiel Stellaria, Nasturtium, Agrostis, Digitaria, Avena, Setaria, Sinapis, Lolium, Solanum, Echinochloa, Scirpus, Monochoria, Sagittaria, Bromus, Alopecurus, Sorghum halepense, Rottboellia, Cyperus, Abutilon, Sida, Xanthium, Amaranthus, Chenopodium, lpomoea, Chrysanthemum, Galium, Viola und Veronica.

Es hat sich überraschenderweise gezeigt, daß spezielle, aus US-A-5,041,157, US-A-5,541,148, US-A-5,006,656, EP-A-0 094 349, EP-A-0 551 650, EP-A-0 268 554, EP-A-0 375 061, EP-A-0 174 562, EP-A-492 366, WO 91/7874, WO 94/987, DE-A-19612943, WO 96/29870, WO 98/13361, WO 98/39297, WO 98/27049, EP-A- 0 716 073, EP-A- 0 613 618, US-A-5,597,776 EP-A-0430 004, DE-A-4 331 448, WO 99/16744, WO 00/30447 und WO 00/00020 bekannte Safener zur Mischung mit dem erfindungsgemäßen herbiziden Mittel geeignet sind. Daher betrifft die vorliegende Erfindung auch ein selektiv-herbizides Mittel zur Bekämpfung von Gräsern und Unkräutern in Kulturen von Nutzpflanzen, insbesondere in Kulturen von Mais und Getreide, welches ein Herbizid der Formel I und einen Safener (Gegenmittel, Antidot) enthält und welches die Nutzpflanzen, nicht aber die Unkräuter vor der phytotoxischen Wirkung des Herbizides bewahrt, sowie die Verwendung dieses Mittels zur Unkrautbekämpfung in Nutzpflanzenkulturen.

Erfindungsgemäß wird somit ein selektiv-herbizides Mittel vorgeschlagen, welches dadurch gekennzeichnet ist, daß es neben üblichen inerten Formulierungshilfsmitteln wie Trägerstoffen, Lösungsmitteln und Netzmitteln als Wirkstoff eine Mischung aus

### a) einer herbizid-wirksamen Menge einer Verbindung der Formel I

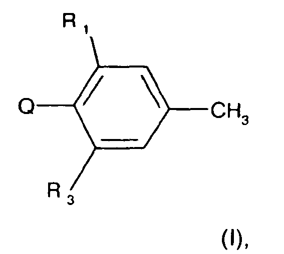

worin R₁, R₃ und Q die oben angegebenen Bedeutungen haben, mit der Maßgabe, daß Q verschieden von Q₁ ist; und

### b) einer herbizid-antagonistisch wirksamen Menge entweder eine Verbindung der Formel X

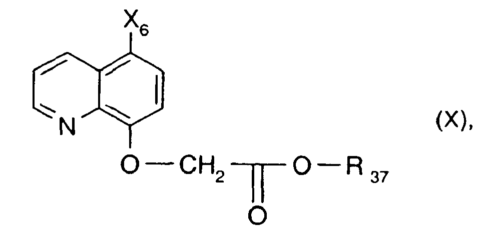

worin
R₃₇ Wasserstoff, C₁-C₈-Alkyl oder durch C₁-C₆-Alkoxy oder C₃-C₆-Alkenyloxy substituiertes C₁-C₈-Alkyl; und X₆ Wasserstoff oder Chlor bedeutet; oder einer Verbindung der Formel XI 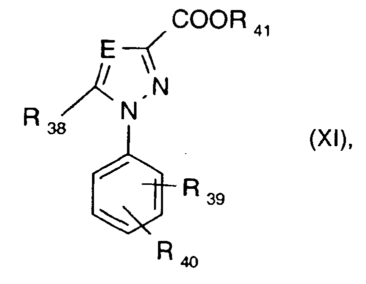 worin E Stickstoff oder Methin;
R₃₈ -CCl₃, Phenyl oder durch Halogen substituiertes Phenyl;
R₃₉ und R₄₀ unabhängig voneinander Wasserstoff oder Halogen; und
R₄₁ C₁-C₄-Alkyl bedeuten; oder einer Verbindung der Formel XII 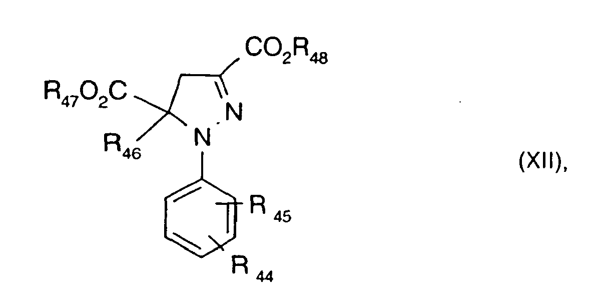 worin R₄₄ und R₄₅ unabhängig voneinander Wasserstoff oder Halogen und
R₄₆, R₄₇ und R₄₈ unabhängig voneinander C₁-C₄-Alkyl bedeuten, oder einer Verbindung der Formel XIII 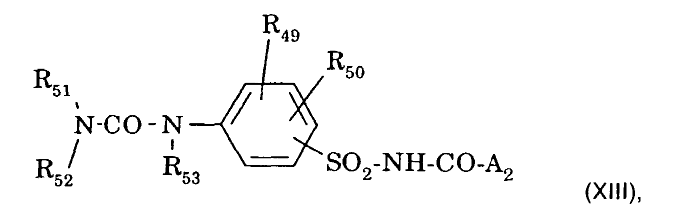 worin A₂ für eine Gruppe 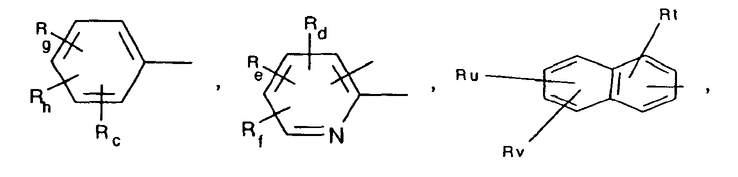 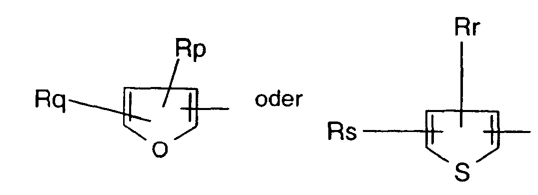 steht,
R₅₁, und R₅₂ unabhängig voneinander Wasserstoff, C₁-C₈-Alkyl, C₃-C₈-Cycloalkyl, C₃-C₆₋Alkenyl, C₃-C₆-Alkinyl, 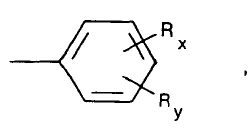 oder durch C₁-C₄-Alkoxy oder 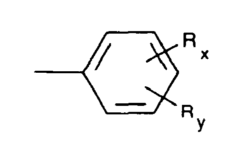 substituiertes C₁-C₄-Alkyl bedeutet; oder R₅₁ und R₅₂ bilden
zusammen eine C₄-C₆-Alkylenbrücke, die durch Sauerstoff, Schwefel, SO, SO₂, NH oder -N(C₁-C₄-Alkyl)- unterbrochen sein kann,
R₅₃ für Wasserstoff oder C₁-C₄-Alkyl;
R₄₉ für Wasserstoff, Halogen, Cyano, Trifluoromethyl, Nitro, C₁-C₄-Alkyl, C₁-C₄-Alkoxy, C₁₋C₄-Alkylthio, C₁-C₄ -Alkylsulfinyl, C₁-C₄-Alkylsulfonyl, -COORⱼ, -CONRₖRₘ, -CORₙ, -SO₂NRₖRₘ oder -OSO₂-C₁-C₄-Alkyl;
R_{g} für Wasserstoff, Halogen, Cyano, Nitro, C₁-C₄-Alkyl, C₁-C₄-Halogenalkyl, C₁-C₄-Alkylthio, C₁-C₄-Alkylsulfinyl, C₁-C₄-Alkylsulfonyl, -COORⱼ, -CONRₖRₘ, -CORₙ, -SO₂NRₖRₘ, -OSO₂-C₁₋C₄-Alkyl, C₁-C₆-Alkoxy, oder C₁-C₆alkoxy substituiert durch C₁-C₄-Alkoxy oder Halogen, C₃₋C₆-Alkenyloxy, oder C₃-C₆-Alkenyloxy substituiert durch Halogen, oder C₃-C₆-Alkinyloxy, oder R₄₉ und R₅₀ zusammen bilden eine C₃-C₄-Alkylenbrücke, die durch Halogen oder C₁-C₄-Alkyl substituiert sein kann, oder bilden eine C₃-C₄-Alkenylenbrücke, die durch Halogen oder C₁₋C₄-Alkyl substituiert sein kann, oder bilden eine C₄-Alkadienylenbrücke, die durch Halogen oder C₁-C₄-Alkyl substituiert sein kann;
R₅₀ und Rₕ unabhängig voneinander für Wasserstoff, Halogen, C₁-C₄-Alkyl, Trifluormethyl, C₁-C₆-Alkoxy, C₁-C₆-Alkylthio oder -COORⱼ;
R_{c} für Wasserstoff, Halogen, Nitro, C₁-C₄-Alkyl oder Methoxy; R_{d} für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ;
Rₑ für Wasserstoff, Halogen, C₁-C₄-Alkyl, -COORⱼ, Trifluormethyl or Methoxy, oder R_{d} und Rₑ bilden zusammen eine C₃-C₄-Alkylenbrücke;
Rp für Wasserstoff, Halogen, C₁-C₄-Alkyl, -COORⱼ, Trifluormethyl or Methoxy; Rq für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ; , oder Rp und Rq bilden zusammen eine C₃-C₄₋Alkylenbrücke;
Rr für Wasserstoff, Halogen, C₁-C₄-Alkyl, -COORⱼ, Trifluormethyl or Methoxy; Rs für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ;, oder Rr und Rs bilden zusammen eine C₃-C₄₋Alkylenbrücke;
Rt für Wasserstoff, Halogen, C₁-C₄-Alkyl, -COORⱼ, Trifluormethyl or Methoxy; Ru für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ; oder Rv und Ru bilden zusammen eine C₃-C₄₋Alkylenbrücke;
R_{f} und Rv für Wasserstoff, Halogen oder C₁-C₄-Alkyl;
Rₓ und R_{y} unabhängig voneinander für Wasserstoff, Halogen, C₁-C₄-Alkyl, C₁-C₄-Alkoxy, C₁₋C₄-Alkylthio, -COOR₅₄, Trifluoromethyl, Nitro oder Cyano;
Rⱼ, Rₖ und Rₘ unabhängig voneinander für Wasserstoff oder C₁-C₄-Alkyl; oder
Rₖ und Rₘ bilden zusammen eine C₄-C₆-Alkylenbrücke, die durch Sauerstoff, NH oder -N(C₁₋C₄-Alkyl)- unterbrochen sein kann;
Rₙ für C₁-C₄-Alkyl, Phenyl, oder durch Halogen, C₁-C₄-Alkyl, Methoxy, Nitro oder Trifluormethyl substituiertes Phenyl;
R₅₄ für Wasserstoff, C₁-C₁₀-Alkyl, C₁-C₄-Alkoxy-C₁-C₄-alkyl, C₁-C₄-Alkylthio-C₁-C₄-alkyl, Di-C₁-C₄-alkylamino-C₁-C₄-alkyl, Halogen-C₁-C₈-alkyl, C₂-C₈-Alkenyl, Halogen-C₂-C₈-alkenyl, C₃-C₈-Alkinyl, C₃-C₇-Cycloalkyl, Halogen-C₃-C₇-cycloalkyl, C₁-C₈-Alkylcarbonyl, Allylcarbonyl, C₃-C₇-Cycloalkylcarbonyl, Benzoyl, das unsubstituiert oder am Phenylring gleich oder verschieden bis zu dreifach durch Halogen, C₁-C₄-Alkyl, Halogen-C₁-C₄-alkyl, Halogen-C₁₋C₄-alkoxy oder C₁-C₄-Alkoxy substituiert ist; oder Furoyl, Thienyl; oder C₁-C₄-Alkyl substituiert durch Phenyl, Halogenphenyl, C₁-C₄-Alkylphenyl, C₁-C₄-Alkoxyphenyl, Halogen-C₁-C₄-alkylphenyl, Halogen-C₁-C₄-alkoxyphenyl, C₁-C₆-Alkoxycarbonyl, C₁-C₄-Alkoxy-C₁-C₈₋alkoxycarbonyl, C₃-C₈-Alkenyloxycarbonyl, C₃-C₈-Alkinyloxycarbonyl, C₁-C₈₋Alkylthiocarbonyl, C₃-C₈-Alkenylthiocarbonyl, C₃-C₈-Alkinylthiocarbonyl, Carbamoyl, Mono-C₁-C₄-alkylaminocarbonyl, Di-C₁-C₄-alkylaminocarbonyl; oder Phenylaminocarbonyl, das unsubstituiert oder am Phenyl gleich oder verschieden bis zu dreifach durch Halogen, C₁₋C₄-Alkyl, Halogen-C₁-C₄-alkyl, Halogen-C₁-C₄-alkoxy oder C₁-C₄-Alkoxy oder einfach durch Cyano oder Nitro substituiert ist, oder Dioxolan-2-yl, das unsubstituiert ist oder durch ein oder zwei C₁-C₄-Alkylreste substituiert ist, oder Dioxan-2-yl, das unsubstituiert ist oder durch ein oder zwei C₁-C₄-Alkylreste substituiert ist, oder C₁-C₄-Alkyl, das durch Cyano, Nitro, Carboxyl oder C₁-C₈-Alkylthio-C₁-C₈-alkoxycarbonyl substituiert ist, bedeutet;
oder einer Verbindung der Formel XIV 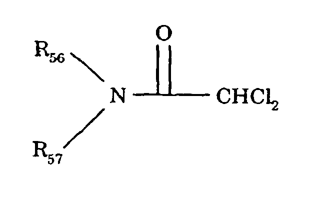 (XIV), worin R₅₆ und R₅₇ unabhängig voneinander für C₁₋C₆-Alkyl oder C₂-C₆-Alkenyl; oder R₅₆ und R₅₇ zusammen für 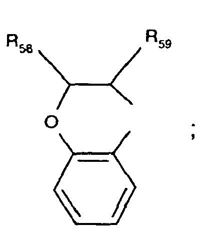 R₅₈ und R₅₉ unabhängig voneinander für Wasserstoff oder C₁-C₆-Alkyl; oder R₅₆ und R₅₇ zusammen 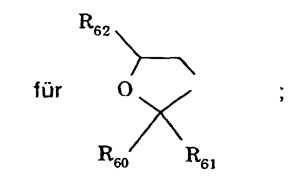 R₆₀ und R₆₁ unabhängig voneinander für C₁-C₄-Alkyl, oder R₆₀ und R₆₁ zusammen -(CH₂)₅- ;
R₆₂ für Wasserstoff, C₁-C₄-Alkyl oder 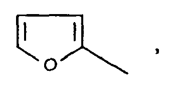 oder R₅₆ und R₅₇ zusammen für 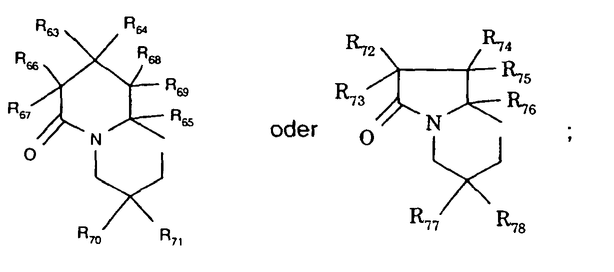 R₆₃, R₆₄, R₆₅, R₆₆, R₆₇, R₆₈, R₆₉, R₇₀, R₇₁, R₇₂, R₇₃, R₇₄, R₇₅, R₇₆, R₇₇ und R₇₈ unabhängig voneinander für Wasserstoff oder C₁-C₄-Alkyl stehen;
oder einer Verbindung der Formel XV 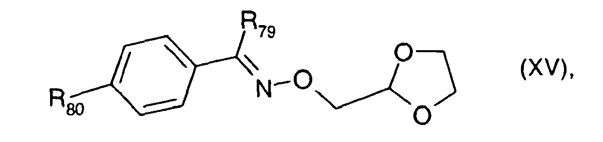 worin R₈₀ Wasserstoff oder Chlor und R₇₉ Cyano oder Trifluormethyl bedeutet,
oder eine Verbindung der Formel XVI 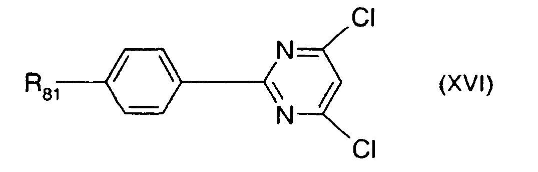 worin R₈₁, Wasserstoff oder Methyl bedeutet,
oder einer Verbindung der Formel XVII 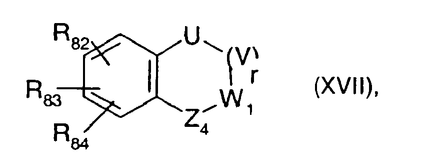 worin
R₈₂Wasserstoff, C₁-C₄-Alkyl oder C₁-C₄-Alkyl substituiert durch C₁-C₄-Alkyl-X₂- oder C₁-C₄₋Halogenalkyl-X₂- bedeutet, oder für C₁-C₄-Halogenalkyl, Nitro, Cyano, -COOR₈₅, -NR₈₆R₈₇,-SO₂NR₈₈R₈₉ oder -CONR₉₀R₉₁ steht;
R₈₃ Wasserstoff, Halogen, C₁-C₄-Alkyl, Trifluormethyl, C₁-C₄-Alkoxy oder C₁-C₄₋Halogenalkoxy bedeutet;
R₈₄ Wasserstoff, Halogen oder C₁-C₄-Alkyl bedeutet;
U, V, W, und Z₄ unabhängig voneinander Sauerstoff, Schwefel, C(R₉₂)R₉₃, Carbonyl, NR₉₄, eine Gruppe 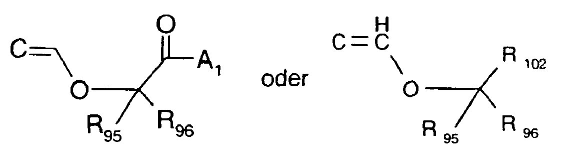 bedeuten, worin R₁₀₂ C₂-C₄-Alkenyl oder C₂-C₄-Alkinyl bedeutet; mit den Maßgaben, daß
a) mindestens eines der Ringglieder U, V, W₁ oder Z₄ Carbonyl ist, und ein zu diesem bzw. diesen Ringgliedern benachbartes Ringglied die Gruppe 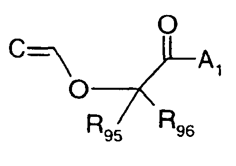 oder 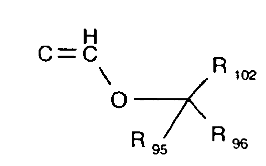 bedeutet, wobei diese Gruppe nur einmal vorkommt; und
b) zwei benachbarte Ringglieder U und V, V und W₁ und W₁ und Z₄ nicht gleichzeitig Sauerstoff bedeuten können;
R₉₅ und R₉₆ unabhängig voneinander Wasserstoff oder C₁-C₈-Alkyl bedeuten; oder
R₉₅ und R₉₆ zusammen eine C₂-C₆-Alkylengruppe bilden;
A₁ R₉₉-Y₁- oder -NR₉₇R₉₈;
X₂ Sauerstoff oder -S(O)ₛ ;
Y₁ Sauerstoff oder Schwefel bedeuten;
R₉₉ Wasserstoff, C₁-C₈-Alkyl, C₁-C₈-Halogenalkyl, C₁-C₄-Alkoxy-C₁-C₈-alkyl, C₃-C₆₋Alkenyloxy-C₁-C₈-alkyl oder Phenyl-C₁-C₈-alkyl bedeutet, wobei der Phenylring durch Halogen, C₁-C₄-Alkyl, Trifluormethyl, Methoxy oder Methyl-S(O)ₛ- substituiert sein kann, oder C₃-C₆-Alkenyl, C₃-C₆-Halogenalkenyl, Phenyl-C₃-C₆-alkenyl, C₃-C₆-Alkinyl, Phenyl-C₃₋C₆-alkinyl, Oxetanyl, Furyl oder Tetrahydrofuryl bedeutet;
R₈₅ Wasserstoff oder C₁-C₄-Alkyl;
R₈₆ Wasserstoff, C₁-C₄-Alkyl oder C₁-C₄-Alkylcarbonyl bedeuten;
R₈₇ Wasserstoff oder C₁-C₄-Alkyl bedeutet; oder
R₈₆ und R₈₇ zusammen eine C₄- oder C₅-Alkylengruppe bilden;
R₈₈, R₈₉, R₉₀ und R₉₁ unabhängig voneinander Wasserstoff oder C₁-C₄-Alkyl bedeuten; oder R₈₈ zusammen mit R₈₉ oder R₉₀ zusammen mit R₉₁ unabhängig voneinander C₄- oder C₅₋Alkylen sind, wobei ein Kohlenstoffatom durch Sauerstoff oder Schwefel, oder ein oder zwei Kohlenstoffatome durch -NR₁₀₀- ersetzt sein können;
R₉₂, R₁₀₀ und R₉₃ unabhängig voneinander Wasserstoff oder C₁-C₈-Alkyl sind; oder
R₉₂ und R₉₃ zusammen C₂-C₆-Alkylen sind;
R₉₄ Wasserstoff oder C₁-C₈-Alkyl bedeutet;
R₉₇ Wasserstoff, C₁-C₈-Alkyl, Phenyl oder Phenyl-C₁-C₈-alkyl bedeutet, wobei die Phenylringe durch Fluor, Chlor, Brom, Nitro, Cyano, -OCH₃, C₁-C₄-Alkyl oder CH₃SO₂₋substituiert sein können, oder für C₁-C₄-Alkoxy-C₁-C₈-alkyl, C₃-C₆-Alkenyl oder C₃-C₆-Alkinyl; R₉₈ Wasserstoff, C₁-C₈-Alkyl, C₃-C₆-Alkenyl oder C₃-C₆-Alkinyl steht; oder
R₉₇ und R₉₈ zusammen C₄- oder C₅-Alkylen bedeuten, wobei ein Kohlenstoffatom durch Sauerstoff oder Schwefel, oder ein oder zwei Kohlenstoffatome durch
-NR₁₀₁- ersetzt sein können;
R₁₀₁ Wasserstoff oder C₁-C₄-Alkyl bedeutet;
r 0 oder 1 ist; und
s 0, 1 oder 2 bedeutet,
oder eine Verbindung der Formel XVIII 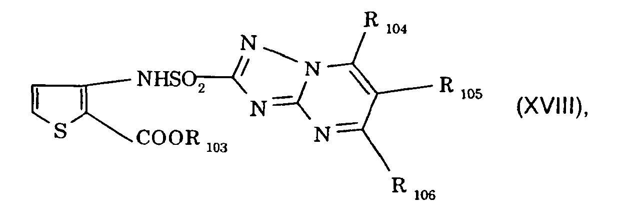 worin R₁₀₃ Wasserstoff, C₁-C₆-Alkyl, C₃-C₆-Cycloalkyl, C₃-C₆-Alkenyl oder C₃-C₆-Alkinyl; und R₁₀₄, R₁₀₅ und R₁₀₆ unabhängig voneinander Wasserstoff, C₁-C₆-Alkyl, C₃-C₆-Cycloalkyl oder C₁-C₆-Alkoxy bedeuten, mit der Maßgabe, daß einer der Substituenten R₁₀₄, R₁₀₅ und R₁₀₆ verschieden von Wasserstoff ist;
oder eine Verbindung der Formel XIX 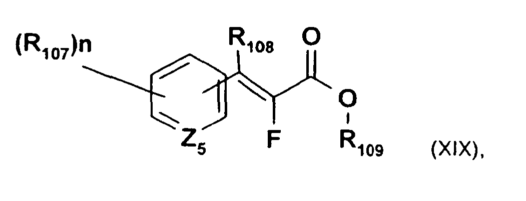 worin Z₅ N oder CH, n für den Fall, daß Z₅ gleich N ist, 0, 1, 2 oder 3 und für den Fall, daß Z₅ CH ist, 0, 1, 2, 3 oder 4 bedeutet, R₁₀₇ Halogen, C₁-C₄-Alkyl, C₁-C₄-Halogenalkyl, C₁-C₄₋Alkoxy, C₁-C₄-Halogenalkoxy, Nitro, C₁-C₄-Alkylthio, C₁-C₄-Alkylsulfonyl, C₁-C₄₋Alkoxycarbonyl, Phenyl oder Phenoxy, oder durch C₁-C₃-Alkyl, C₁-C₃-Halogenalkyl, C₁-C₃₋Alkoxy, C₁-C₃-Halogenalkoxy, Halogen, Cyano oder Nitro substituiertes Phenyl oder Phenoxy bedeutet;
R₁₀₈ Wasserstoff oder C₁-C₄-Alkyl bedeutet, R₁₀₉ Wasserstoff, C₁-C₄-Alkyl, C₃-C₆-Cycloalkyl, C₂-C₆-Alkenyl, C₂-C₆-Alkinyl, C₁-C₄-Halogenalkyl, C₂-C₆-Halogenalkenyl, C₂-C₆₋Halogenalkinyl, C₁-C₄-Alkylthio-C₁-C₄-alkyl, C₁-C₄-Alkylsulfonyl- C₁-C₄-alkyl, C₁-C₄-Alkoxy-C₁-C₄-alkyl, C₁-C₄-Alkenyloxy- C₁-C₄-alkyl oder C₁-C₄-Alkinyloxy-C₁-C₄-alkyl ist;
oder eine Verbindung der Formel XX 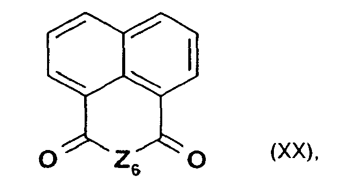 worin Z₆ Sauerstoff oder N-R₁₁₀ und R₁₁₀ eine Gruppe der Formel 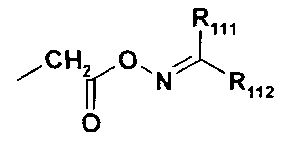 bedeutet, worin R₁₁₁ und R₁₁₂ unabhängig voneinander Cyano, Wasserstoff, C₁-C₄-Alkyl, C₃₋C₆-Cycloalkyl, C₂-C₆-Alkenyl, Aryl, Phenyl oder Heteroaryl, oder durch C₁-C₃-Alkyl, C₁-C₃₋Halogenalkyl, C₁-C₃-Alkoxy, C₁-C₃-Halogenalkoxy, Halogen, Cyano oder Nitro substituiertes Phenyl, Aryl oder Heteroaryl bedeuten;
oder eine Verbindung der Formel XXI 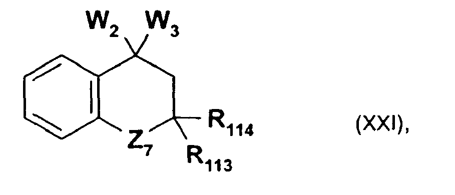 worin Z₇ Sauerstoff, Schwefel, S=O, SO₂ oder CH₂ bedeutet, R₁₁₃ und R₁₁₄ unabhängig voneinander Wasserstoff, Halogen oder C₁-C₄-Alkyl bedeuten, W₂ und W₃ unabhängig voneinander CH₂COOR₁₁₅ oder COOR₀₁₁₅ oder zusammen eine Gruppe der Formel -(CH₂)C(O)-O-C(O)-(CH₂)- bedeuten, und R₁₁₅ und R₀₁₁₅ unabhängig voneinander Wasserstoff, C₁-C₄-Alkyl, C₂-C₄-Alkenyl, C₂-C₆-Alkinyl, C₃-C₆-Cycloalkyl, C₁-C₄-Halogenalkyl, ein Metall- oder ein Ammonium- Kation bedeuten;
oder eine Verbindung der Formel XXII 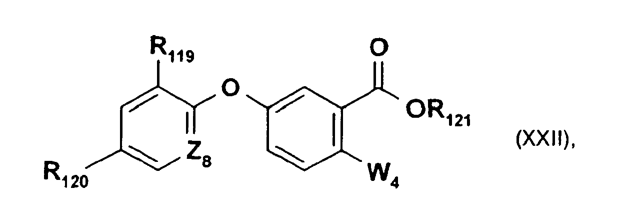 worin R₁₁₉ und R₁₂₀ unabhängig voneinander Wasserstoff, Halogen oder C₁-C₄-Halogenalkyl sind, R₁₂₁ Wasserstoff, C₁-C₄-Alkyl, C₃-C₄-Alkenyl, C₃-C₄-Alkinyl, C₁-C₄-Halogenalkyl, C₃-C₆₋Cycloalkyl, ein Metalkation oder ein Ammoniumkation bedeuten, Z₈ N, CH, C-F oder C-Cl bedeuten und W₄ eine Gruppe der Formel 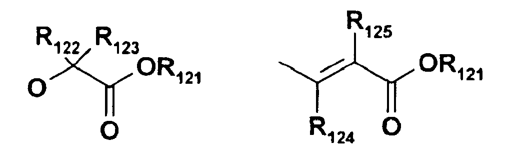 bedeutet, worin R₁₂₂ und R₁₂₃ unabhängig voneinander Wasserstoff oder C₁-C₄-Alkyl und R₁₂₄ und R₁₂₅ unabhängig voneinander Wasserstoff oder C₁-C₄-Alkyl bedeuten;
oder eine Verbindung der Formel XXIII 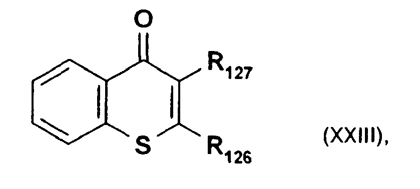 worin R₁₂₆ Wasserstoff, Cyano, Halogen, C₁-C₄-Alkyl, C₃-C₆-Cycloalkyl, C₁-C₄-Alkoxy, C₁-C₄₋Alkoxycarbonyl, C₁-C₄-Alkylthiocarbonyl, -NH-R₁₂₈, -C(O)NH-R₀₁₂₈, Aryl oder Heteroaryl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Aryl oder Heteroaryl bedeutet;
R₁₂₇ Wasserstoff, Cyano, Nitro, Halogen, C₁-C₄-Alkyl, C₁-C₄-Halogenalkyl, C₁-C₄-Alkoxy, C₁₋C₄-Thioalkyl bedeuten, und
R₁₂₈ und R₀₁₂₈ unabhängig voneinander C₁-C₄-Alkyl, C₁-C₄-Halogenalkyl, C₃-C₄-Alkenyl, C₃₋C₄-Alkinyl, C₃-C₄-Cycloalkyl, Aryl oder Heteroaryl, oder durch C₁-C₃-Alkyl, C₁-C₃₋Halogenalkyl, C₁-C₃-Alkoxy, C₁-C₃-Halogenalkoxy, Halogen, Cyano oder Nitro substituiertes Aryl oder Heteroaryl, Formyl, C₁-C₄-Alkylcarbonyl, C₁-C₄-Alkylsufonyl bedeuten;
oder eine Verbindung der Formel XXIV 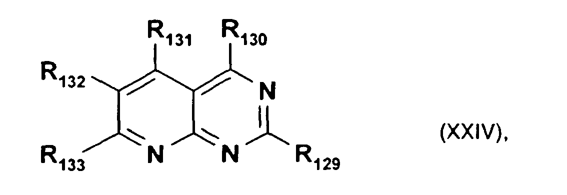 worin R₁₂₉ und R₁₃₀ unabhängig voneinander Wasserstoff, C₁-C₄-Alkyl, C₁-C₄-Halogenalkyl, C₁-C₄-Alkoxy, Mono-C₁-C₈- oder Di-C₁-C₈-Alkylamino, C₃-C₆-Cycloalkyl, C₁-C₄-Thioalkyl, Phenyl oder Heteroaryl sind, R₁₃₁ die Bedeutung von R₁₂₉ hat und zusätzlich OH, NH₂, Halogen, Di- C₁-C₄-Aminoalkyl, C₁-C₄-Alkylthio, C₁-C₄-Alkylsulfonyl oder C₁-C₄₋Alkoxycarbonyl ist, R₁₃₂ die Bedeutung von R₁₂₉ hat und zusätzlich Cyano, Nitro, Carboxyl, C₁-C₄-Alkoxycarbonyl, Di- C₁-C₄-Aminoalkyl, C₁-C₄-Alkylthio, C₁-C₄- Alkylsulfonyl, SO₂-OH, i- C₁-C₄-Aminoalkylsulfonyl oder C₁-C₄-Alkoxysulfonyl ist, R₁₃₃ die Bedeutung von R₁₂₉ hat und zusätzlich OH, NH₂, Halogen, Di- C₁-C₄-Aminoalkyl, Pyrrolidinl-yl, Piperidin-1-yl, Morpholin-1-yl, C₁-C₄-Alkylthio, C₁-C₄-Alkylsulfonyl, C₁-C₄-Alkoxycarbonyl, Phenoxy, Naphtoxy, Phenylamino, Benzoyloxy oder Phenylsulfonyloxy ist;
oder eine Verbindung der Formel XXV 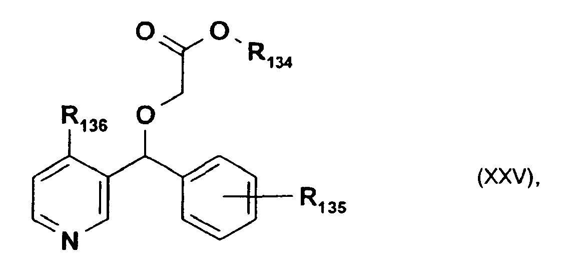 worin R₁₃₄Wasserstoff, C₄-Alkyl, C₁-C₄-Halogenalkyl, C₂-C₄-Alkenyl, C₂-C₄-Alkinyl oder C₁₋C₄-Alkoxy-C₁-C₄-Alkyl bedeutet, R₁₃₅ Wasserstoff, Halogen, C₁-C₄-Alkyl, C₁-C₄-Halogenalkyl oder C₁-C₄-Alkoxy und R₁₃₆ Wasserstoff, Halogen, C₁-C₄-Alkyl, C₁-C₄-Halogenalkyl oder C₁₋C₄-Alkoxy bedeuten, mit der Maßgabe, daß R₁₃₅ und R₁₃₆ nicht gleichzeitig Wasserstoff bedeuten,
oder der Formel XXVI 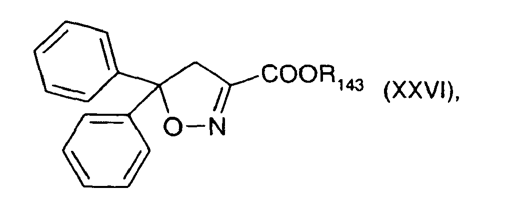 worin
R₁₄₃ Wasserstoff, ein Alkali-, Erdalkali-, Sulfonium- oder Ammonium-Kation oder Ethyl bedeutet;
oder der Formel XXVII 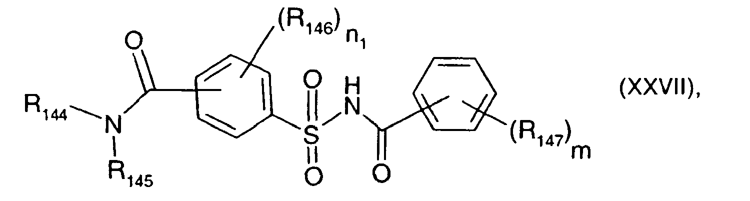 worin R₁₄₄ und R₁₄₅ unabhängig voneinander Wasserstoff, C₁-C₆-Alkyl, C₂-C₆-Alkenyl, C₂-C₆₋Alkinyl oder C₃-C₆-Cycloalkyl bedeuten;
R₁₄₆ Wasserstoff, Halogen, C₁-C₄-Alkyl, C₁-C₆-Halogenalkyl oder C₁-C₆-Halogenalkoxy bedeutet;
R₁₄₇ Wasserstoff, Halogen, C₁-C₄-Alkyl, C₁-C₄-Halogenalkyl, C₁-C₄-Alkoxy, C₁-C₄₋Halogenalkoxy, C₁-C₄-Alkylthio, C₁-C₄-Alkoxycarbonyl oder Nitro bedeutet;
n, 0, 1, 2 oder 3; und
m 1 oder 2 bedeutet;
oder der Formel XXVIII 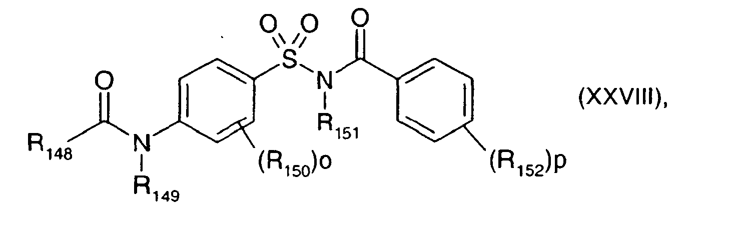 worin
R₁₄₈ Wasserstoff, C₁-C₆-Alkyl, C₁-C₆-Alkoxy, C₁-C₆-Alkylthio, C₃-C₈-Cycloalkyl, Phenyl, Phenyl-C₁-C₆-alkyl oder Heteroaryl bedeutet; wobei die genannten Gruppen durch Halogen, Cyano, Nitro, Amino, Hydroxy, Carbonyl, Carboxyl, Formyl, Carbonamid oder Sulfonamid substituiert sein können;
R₁₄₉ Wasserstoff, C₁-C₆-Alkyl oder C₁-C₄-Halogenalkyl bedeutet;
jedes R₁₅₀ unabhängig Wasserstoff, Halogen, C₁-C₄-Alkyl, C₁-C₄-Halogenalkyl, C₁-C₄-Alkoxy, C₁-C₄-Alkylthio, C₁-C₄-Alkylsulfonyl, Cyano, Nitro, Formyl oder Carboxyl bedeutet;
R₁₅₁ Wasserstoff, C₁-C₆-Alkyl oder C₁-C₄-Halogenalkyl bedeutet;
jedes R₁₅₂ unabhängig Wasserstoff, Halogen, C₁-C₄-Alkyl, C₁-C₄-Halogenalkyl, C₁-C₄-Alkoxy, C₁-C₄-Alkylthio, C₁-C₄-Alkylsulfonyl, Cyano, Nitro, Formyl oder Carboxyl bedeutet;
O für 0, 1, oder 2 steht, und
p 0, 1 oder 2 bedeutet;
oder der Formel XXIX 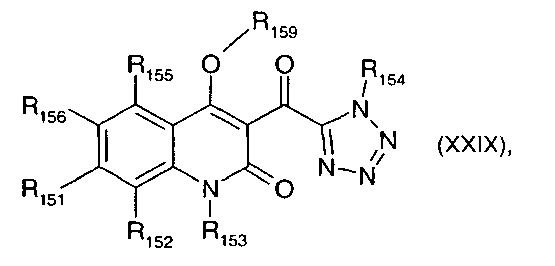 worin
R₁₅₉ Wasserstoff, Formyl, C₁₋₆-Alkylcarbonyl, C₁₋₆-Alkenylcarbonyl, C₁₋₆-Alkinylcarbonyl, C₁₋₆₋Alkoxycarbonyl, C₁₋₆-Alkylthiocarbonyl, C₃₋₈-Cycloalkylcarbonyl, Phenyl-C₁-₆-alkylcarbonyl, Phenylcarbonyl. C₁₋₆-Alkylsulfonyl, C₁₋₆-Alkenylsulfonyl oder Phenylsulfonyl bedeutet, wobei die vorstehend genannten Kohlenwasserstoffgruppen durch ein oder mehrere Halogenatome, Cyano, Nitro, Amino, Methoxy, Ethoxy oder Phenyl substituiert sein können; R₁₅₃ Wasserstoff, C₁₋₆-Alkyl, C₁₋₆-Alkenyl, C₁₋₆-Alkinyl, C₃₋₈-Cycloalkyl, Formyl, C₁₋₆₋Alkylcarbonyl, C₁₋₆-Alkenylcarbonyl, C₁₋₆-Alkinylcarbonyl, C₁₋₆-Alkoxycarbonyl, C₁₋₆₋Alkylthiocarbonyl, C₃₋₈-Cycloalkylcarbonyl, C₁₋₆-Alkylsulfonyl, C₁₋₆-Alkenylsulfonyl oder Phenylsulfonyl bedeutet, wobei die vorstehend genannten Kohlenwasserstoffgruppen durch ein oder mehrere Halogenatome, Cyano, Nitro, Amino, Methoxy, Ethoxy oder Phenyl substituiert sein können;
R₁₅₄ Wasserstoff, C₁₋₆-Alkyl, C₁₋₆-Alkenyl, C₁₋₆-Alkinyl, C₃₋₈-Cycloalkyl, Formyl, C₁₋₆₋Alkylcarbonyl, C₁₋₆-Alkenylcarbonyl, C₁₋₆-Alkinylcarbonyl, C₁₋₆-Alkoxycarbonyl, C₁₋₆₋Alkylthiocarbonyl, C₃₋₈-Cycloalkylcarbonyl, C₁₋₆-Alkylsulfonyl, C₁₋₆-Alkenylsulfonyl oder Phenylsulfonyl bedeutet, wobei die vorstehend genannten Kohlenwasserstoffgruppen durch ein oder mehrere Halogenatome, Cyano, Nitro, Amino, Methoxy, Ethoxy oder Phenyl substituiert sein können;
R₁₅₅, R₁₅₆, R₁₅₇, und R₁₅₈ unabhängig voneinander Wasserstoff, Halogen, Amino, C₁₋₃₋Alkylamino, C₁₋₆-Dialkylamino, Hydroxy, Cyano, Nitro, Formyl, Carboxyl, C₁₋₆-Alkoxy, C₁₋₆-Halogenalkoxy, C₁₋₆-Alkylcarbonyl, C₁₋₆-Alkoxycarboxyl, C₁₋₆-Alkyl, C₁₋₆-Halogenalkyl, C₁₋₆₋Alkenyl oder C₁₋₆-Alkinyl bedeuten;
oder R₁₅₃ und R₁₅₈ bilden gemeinsam mit den Ringatomen, an die sie gebunden sind, einen fünf- oder sechsgliedrigen teilgesättigten oder ungesättigten Ring, der bis zu 2 gleiche oder verschiedene Heteroatome aus der Gruppe Sauerstoff, Schwefel und Stickstoff enthalten kann, wobei dieser Ring durch einen Rest Oxo substituiert sein kann;
enthält.

Bevorzugt enthalten die erfindungsgemäßen Mittel eine herbizid-antagonistisch wirksame Menge eines Safeners der Formel X, XI, XII, XIII, XIV, XV, XVI, XVII, XVIII, XIX, XX, XXI, XXII, XXIII, XXIV oder XXV.
Vorzugsweise enthält das erfindungsgemäße selektiv-herbizide Mittel als herbizid-antagonistisch wirksame Menge entweder eine Verbindung der Formel X 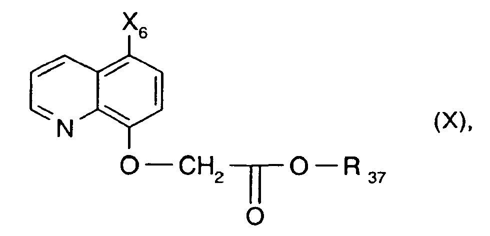 worin R₃₇ Wasserstoff, C₁-C₈-Alkyl oder durch C₁-C₆-Alkoxy oder C₃-C₆-Alkenyloxy substituiertes C₁-C₈-Alkyl; und X₆ Wasserstoff oder Chlor bedeutet; oder eine Verbindung der Formel XI 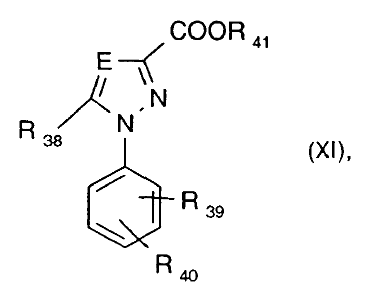 worin
E Stickstoff oder Methin; R₃₈ -CCl₃, Phenyl oder durch Halogen substituiertes Phenyl;
R₃₉ und R₄₀ unabhängig voneinander Wasserstoff oder Halogen; und
R₄₁ C₁-C₄-Alkyl bedeuten; oder eine Verbindung der Formel XII 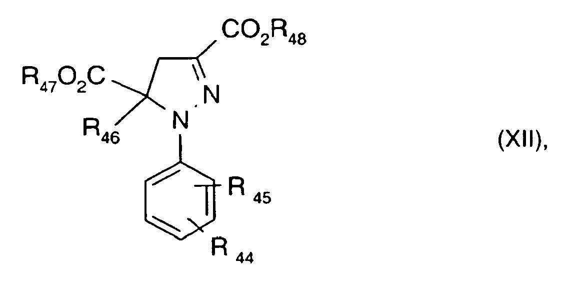 worin R₄₄ und R₄₅ unabhängig voneinander Wasserstoff oder Halogen und
R₄₆, R₄₇ und R₄₈ unabhängig voneinander C₁-C₄-Alkyl bedeuten.

Die oben genannten Bevorzugungen der Verbindungen der Formel I gelten auch bei Mischungen der Verbindungen der Formel I mit den Safenern der Formeln X bis XVIII. Bevorzugte erfindungsgemäße Mittel enthalten einen Safener ausgewählt aus der Gruppe der Formel Xa 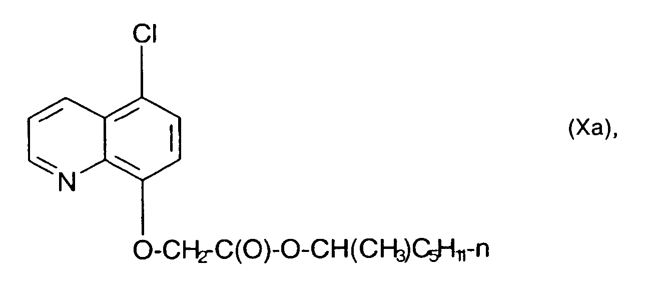 der Formel Xb 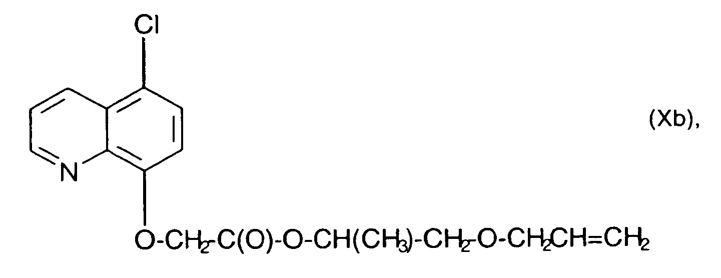 und der Formel Xla

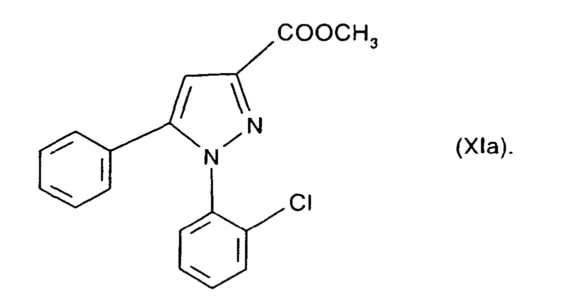

Weitere bevorzugte Verbindungen der Formeln X, XI und XII sind auch in den Tabellen 9, 10 und 11 aufgelistet.

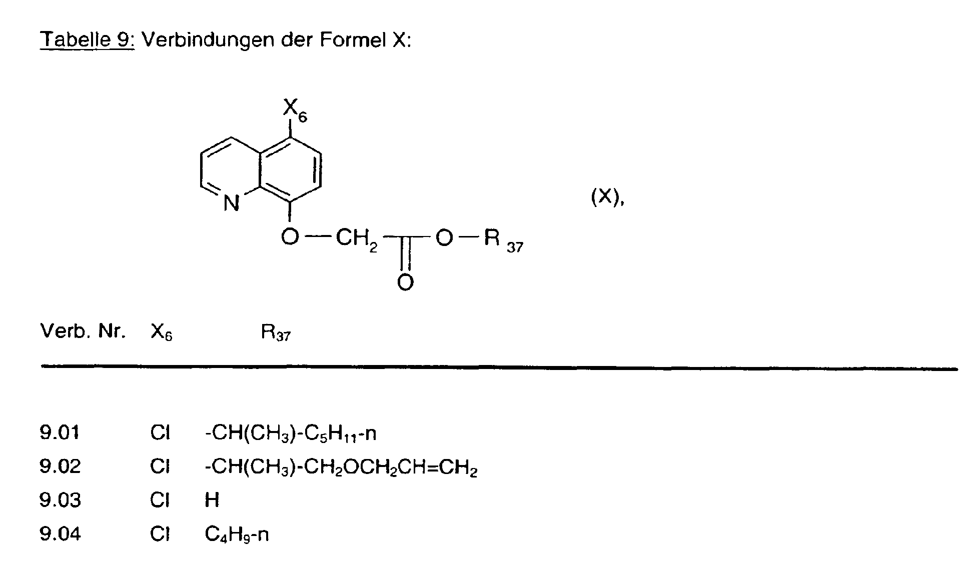

Bevorzugte Verbindungen der Formel XI sind in der folgenden Tabelle 10 aufgelistet.

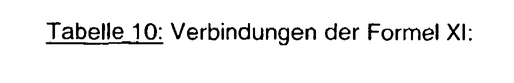

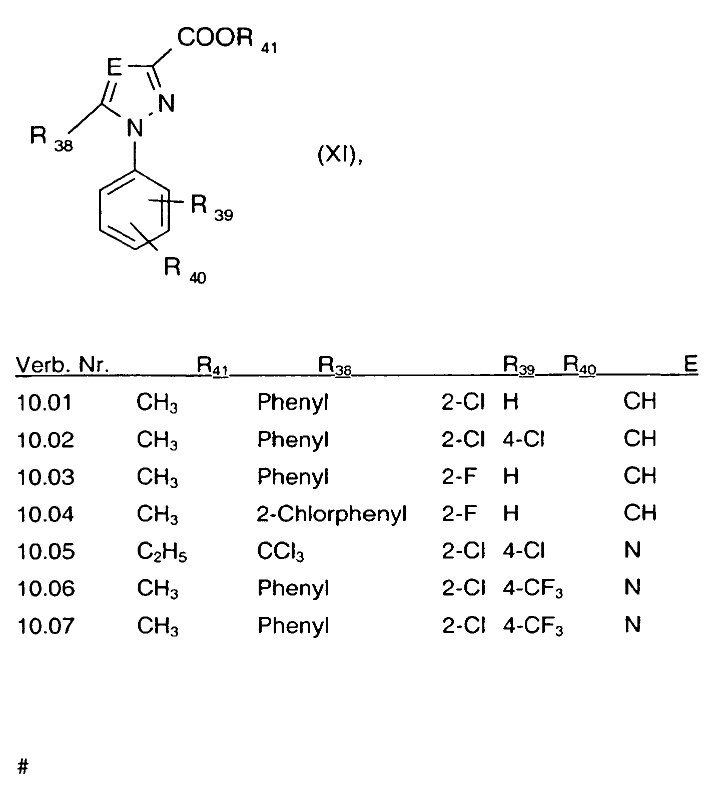

Bevorzugte Verbindungen der Formel XII sind in der folgenden Tabelle 11 aufgelistet.

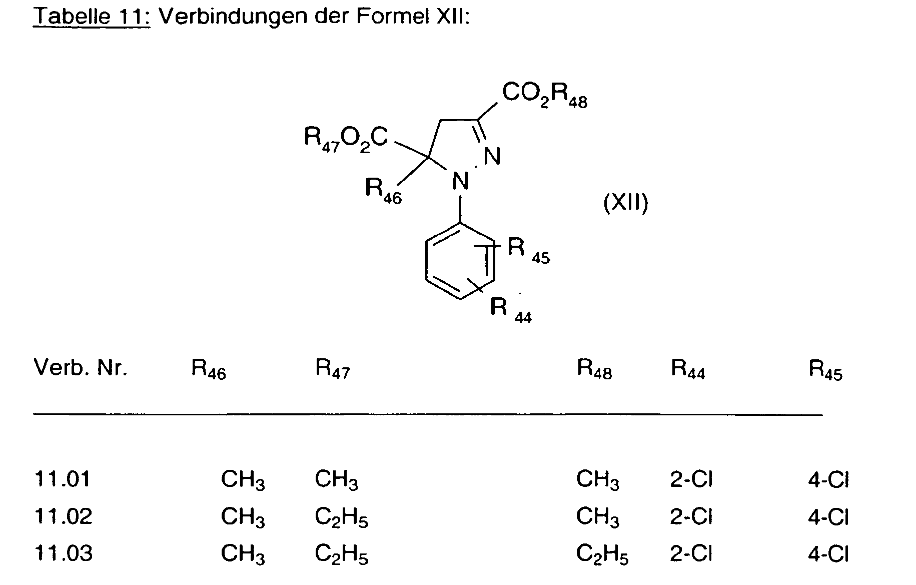

Bevorzugte Verbindungen der Formel XIII sind in der folgenden Tabelle 12 als Verbindungen der Formel Xllla aufgelistet:

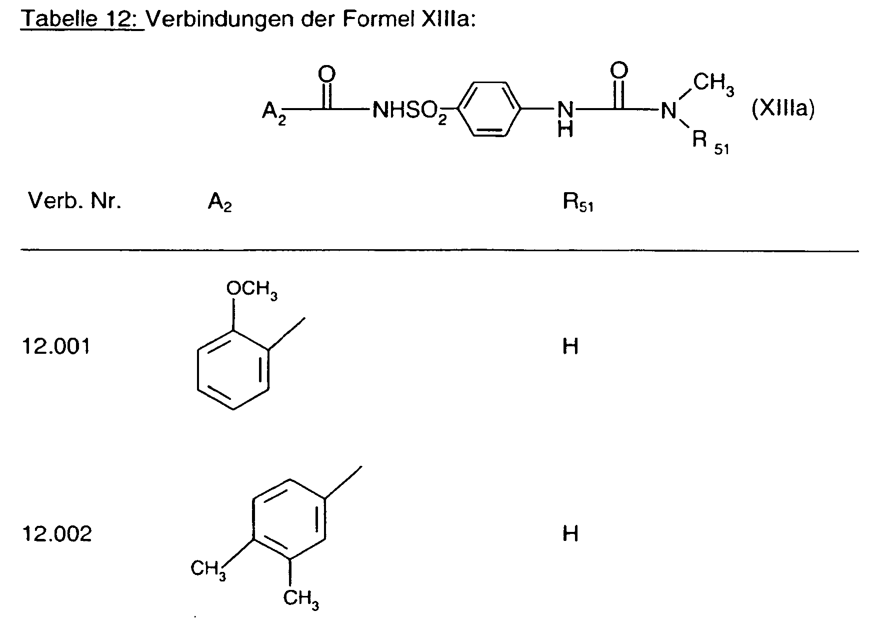

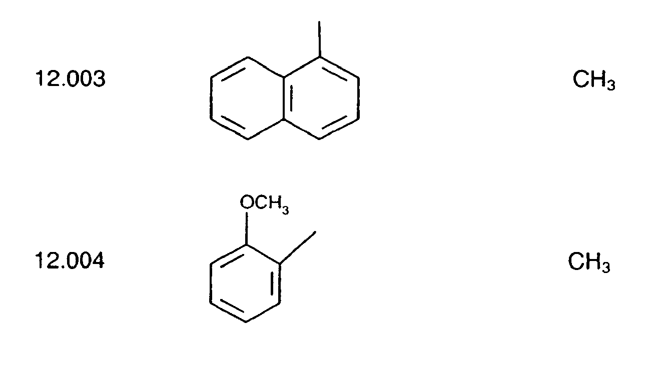

Bevorzugte Verbindungen der Formel XIV sind in der folgenden Tabelle 13 aufgelistet:

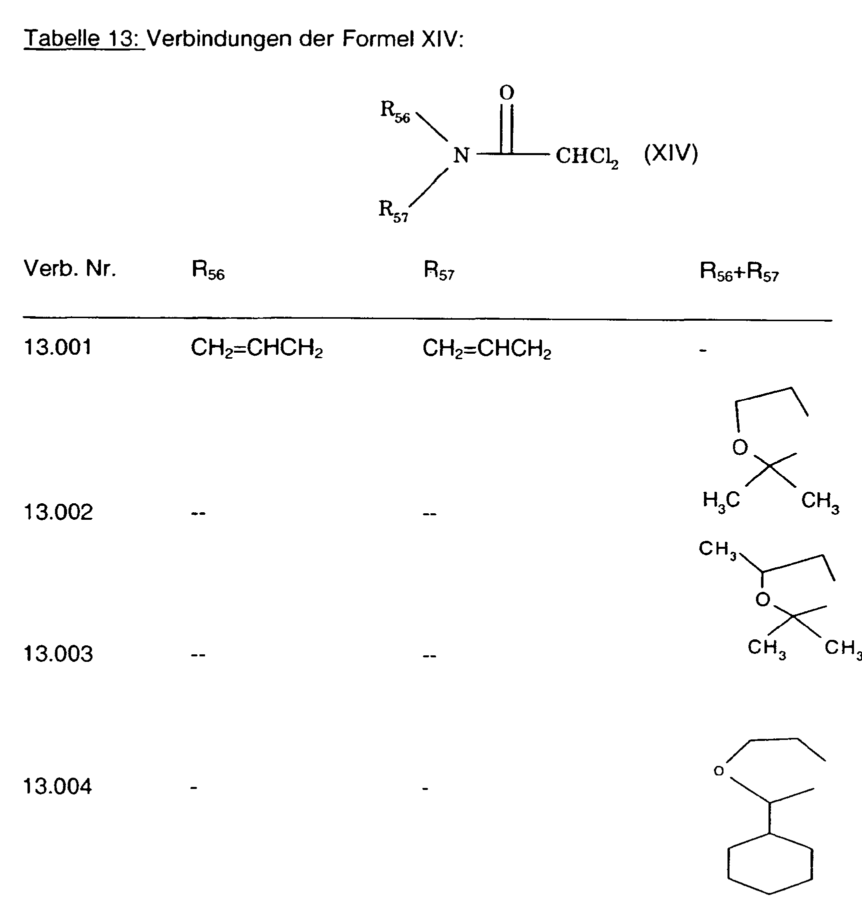

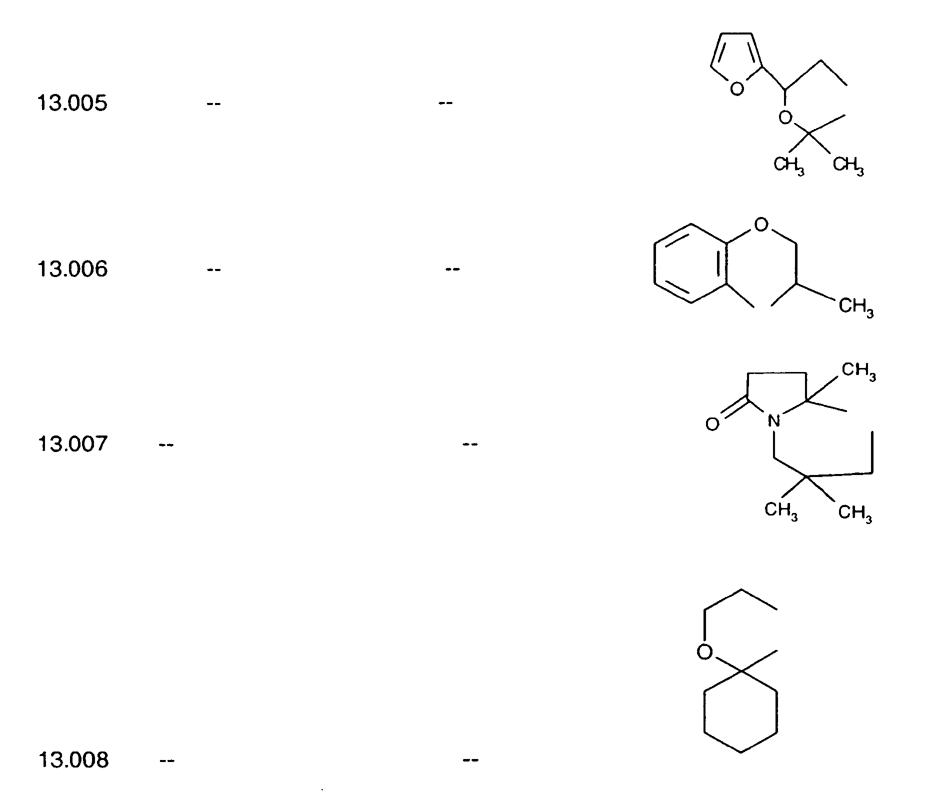

Bevorzugte Verbindungen der Formel XV sind in der folgenden Tabelle 14 aufgelistet:

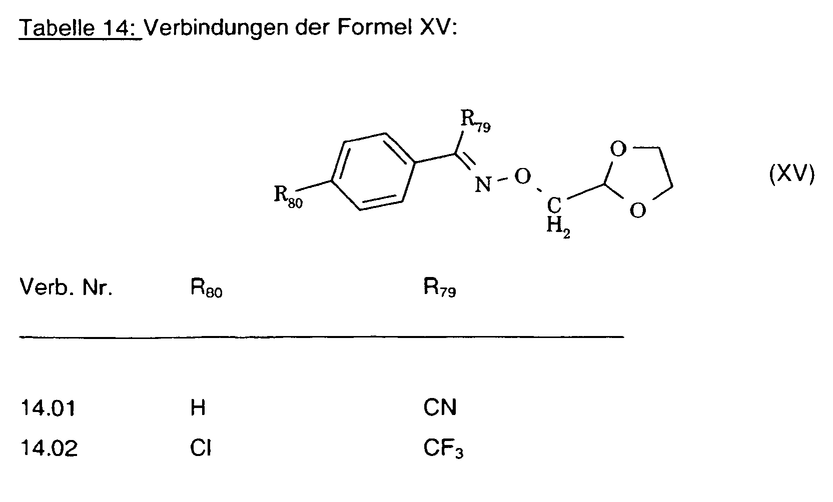

Bevorzugte Verbindungen der Formel XVI sind in der folgenden Tabelle 15 aufgelistet:

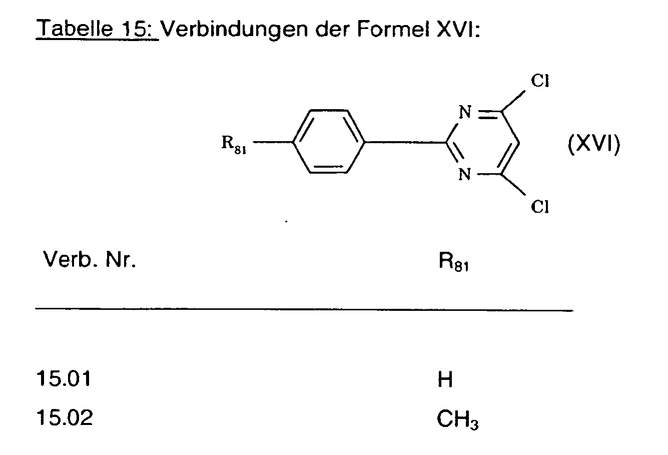

Bevorzugte Verbindungen der Formel XVII sind in der folgenden Tabelle 16 als Verbindungen der Formel XVlla aufgelistet:

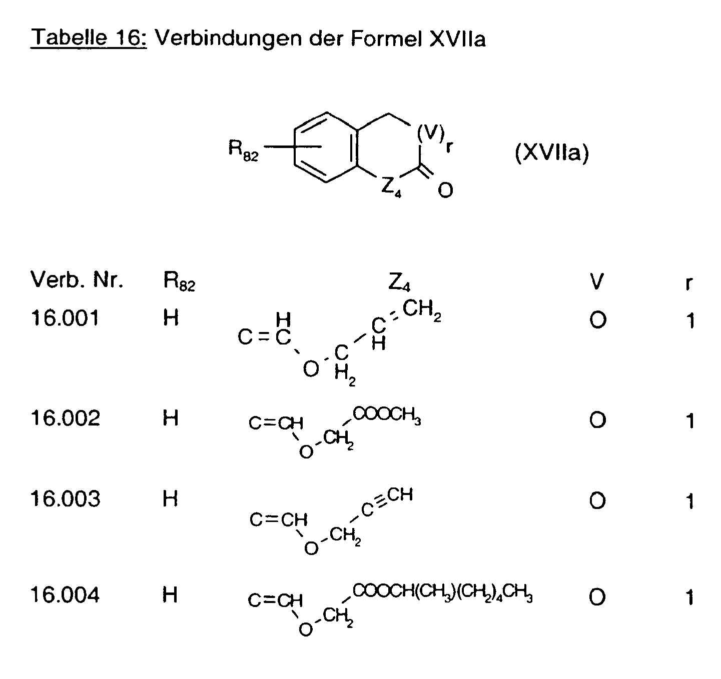

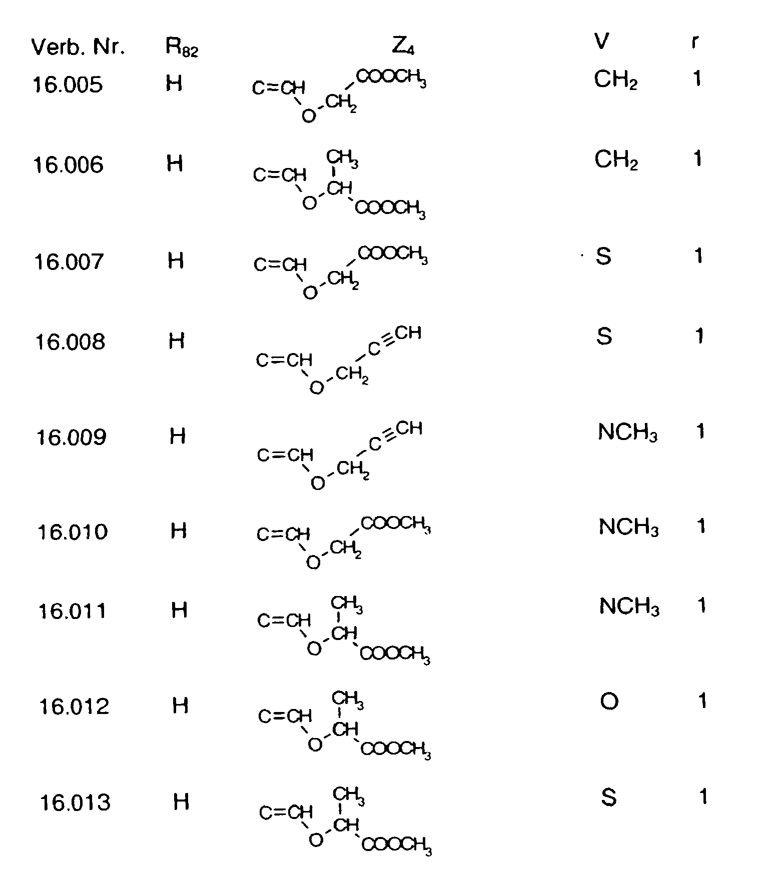

Bevorzugte Verbindungen der Formel XVII sind in der folgenden Tabelle 17 als Verbindungen der Formel XVIIb aufgelistet:

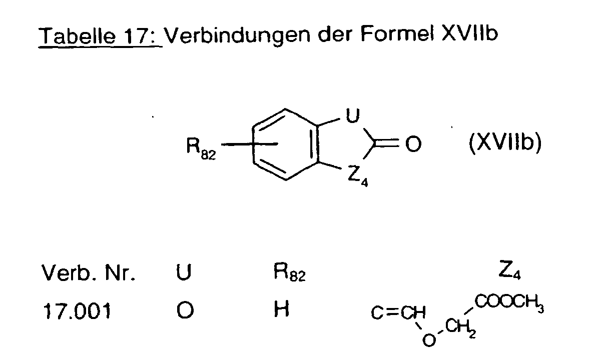

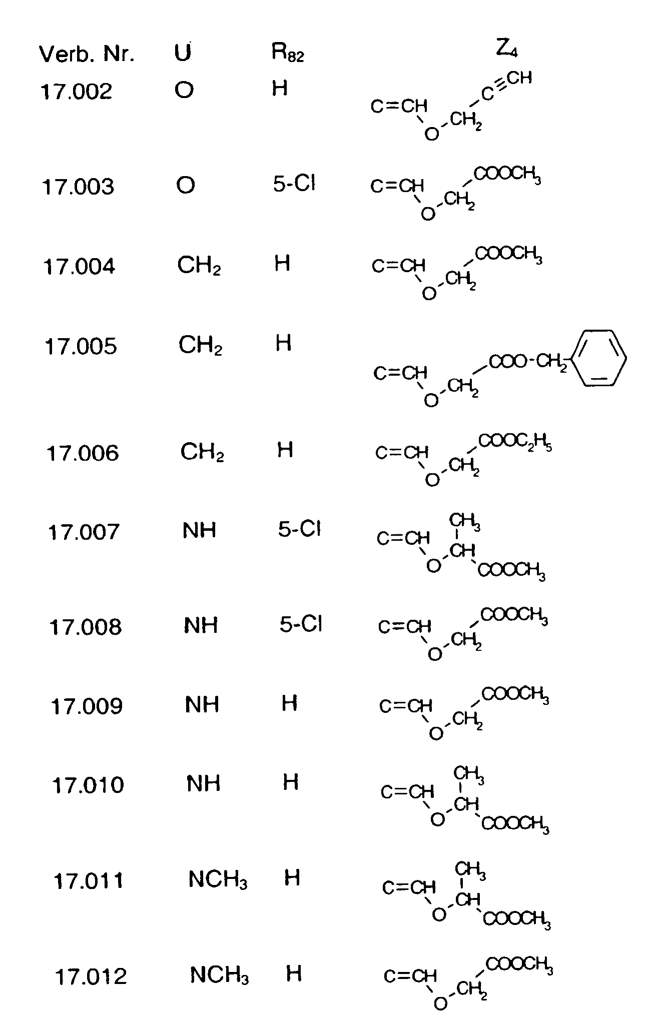

Bevorzugte Verbindungen der Formel XVII sind in der folgenden Tabelle 18 als Verbindungen der Formel XVIIc aufgelistet:

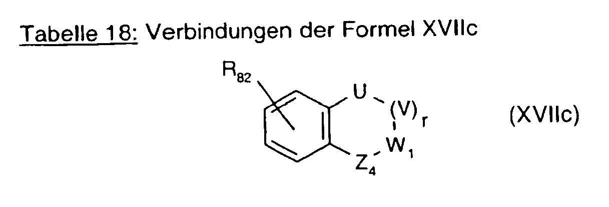

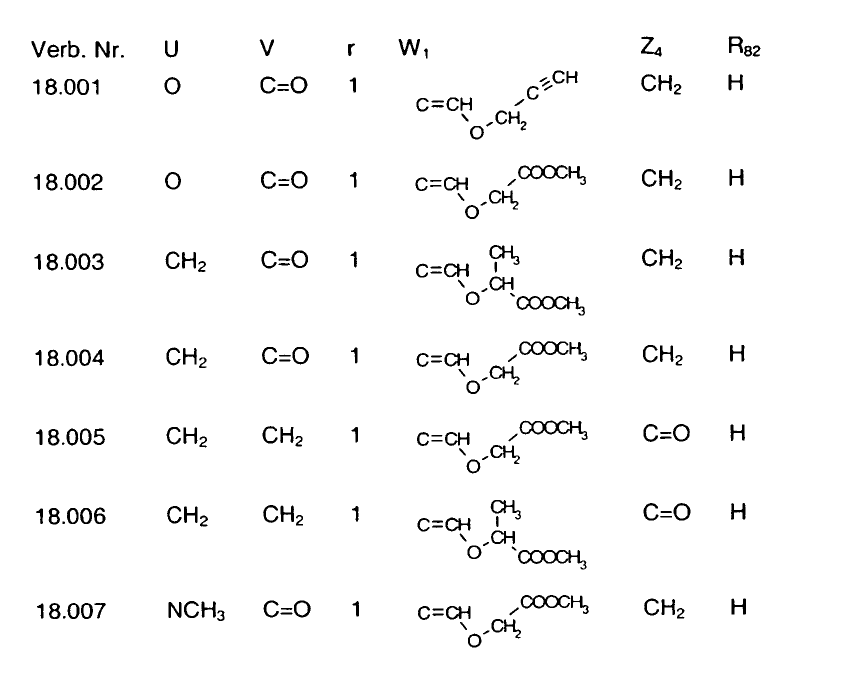

Bevorzugte Verbindungen der Formel XVII sind in der folgenden Tabelle 19 als Verbindungen der Formel XVlld aufgelistet:

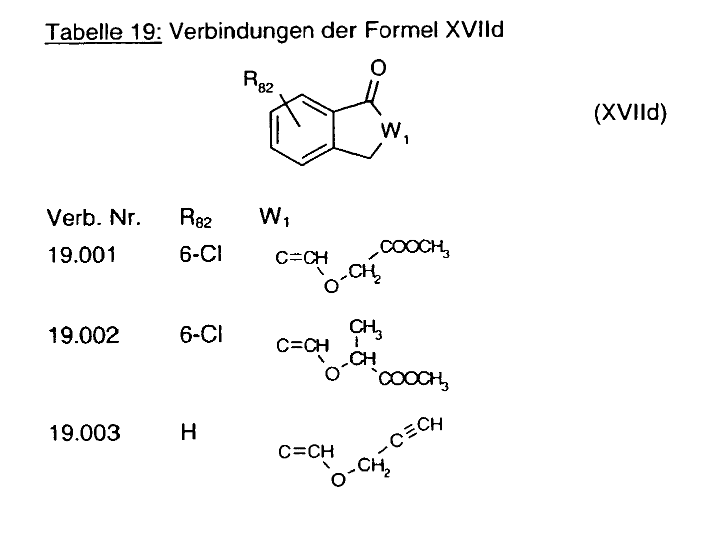

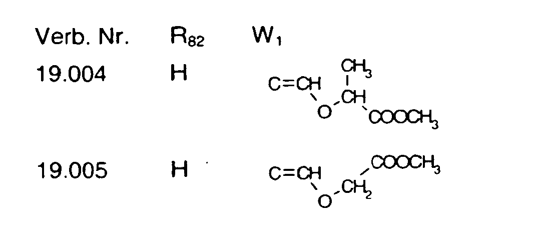

Bevorzugte Verbindungen der Formel XVIII sind in der folgenden Tabelle 20 aufgelistet:

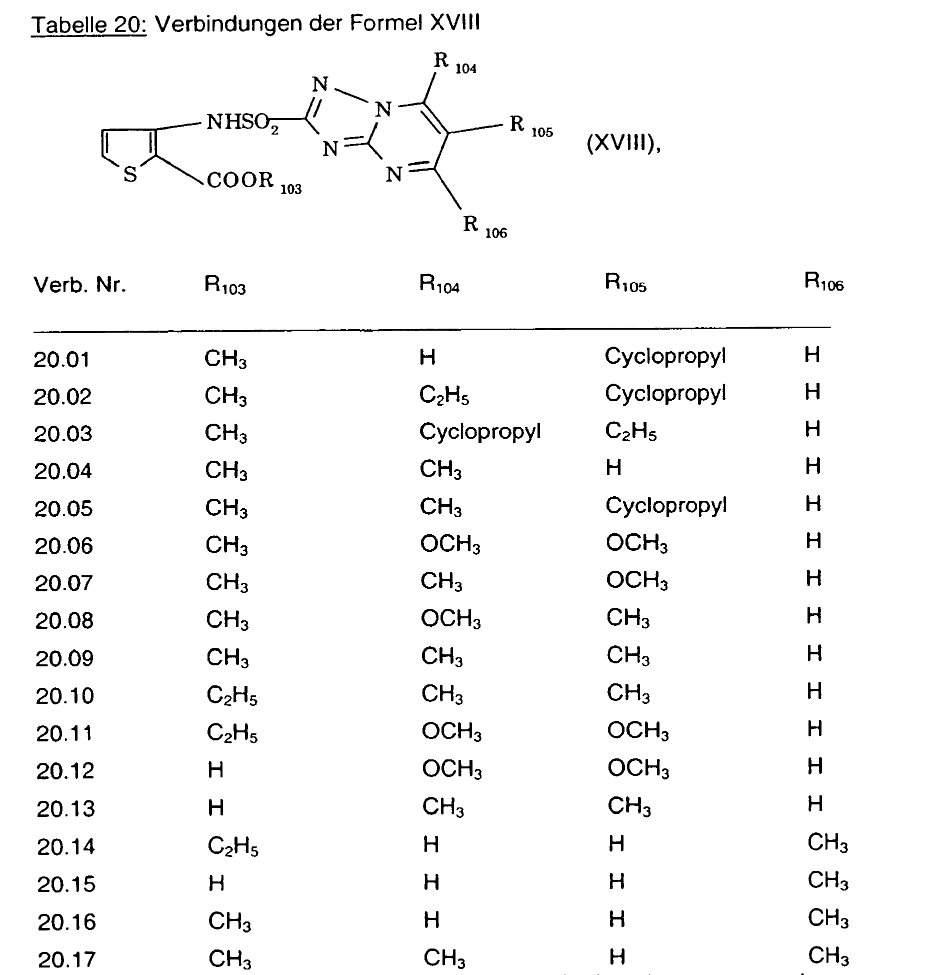

Von den Verbindungen der Formel XXVIII sind diejenigen bevorzugt, worin
R₁₄₈ Wasserstoff, C₁-C₆-Alkyl, C₃-C₈-Cycloalkyl oder Phenyl bedeutet, wobei die genannten Gruppen durch Halogen, Cyano, Nitro, Amino, Hydroxy, Carbonyl, Carboxyl Formyl, Carbonamid oder Sulfonamid substituiert sein können;
R₁₄₉ Wasserstoff bedeutet;
jedes R₁₅₀ unabhängig Wasserstoff, Halogen, C₁-C₄-Alkyl, C₁-C₄-Halogenalkyl, C₁-C₄-Alkoxy, C₁-C₄-Alkylthio, Cyano, Nitro oder Formyl bedeutet;
R₁₅₁ Wasserstoff bedeutet; und
jedes R₁₅₂ unabhängig Wasserstoff, Halogen, C₁-C₄-Alkyl, C₁-C₄-Halogenalkyl, C₁-C₄-Alkoxy, C₁-C₄-Alkylthio, Cyano, Nitro oder Formyl bedeutet.

Besonders bevorzugte Verbindungen der Formel XXVIII sind ausgewählt aus der Gruppe
2-Methoxy-N-[4-(2-methoxybenzoylsulfamoyl)phenyl]-acetamid,
N-[4-(2-Methoxybenzoylsulfamoyl)phenyl]-cyclopropancarboxamid,
N-[4-(2-Methoxy-benzoylsulfamoyl)-phenyl]- cyclobutancarboxamid,
N-[4-(2-Chlorobenzoylsulfamoyl)phenyl]-cyclopropancarboxamid,
N-[4-(2-Chloro-benzoylsulfamoyl)-phenyl]-acetamid,
N-[4-(2-Trifluoromethoxy-benzoylsulfamoyl)-phenyl]-acetamid,
N-[4-(2-Trifluormethylbenzoylsulfamoyl)phenyl]-cyclopropancarboxamid,
N-[4-(2-Trifluormethoxybenzoylsulfamoyl)phenyl]-cyclopropancarboxamid,
N-[4-(2-Trifluormethoxybenzoylsulfamoyl)phenyl]-cyclobutancarboxamid und
N-[4-(2-Trifluoromethyl-benzoylsulfamoyl)-phenyl]-acetamid.

Von den Verbindungen der Formel XXIX sind diejenigen bevorzugt, worin
R₁₅₉ Wasserstoff, Formyl. C₁-₆-Alkylcarbonyl, C₁₋₆-Alkenylcarbonyl, C₁₋₆-Alkinylcarbonyl, C₁₋₆₋Alkoxycarbonyl, C₁₋₆-Alkylthiocarbonyl, C₃₋₈-Cyccloalkylcarbonyl, Phenyl- C₁₋₆-alkylcarbonyl oder Phenylcarbonyl bedeutet, wobei die vorstehend genannten Kohlenwasserstoffreste durch ein oder mehrere Halogenatome, Cyano, Nitro, Amino, Methoxy, Ethoxy oder Phenyl substituiert sein können;
R₁₅₃ Wasserstoff, C₁₋₆-Alkyl, C₁₋₆-Alkenyl, C₁₋₆-Alkinyl, Formyl, C₁₋₆-Alkylcarbonyl oder C₁₋₆₋Alkoxycarbonyl bedeutet, wobei die vorstehend genannten Kohlenwasserstoffreste durch ein oder mehrere Halogenatome, Cyano, Nitro, Amino, Methoxy, Ethoxy oder Phenyl
substituiert sein können;
R₁₅₄ Wasserstoff, C₁₋₆-Alkyl, C₁₋₆-Alkenyl, C₁₋₆-Alkinyl, Formyl, C₁₋₆-Alkylcarbonyl oder C₁₋₆₋Alkoxycarbonyl bedeutet, wobei die vorstehend genannten Kohlenwasserstoffreste durch ein oder mehrere Halogenatome, Cyano, Nitro, Amino, Methoxy, Ethoxy oder Phenyl substituiert sein können;
R₁₅₅, R₁₅₆, R₁₅₇, und R₁₅₈ unabhängig voneinander Wasserstoff, Halogen, Cyano, Nitro, Formyl, Carboxyl, C₁₋₆-Alkoxy, C₁₋₆-Halogenalkoxy, C₁₋₆-Alkylcarbonyl, C₁₋₆-Alkoxycarboxyl, C₁₋₆-Alkyl oder C₁₋₆-Halogenalkyl bedeuten;
oder R₁₅₃ und R₁₅₈ bilden gemeinsam mit den Ringatomen, an die sie gebunden sind, einen fünf oder sechsgliedrigen teilgesättigten oder ungesättigten Ring, der bis zu 2 gleiche oder verschiedene Heteroatome aus der Gruppe Sauerstoff, Schwefel und Stickstoff enthalten kann, wobei dieser Ring durch einen Rest Oxo substituiert sein kann.

Besonders bevorzugte Verbindungen der Formel XXIX sind dadurch gekennzeichnet, daß R₁₅₉ Wasserstoff, Formyl, C₁₋₆-Alkylcarbonyl, C₁₋₆-Alkenylcarbonyl, C₁₋₆-Alkinylcarbonyl, C₁₋₆₋Alkoxycarbonyl, C₁₋₆-Alkylthiocarbonyl, C₃₋₈-Cyccloalkylcarbonyl oder Phenylcarbonyl
bedeutet;
R₁₅₃ Wasserstoff, C₁₋₆-Alkyl, C₁₋₆-Alkenyl, C₁₋₆-Alkinyl, Formyl, C₁₋₆-Alkylcarbonyl oder C₁₋₆₋Alkoxycarbonyl bedeutet;
R₁₅₄ : Wasserstoff, C₁₋₆-Alkyl, C₁₋₆-Alkenyl, C₁₋₆-Alkinyl, Formyl, C₁₋₆-Alkylcarbonyl oder C₁₋₆₋Alkoxycarbonyl bedeutet;
R₁₅₅, R₁₅₆, R₁₅₇, und R₁₅₈ unabhängig voneinander Wasserstoff, Halogen, Cyano, Nitro, Formyl, C₁₋₆-Alkyl, C₁₋₆-Halogenalkyl, C₁₋₆-Alkoxy oder C₁₋₆-Halogenalkoxy bedeuten;
oder R₁₅₃ und R₁₅₈ bilden gemeinsam mit den Ringatomen, an die sie gebunden sind, einen fünf oder sechsgliedrigen teilgesättigten oder ungesättigten Ring, der bis zu 2 gleiche oder verschiedene Heteroatome aus der Gruppe Sauerstoff, Schwefel und Stickstoff enthalten kann, wobei dieser Ring durch einen Rest Oxo substituiert sein kann.

Ganz besonders bevorzugte Verbindungen der Formel XXIX sind ausgewählt aus der Gruppe
4-Hydroxy-1-methyl-3-(1.H.-tetrazol-5-carbonyl)-1.H.-chinolin-2-on,
1-Ethyl-4-hydroxy-3-(1.H.-tetrazol-5-carbonyl)-1.H.-chinolin-2-on,
6-Hydroxy-5-(1.H.-tetrazol-5-carbonyl)-1,2-dihydro-pyrrolo[3,2,1-.ij.]chinolin-4-on,
3-(-Acetyl-1.H.-tetrazol-5-carbonyl)-4-hydroxy-1 -methyl-1.H.-chinolin-2-on,
6-Chloro-4-hydroxy-1-methyl-3-(1.H.-tetrazol-5-carbonyl)-1.H.-chinolin-2-on,
6-Fluoro-4-hydroxy-1-methyl-3-(1.H.-tetrazol-5-carbonyl)-1.H.-chinolin-2-on,
4-Hydroxy-1,6-dimethyl-3-(1.H.-tetrazol-5-carbonyl)-1.H.-chinolin-2-on,
4-Hydroxy-6-methoxy-1-methyl-3-(1.H.-tetrazol-5-carbonyl)-1.H.-chinolin-2-on,
4-Hydroxy-6-methoxy-1-methyl-3-(1.H.-tetrazole-5-carbonyl)-1.H.-chinolin-2-on,
Acetic acid 1-methyl-2-oxo-3-(1.H.-tetrazol-5-carbonyl)-1,2-dihydro-chinolin-4-yl ester und
2,2-Dimethyl-propionic acid 1-methyl-2-oxo-3-(1.H.-tetrazol-5-carbonyl)-1,2-dihydro-chinolin-4-yl ester.

Die Erfindung betrifft auch ein Verfahren zum selektiven Bekämpfen von Unkräutern in Nutzpflanzenkulturen, welches darin besteht, daß man die Nutzpflanzen, deren Samen oder Stecklinge oder deren Anbaufläche gleichzeitig oder getrennt mit einer herbizid wirksamen Menge des Herbizids der Formel I und einer herbizid-antagonistisch wirksamen Menge des Safeners der Formel X, XI, XII, XIII, XIV, XV, XVI, XVII, XVIII, XIX, XX, XXI, XXII, XXIII, XXIV, XXV, XXVI, XXVII, XXVIII oder XXIX, vorzugsweise der Formel X, XI, XII, XIII, XIV, XV, XVI, XVII oder XVIII behandelt.
Als Kulturpflanzen, welche durch die Safener der Formel X, XI, XII, XIII, XIV, XV, XVI, XVII oder XVIII gegen die schädigende Wirkung der oben erwähnten Herbizide geschützt werden können, kommen insbesondere Getreide, Baumwolle, Soja, Zuckerrüben, Zuckerrohr, Plantagen, Raps, Mais und Reis, ganz besonders in Mais und Getreide in Betracht. Unter Kulturen sind auch solche zu verstehen, die durch konventionelle züchterische oder gentechnologische Methoden gegen Herbizide bzw. Herbizidklassen tolerant gemacht worden sind.

Bei den zu bekämpfenden Unkräutern kann es sich sowohl um monokotyle wie um dikotyle Unkräuter handeln, wie zum Beispiel die monokotylen Unkräuter Avena, Agrostis, Phalaris, Lolium, Bromus, Alopecurus, Setaria, Digitaria, Brachiaria, Echinochloa, Panicum, Sorghum hal./bic., Rottboellia, Cyperus, Brachiaria, Echinochloa, Scirpus, Monochoria, Sagittaria, und Stellaria und die dikotylen Unkräuter Sinapis, Chenopodium, Galium, Viola, Veronica, Matricaria, Papaver, Solanum, Abutilon, Sida, Xanthium, Amaranthus, Ipomoea und Chrysanthemum.

Als Anbauflächen gelten die bereits mit den Kulturpflanzen bewachsenen oder mit dem Saatgut dieser Kulturpflanzen beschickten Bodenareale wie auch die zur Bebauung mit diesen Kulturpflanzen bestimmten Böden.

Ein Safener der Formel X, XI, XII, XIII, XIV, XV, XVI, XVII, XVIII, XIX, XX, XXI, XXII, XXIII, XXIV, XXV, XXVI, XXVII, XXVIII oder XXIX kann je nach Anwendungszweck zur Vorbehandlung des Saatgutes der Kulturpflanze (Beizung des Samens oder der Stecklinge) eingesetzt oder vor oder nach der Saat in den Boden gegeben werden. Er kann aber auch für sich allein oder zusammen mit dem Herbizid nach dem Auflaufen der Pflanzen appliziert werden. Die Behandlung der Pflanzen oder des Saatgutes mit dem Safener kann daher grundsätzlich unabhängig vom Zeitpunkt der Applikation des Herbizids erfolgen. Die Behandlung der Pflanze kann man jedoch auch durch gleichzeitige Applikation von Herbizid und Safener (z.B. als Tankmischung) vornehmen. Die zu applizierende Aufwandmenge Safener zu Herbizid richtet sich weitgehend nach der Anwendungsart. Bei einer Feldbehandlung, welche entweder unter Verwendung einer Tankmischung mit einer Kombination von Safener und Herbizid oder durch getrennte Applikation von Safener und Herbizid erfolgt, liegt in der Regel ein Verhältnis von Herbizid zu Safener von 100:1 bis 1:10, bevorzugt 20:1 bis 1:1, vor. In der Regel werden bei der Feldbehandlung 0,001 bis 1,0 kg Safener/ha, vorzugsweise 0,001 bis 0,25 kg Safener/ha, appliziert.

Die Aufwandmengen an Herbizid liegt in der Regel zwischen 0,001 bis 2 kg/ha, vorzugsweise jedoch zwischen 0,005 bis 0,5 kg/ha.

Die erfindungsgemäßen Mittel sind für alle in der Landwirtschaft üblichen Applikationsmethoden wie z.B. preemergente Applikation, postemergente Applikation und Saatbeizung geeignet.

Bei der Samenbeizung werden im allgemeinen 0,001 bis 10 g Safener/kg Samen, vorzugsweise 0,05 bis 2 g Safener/kg Samen, appliziert. Wird der Safener in flüssiger Form kurz vor der Aussaat unter Samenquellung appliziert, so werden zweckmäßigerweise Safenerlösungen verwendet, welche den Wirkstoff in einer Konzentration von 1 bis 10000, vorzugsweise von 100 bis 1000 ppm, enthalten.

Zur Applikation werden die Safener der Formel X, XI, XII, XIII, XIV, XV, XVI, XVII, XVIII, XIX, XX, XXI, XXII, XXIII, XXIV, XXV, XXVI, XXVII, XXVIII oder XXIX oder Kombinationen von diesen Safenern mit den Herbiziden der Formel I zweckmäßigerweise zusammen mit den in der Formulierungstechnik üblichen Hilfsmitteln zu Formulierungen verarbeitet, z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten oder Mikrokapseln.

Solche Formulierungen sind beispielsweise in der WO 97/34485auf den Seiten 9 bis 13 beschrieben. Die Formulierungen werden in bekannter Weise hergestellt, z.B. durch inniges Vermischen und/oder Vermahlen der Wirkstoffe mit flüssigen oder festen Formulierungshilfsmitteln wie z.B. Lösungsmitteln oder festen Trägerstoffen. Ferner können zusätzlich oberflächenaktive Verbindungen (Tenside) bei der Herstellung der Formulierungen verwendet werden. Für diesen Zweck geeignete Lösungsmittel und feste Trägerstoffe sind z.B. in der WO 97/34485auf der Seite 6 angegeben.

Als oberflächenaktive Verbindungen kommen je nach der Art des zu formulierenden Wirkstoffes der Formel I nichtionogene, kation- und/oder anionaktive Tenside und Tensidgemische mit guten Emulgier-, Dispergier- und Netzeigenschaften in Betracht. Beispiele für geeignete anionische, nichtionische und kationische Tenside sind beispielsweise in der WO 97/34485 auf den Seiten 7 und 8 aufgezählt. Ferner sind auch die in der Formulierungstechnik gebräuchlichen Tenside, die u.a. in "Mc Cutcheon's Detergents and Emulsifiers Annual" MC Publishing Corp., Ridgewood New Jersey, 1981, Stache, H., "Tensid-Taschenbuch", Carl Hanser Verlag, München/Wien, 1981 und M. und J. Ash, "Encyclopedia of Surfactants", Vol I-III, Chemical Publishing Co., New York, 1980-81 beschrieben sind, zur Herstellung der erfindungsgemäßen herbiziden Mittel geeignet.

Die herbiziden Formulierungen enthalten in der Regel 0,1 bis 99 Gew%, insbesondere 0,1 bis 95 Gew.-% Wirkstoffgemisch aus der Verbindung der Formel I mit den Verbindungen der Formeln X, XI, XII, XIII, XIV, XV, XVI, XVII, XVIII, XIX, XX, XXI, XXII, XXIII, XXIV, XXV, XXVI, XXVII, XXVIII oder XXIX, 1 bis 99,9 Gew.% eines festen oder flüssigen Formulierungshilfstoffes und 0 bis 25 Gew.%, insbesondere 0,1 bis 25 Gew.% eines Tensides. Während als Handelsware üblicherweise konzentrierte Mittel bevorzugt werden, verwendet der Endverbraucher in der Regel verdünnte Mittel.

Die Mittel können auch weitere Zusätze wie Stabilisatoren z.B. gegebenenfalls epoxydierte Pflanzenöle (epoxydiertes Kokosnußöl, Rapsöl oder Sojaöl), Entschäumer, z.B. Silikonöl, Konservierungsmittel, Viskositätsregulatoren, Bindemittel, Haftmittel sowie Dünger oder andere Wirkstoffe enthalten. Für die Verwendung von Safenern der Formeln X, XI, XII, XIII, XIV, XV, XVI, XVII, XVIII, XIX, XX, XXI, XXII, XXIII, XXIV, XXV, XXVI, XXVII, XXVIII oder XXIX oder sie enthaltender Mittel zum Schützen von Kulturpflanzen gegen schädigende Wirkungen von Herbiziden der Formell kommen verschiedene Methoden und Techniken in Betracht, wie beispielsweise die folgenden:

### i) Samenbeizung

a) Beizung der Samen mit einem als Spritzpulver formulierten Wirkstoff der Formeln X, XI, XII, XIII, XIV, XV, XVI, XVII, XVIII, XIX, XX, XXI, XXII, XXIII, XXIV, XXV, XXVI, XXVII, XXVIII oder XXIX, durch Schütteln in einem Gefäß bis zur gleichmäßigen Verteilung auf der Samenoberfläche (Trockenbeizung). Man verwendet dabei etwa 1 bis 500 g Wirkstoff der Formel X, XI, XII, XIII, XIV, XV, XVI, XVII, XVIII, XIX, XX, XXI, XXII, XXIII, XXIV, XXV, XXVI, XXVII, XXVIII oder XXIX (4 g bis 2 kg Spritzpulver) pro 100 kg Saatgut.
b) Beizung der Samen mit einem Emulsionskonzentrat des Wirkstoffs der Formel X, XI, XII, XIII, XIV, XV, XVI, XVII, XVIII, XIX, XX, XXI, XXII, XXIII, XXIV, XXV, XXVI, XXVII, XXVIII oder XXIX nach der Methode a) (Naßbeizung).
c) Beizung durch Tauchen des Saatguts in eine Brühe mit 100-1000 ppm Wirkstoff der Formel X, XI, XII, XIII, XIV, XV, XVI, XVII, XVIII, XIX, XX, XXI, XXII, XXIII, XXIV, XXV, XXVI, XXVII, XXVIII oder XXIX während 1 bis 72 Stunden und gegebenenfalls nachfolgendes Trocknen der Samen (Tauchbeizung).

Die Beizung des Saatguts oder die Behandlung des angekeimten Sämlings sind naturgemäß die bevorzugten Methoden der Applikation, weil die Wirkstoffbehandlung vollständig auf die Zielkultur gerichtet ist. Man verwendet in der Regel 1 bis 1000 g Antidot, vorzugsweise 5 bis 250 g Antidot, pro 100 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

### ii) Applikation als Tankmischung

Eine flüssige Aufarbeitung eines Gemisches von Antidot und Herbizid (gegenseitiges Mengenverhältnis zwischen 10:1 und 1:100) wird verwendet, wobei die Aufwandmenge an Herbizid 0,005 bis 5,0 kg pro Hektar beträgt. Solche Tankmischungen werden vor oder nach der Aussaat appliziert.

### iii) Applikation in der Saatfurche

Der Wirkstoff der Formel X, XI, XII, XIII, XIV, XV, XVI, XVII, XVIII, XIX, XX, XXI, XXII, XXIII, XXIV, XXV, XXVI, XXVII, XXVIII oder XXIX wird als Emulsionskonzentrat, Spritzpulver oder als Granulat in die offene besäte Saatfurche eingebracht. Nach dem Decken der Saatfurche wird in üblicher Weise das Herbizid im Vorauflaufverfahren appliziert.

### iv) Kontrollierte Wirkstoffabgabe

Der Wirkstoff der Formel X, XI, XII, XIII, XIV, XV, XVI, XVII, XVIII, XIX, XX, XXI, XXII, XXIII, XXIV, XXV, XXVI, XXVII, XXVIII oder XXIX wird in Lösung auf mineralische Granulatträger oder polymerisierte Granulate (Harnstoff/Formaldehyd) aufgezogen und getrocknet. Gegebenenfalls kann ein Überzug aufgebracht werden (Umhüllungsgranulate), der es erlaubt, den Wirkstoff über einen bestimmten Zeitraum dosiert abzugeben.

Erfindungsgemäße herbizide und den Pflanzenwuchs hemmende Mittel mit einem herbizid wirksamen Gehalt an Verbindung der Formel I sowie einem herbizid-antagonistisch wirksamen Menge an Verbindung der Formeln X, XI, XII, XIII, XIV, XV, XVI, XVII, XVIII, XIX, XX, XXI, XXII, XXIII, XXIV, XXV, XXVI, XXVII, XXVIII oder XXIX können durch Zugabe von Sprühtank-Adjuvantien in ihrer Wirksamkeit gesteigert werden.
Diese Adjuvantien können beispielsweise sein: nichtionische Tenside, Mischungen von nichtionischen Tensiden, Mischungen von anionischen Tensiden mit nichtionischen Tensiden, kationische Tenside, silizium-organische Tenside, Mineralölderivate mit und ohne Tenside , Pflanzenölderivate mit und ohne Tensidzusatz, alkylierte Derivate von Ölen pflanzlichen oder mineralischen Ursprungs mit und ohne Tenside, Fischöle und andere tierische Öle tierischer Natur sowie deren Alkylderivate mit und ohne Tenside, natürlich vorkommende höhere Fettsäuren, vorzugsweise mit 8 bis 28 Kohlenstoffatomen, und deren Alkylesterderivate, organische Säuren enthaltend ein aromatisches Ringsystem und einen oder mehrere Carbonsäurerester, sowie deren Alkylderivaten, ferner Suspensionen von Polymeren des Vinylacetats oder Copolymeren von Vinylacetat-Acrylsäureestern. Mischungen einzelner Adjuvantien untereinander sowie in Kombination mit organischen Lösungsmitteln können zu einer weiteren Steigerung der Wirkung führen.

Als nichtionische Tenside kommen beispielsweise Polyglykoletherderivate von aliphatischen oder cycloaliphatischen Alkoholen, gesättigten oder ungesättigten Fettsäuren und Alkylphenolen in Frage, vorzugsweise die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, vorzugsweise 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit vorzugsweise 1 bis 10 Kohlenstoffatomen in der Alkylkette. Die genannten Verbindungen enthalten üblicherweise pro Propylenglykol-Einheit 1 bis 5 Ethylenglykoleinheiten.

Als weitere Beispiele nichtionischer Tenside seien auch Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei anionischen Tensiden werden vor allem Alkylsulfate, Alkylsulfonate, Alkylarylsulfonate, alkylierte Phosphorsäuren bevorzugt sowie deren ethoxylierte Derivate. Die Alkylreste enthalten üblicherweise 8 bis 24 Kohlenstoffatome.

Bevorzugte nicht-ionische Tenside sind unter den folgenden Handelsnamen bekannt:

Polyoxyethylen Cocoalkylamin (z.B. AMIET® 105 (Kao Co.)), Polyoxyethylen Oleylamin (z.B. AMIET® 415 (Kao Co.)), Nonylphenolpolyethoxyethanole, Polyoxyethylen Stearylamin (z.B. AMIET® 320 (Kao Co.)), N-polyethoxyethylamines (z.B. GENAMIN® (Hoechst AG)), N,N,N',N'-Tetra(Polyethoxypolypropoxyethyl)ethylen-diamine (z.B. TERRONIL®und TETRONIC® (BASF Wyandotte Corp.)), BRIJ® (Atlas Chemicals), ETHYLAN® CD und ETHYLAN® D (Diamond Shamrock), GENAPOL® C, GENAPOL® O, GENAPOL® S und GENAPOL® X080 (Hoechst AG), EMULGEN® 104P, EMULGEN® 109P und EMULGEN® 408 (Kao Co.); DISTY® 125 (Geronazzo), SOPROPHOR® CY 18 (Rhone Poulenc S.A.); NONISOL® (Ciba-Geigy), MRYJ® (ICI); TWEEN® (ICI); EMULSOGEN® (Hoechst AG); AMIDOX® (Stephan Chemical Co.), ETHOMID® (Armak Co.); PLURONIC® (BASF Wyandotte Corp.), SOPROPHOR® 461 P (Rhône Poulenc S.A.), SOPROPHOR® 496/P (Rhone Poulenc S.A.), ANTAROX FM-63 (Rhone Poulenc S.A.), SLYGARD 309 (Dow Corning), SILWET 408, SILWET L-7607N (Osi-Specialities).

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi-(2-chlorethyl)-ethylammoniumbromid.

Die verwendeten Öle sind entweder von mineralischer oder natürlicher Herkunft. Die natürlichen Öle können zudemnoch von tierischem oder pflanzlichen Ursprung sein. Bei tierischen Ölen werden vor allem Derivate von Rindertalg bevorzugt, aber auch Fischöle (z.B. Sardinenöl) und deren Derivate werden verwendet. Pflanzliche Öle sind meist Saatöle verschiedener Herkunft. Als Beispiele für besonders verwendete Pflanzenöle können Kokos-, Raps- oder Sonneblumenöle sowie deren Derivate genannt werden.

In dem erfindungsgemäßen Mittel betragen die Aufwandmengen an Öladditiv in der Regel zwischen 0,01 und 2 % in Bezug auf die Spritzbrühe. Beispielsweise kann das Öladditv nach Herstellung der Spritzbrühe in der gewünschten Konzentration in den Sprühtank gegeben werden.

Im erfindungsgemäßen Mittel bevorzugte Öladditive enthalten ein Öl pflanzlichen Ursprungs wie beispielsweise Rapsöl oder Sonnenblumenöl, Alkylester von Ölen pflanzlichen Ursprungs wie beispielsweise die Methylderivate, oder Mineralöle.

Besonders bevorzugte Öladditive enthalten Alkylester von höheren Fettsäuren (C₈-C₂₂). insbesondere die Methylderivate von C₁₂-C₁₈ Fettsäuren, beispielsweise die Methylester der Laurinsäure, Palmitinsäure und Ölsäure. Diese Ester sind bekannt als Methyllaurat (CAS-111-82-0), Methylpalmitat (CAS-112-39-0) und Methyloleat (CAS-112-62-9).

Das Ausbringen und die Wirkung der Öladditive kann durch deren Kombination mit oberflächenaktiven Substanzen wie nichtionische-, anionische oder kationische Tenside verbessert werden. Beispiele für geeignete anionische, nichtionische und kationische Tenside sind in der WO 97/34485 auf den Seiten 7 und 8 aufgezählt.

Bevorzugte oberflächenaktive Substanzen sind anionische Tenside vom Typ der Dodecylbenzylsulfonate, insbesondere die Calciumsalze davon sowie nichtionische Tenside vom Typ der Fettalkoholethoxylate. Insbeondere bevorzugt sind ethoxylierte C₁₂-C₂₂₋Fettalkohole mit einem Ethoxylierungsgrad zwischen 5 und 40. Beispiele für kommerziell erhältliche, bevorzugte Tenside sind die Genapol Typen (Clariant AG, Muttenz, Schweiz).

Die Konzentration der oberflächenaktiven Substanzen in Bezug auf das gesamte Additiv beträgt im allgemeinen zwischen 1 und 30 Gew.%.

Beispiele für Öladditive, die aus Mischungen von Ölen bzw. Mineralölen oder deren Derivaten mit Tensiden bestehen, sind Edenor ME SU®, Emery 2231® (Henkel Tochtergesellschaft Cognis GMBH, DE), Turbocharge® (Zeneca Agro, Stoney Creek, Ontario , CA) oder, besonders bevorzugt, Actipron® (BP Oil UK Limited, GB).

Ferner kann die Zugabe eines organischen Lösungsmittels zu dem Öladditiv/Tensidgemisch eine weitere Steigerung der Wirkung bewirken. Geeignete Lösungsmittel sind beispielsweise Solvesso® (ESSO) oder Aromatic Solvent® (Exxon Corporation) Typen.
Die Konzentration derartiger Lösungsmittel kann von 10 bis 80 Gew.% des Gesamtgewichtes betragen.

Derartige Öladditive, die beispielsweise auch in US-A-4,834,908 beschrieben sind, sind für das erfindungsgemäße Mittel besonders bevorzugt. Ein ganz besonders bevorzugtes Öladditiv ist unter dem Namen MERGE® bekannt, kann von der BASF Corporation bezogen werden und ist beispielsweise in US-A-4,834,908 in col. 5, als Example COC-1 im wesentlichen beschrieben. Ein weiteres erfindungsgemäß bevorzugtes Öladditiv ist SCORE® (Novartis Crop Protection Canada.)

In der Formulier- und Adjuvanttechnik gebräuchliche Tenside, Öle, insbesondere Pflanzenöle, Derivate davon wie alkylierte Fettsäuren und Mischungen davon, z.B. mit vorzugsweise anionischen Tensiden wie alkylierten Phosphorsäuren, Alkylsulfate und Alkylarylsulfonaten sowie höheren Fettsäuren, die auch in den erfindungsgemäßen Mitteln und Sprühtanklösungen davon verwendet werden können, sind u.a. in "Mc Cutcheon's Detergents and Emulsifiers Annual" MC Publishing Corp., Ridgewood New Jersey, 1998, Stache, H., "Tensid-Taschenbuch", Carl Hanser Verlag, München/Wien, 1990, M. und J. Ash, "Encyclopedia of Surfactants", Vol I-IV, Chemical Publishing Co., New York, 1981-89, G. Kapusta, "A Compendium of Herbicide Adjuvants", Southern Illinois Univ. , 1998, L. Thomson Harvey, "A Guide to Agricultural Spray Adjuvants Used in the United States", Thomson Pubns., 1992 beschrieben.

Insbesondere setzen sich bevorzugte Formulierungen folgendermaßen zusammen: (% = Gewichtsprozent)

### Emulgierbare Konzentrate:

| | |
|---|---|
| Aktives Wirkstoffgemisch: | 1 bis 90 %, vorzugsweise 5 bis 20 % |
| oberflächenaktives Mittel: | 1 bis 30 %, vorzugsweise 10 bis 20 % |
| flüssiges Trägermittel: | 5 bis 94 %, vorzugsweise 70 bis 85 % |

### Stäube:

| | |
|---|---|
| Aktives Wirkstoffgemisch: | 0,1 bis 10 %, vorzugsweise 0,1 bis 5 % |
| festes Trägermittel: | 99,9 bis 90 %, vorzugsweise 99,9 bis 99 % |

### Suspensions-Konzentrate:

| | |
|---|---|
| Aktives Wirkstoffgemisch: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

### Benetzbare Pulver:

| | |
|---|---|
| Aktives Wirkstoffgemisch: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

### Granulate:

| | |
|---|---|
| Aktives Wirkstoffgemisch: | 0,1 bis 30 %, vorzugsweise 0,1 bis 15 % |
| festes Trägermittel: | 99,5 bis 70 %, vorzugsweise 97 bis 85 % |

Die folgenden Beispiele erläutern die Erfindung weiter, ohne sie zu beschränken.

### Formulierungsbeispiele für Mischungen aus Herbiziden der Formel I und Safener der Formel X, XI, XII, XIII, XIV, XV, XVI, XVII, XVIII, XIX, XX, XXI, XXII, XXIII, XXIV, XXV, XXVI, XXVII, XXVIII oder XXIX (% = Gewichtsprozent)

| F1. Emulsionskonzentrate | a) | b) | c) | d) |
|---|---|---|---|---|
| Wirkstoffgemisch | 5 % | 10 % | 25 % | 50 % |
| Ca-Dodecylbenzolsulfonat | 6 % | 8 % | 6 % | 8 % |
| Ricinusöl-polyglykolether (36 Mol EO) | 4 % | - | 4 % | 4 % |
| Octylphenol-polyglykolether (7-8 Mol EO) | - | 4 % | - | 2 % |
| Cyclohexanon | - | - | 10 % | 20 % |
| Arom. Kohlenwasserstoffgemisch C₉-C₁₂ | 85 % | 78% | 55% | 16 % |

Aus solchen Konzentraten können durch Verdünnung mit Wasser Emulsionen jeder gewünschten Konzentration hergestellt werden.

| F2. Lösungen | a) | b) | c) | d) |
|---|---|---|---|---|
| Wirkstoffgemisch | 5% | 10 % | 50 % | 90 % |
| 1-Methoxy-3-(3-methoxypropoxy)-propan | - | 20 % | 20 % | - |
| Polyethylenglykol MG 400 | 20 % | 10 % | - | - |
| N-Methyl-2-pyrrolidon | - | - | 30 % | 10 % |
| Arom. Kohlenwasserstoffgemisch C₉-C₁₂ | 75 % | 60 % | - | - |

Die Lösungen sind zur Anwendung in Form kleinster Tropfen geeignet.

| F3. Spritzpulver | a) | b) | c) | d) |
|---|---|---|---|---|
| Wirkstoffgemisch | 5 % | 25 % | 50 % | 80 % |
| Na-Ligninsulfonat | 4 % | - | 3% | - |
| Na-Laurylsulfat | 2 % | 3 % | - | 4 % |
| Na-Diisobutyl-naphthalinsulfonat | - | 6 % | 5 % | 6 % |
| Octylphenol-polyglykolether (7-8 Mol EO) | - | 1 % | 2 % | - |
| Hochdisperse Kieselsäure | 1% | 3 % | 5 % | 10 % |
| Kaolin | 88% | 62 % | 35 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F4. Umhüllungs-Granulate | a) | b) | c) |
|---|---|---|---|
| Wirkstoffgemisch | 0.1 % | 5% | 15 % |
| Hochdisperse Kieselsäure | 0.9 % | 2 % | 2 % |
| Anorg. Trägermaterial (Æ 0.1 - 1 mm) | 99.0 % | 93 % | 83% |

wie z.B. CaCO₃ oder SiO₂
Der Wirkstoff wird in Methylenchlorid gelöst, auf den Träger aufgesprüht und das Lösungsmittel anschließend im Vakuum abgedampft.

| F5. Umhüllungs-Granulate | a) | b) | c) |
|---|---|---|---|
| Wirkstoffgemisch | 0.1 % | 5 % | 15 % |
| Polyethylenglykol MG 200 | 1.0 % | 2 % | 3 % |
| Hochdisperse Kieselsäure | 0.9 % | 1 % | 2 % |
| Anorg. Trägermaterial (Æ 0.1 - 1 mm) | 98.0 % | 92 % | 80 % |

wie z. B. CaCO₃ oder SiO₂

Der fein gemahlene Wirkstoff wird in einem Mischer auf das mit Polyethylenglykol angefeuchtete Trägermaterial gleichmäßig aufgetragen. Auf diese Weise erhält man staubfreie Umhüllungs-Granulate.

| F6. Extruder-Granulate | a) | b) | c) | d) |
|---|---|---|---|---|
| Wirkstoffgemisch | 0.1 % | 3 % | 5 % | 15 % |
| Na-Ligninsulfonat | 1.5 % | 2 % | 3 % | 4 % |
| Carboxymethylcellulose | 1.4 % | 2 % | 2 % | 2 % |
| Kaolin | 97.0 % | 93 % | 90 % | 79 % |

Der Wirkstoff wird mit den Zusatzstoffen vermischt, vermahlen und mit Wasser angefeuchtet. Dieses Gemisch wird extrudiert und anschließend im Luftstrom getrocknet.

| F7. Stäubemittel | a) | b) | c) |
|---|---|---|---|
| Wirkstoffgemisch | 0.1 % | 1 % | 5 % |
| Talkum | 39.9 % | 49 % | 35 % |
| Kaolin | 60.0 % | 50 % | 60 % |

Man erhält anwendungsfertige Stäubemittel, indem der Wirkstoff mit den Trägerstoffen vermischt und auf einer geeigneten Mühle vermahlen wird.

| F8. Suspensions-Konzentrate | a) | b) | c) | d) |
|---|---|---|---|---|
| Wirkstoffgemisch | 3% | 10 % | 25% | 50 % |
| Ethylenglykol | 5% | 5% | 5% | 5% |
| Nonylphenol-polyglykolether (15 Mol EO) | - | 1 % | 2 % | - |
| Na-Ligninsulfonat | 3% | 3% | 4% | 5% |
| Carboxymethylcellulose | 1 % | 1 % | 1 % | 1 % |
| 37%ige wäßrige Formaldehyd-Lösung | 0.2 % | 0.2 % | 0.2 % | 0.2 % |
| Silikonöl-Emulsion | 0.8 % | 0.8 % | 0.8 % | 0.8 % |
| Wasser | 87% | 79% | 62 % | 38% |

Der feingemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Es ist oft praktischer, den Wirkstoff der Formel I und den Mischungspartner der Formel X, XI, XII, XIII, XIV, XV, XVI, XVII, XVIII, XIX, XX, XXI, XXII, XXIII, XXIV, XXV, XXVI, XXVII, XXVIII oder XXIX einzeln zu formulieren und sie dann kurz vor dem Ausbringen im Applikator im gewünschten Mischungsverhältnis als "Tankmischung" im Wasser zusammenzubringen.

Die Fähigkeit der Safener der Formel X, XI, XII, XIII, XIV, XV, XVI, XVII, XVIII, XIX, XX, XXI, XXII, XXIII, XXIV, XXV, XXVI, XXVII, XXVIII oder XXIX Kulturpflanzen vor der phytotoxischen Wirkung von Herbiziden der Formel I zu schützen, wird in den folgenden Beispielen veranschaulicht.

### Biologisches Beispiel 1: Safeningwirkung

Unter Gewächshausbedingungen werden die Testpflanzen in Kunstofftöpfen bis zum 4-Blattstadium angezogen. In diesem Stadium werden zum einen das Herbizid allein, als auch die Mischungen des Herbizids mit den als Safener zu prüfenden Testsubstanzen auf die Testpflanzen appliziert. Die Applikation erfolgt als wäßrige Suspension der Prüfsubstanzen, hergestellt aus einem 25 %igen Spritzpulver (Beispiel F3, b)), mit 500 I Wasser/ha. 2 bis 3 Wochen nach Applikation wird die phytotoxische Wirkung des Herbizids auf die Kulturpflanzen wie z.B. Mais und Getreide mit einer Prozentskala ausgewertet. 100 % bedeutet Testpflanze ist abgestorben, 0 % bedeutet keine phytotoxische Wirkung.

Die in diesem Versuch erhaltenen Resultate zeigen, daß mit den Verbindungen der Formel X, XI, XII, XIII, XIV, XV, XVI, XVII, XVIII, XIX, XX, XXI, XXII, XXIII, XXIV, XXV, XXVI, XXVII, XXVIII oder XXIX die durch das Herbizid der Formel I verursachten Schädigungen der Kulturpflanze deutlich reduziert werden können.

Die Verbindung der Formel I läßt sich mit Vorteil mit einer Reihe von weiteren bekannten Herbiziden mischen. Man erhält dadurch beispielsweise eine wesentliche Verbreiterung des Unkrautspektrums und in vielen Fällen auch eine Erhöhung der Selektivität bezüglich der Nutzpflanzen. Insbesondere sind die Mischungen der Verbindung der Formel I mit mindestens einem der folgenden Herbizide von Bedeutung:
Herbizide aus der Klasse der Phenoxy-phenoxypropionsäuren wie beispielsweise Diclofopmethyl, Fluazifop-P-butyl- Quizalafop-P-ethyl, Propaquizafop, Clodinafop-P-propargyl, Cyhalfop-butyl, Fenoxaprop-P-Ethyl, Haloxyfop-methyl oder Haloxyfop-etoethyl;
Herbizide aus der Klasse der Hydroxylamine wie z.B. Sethoxidim, Alloxydim, Clethodim, Cycloxydim, Tepralkoxydim, Tralkoxydim oder Butroxidim;
Herbizide aus der Klasse der Sulfonylharnstoffe, wie z.b. Amidosulfuron, Azimsulfuron, Bensulfuron-methyl, Chlorimuron-ethyl, Cinosulfuron, Chlorsulfuron, Chlorimuron, Cyclosulfamuron, Ethametsulfuron-methyl, Ethoxysulfuron, Fluazasulfuron, Flupyrsulfuron, Imazosulfuron, lodosulfuron (CAS RN 144550-36-7 und 185119-76-0), Metsulfuron-methyl, Nicosulfuron, Oxasulfuron, Primisulfuron, Pyrazosulfuron-ethyl, Sulfosulfuron, Rimsulfuron, Thifensulfuron-methyl, Triasulfuron, Tribenuron-mehtyl, Triflusulfuron-methyl, Prosulfuron, Flucarbazon oder Tritosulfuron (CAS RN 142469-14-5);
Herbizide aus der Klasse der Imidazolinone, wie Imazethapyr, Imazamethabenz, Imazamethapyr, Imazaquin, Imazamox oder Imazapyr;
Herbizide aus der Klasse der Pyrimidine, wie Pyrithiobac-sodium, Pyriminobac, Bispyribacsodium;
Herbizide aus der Klasse der Triazine, wie z.B. Atrazin, Simazin, Simethryne, Terbutryne, Terbuthylazine;
Herbizide aus der Klasse der Harnstoffe, wie Isoproturon, Chlortoluron, Diuron, Dymron, Fluometuron, Linuron oder Methabenzthiazuron;
   Herbizide aus der Klasse der Phosphonsäurederivate, wie z.B. Glyphosate, Glufosinate, Sulfosate oder Phosphinothricin;
Herbizide aus der Klasse der PPO, wie z.B. Nitrofen, Bifenox, Acifluorfen, Lactofen, Oxyfluorfen, Ethoxyfen, Fluoroglycofen, Fomesafen, Halosafen, Azafenidin (CAS RN. - 68049-83-2), Benzfendizone (CAS RN 158755-95-4), Butafenacil (bekannt aus US-A-5,183,492,
   CAS RN 158755-95-4), Carfentrazone-ethyl, Cinidon-ethyl (CAS RN 142891-20-1), Flumichlorac-pentyl, Flumioxazin, Fluthiacet-methyl, Oxadiargyl, Oxadiazon, Pentoxazon, Sulfentrazone, Fluazolate (CAS RN 174514-07-9) oder Pyraflufen-ethyl;
Herbizide aus der Klasse der Chloracetanilide wie z.B. Alachlor, Acetochlor, Butachlor, Dimethachlor, Dimethenamid, S-Dimethenamid, Metazachlor, Metolachlor, S-Metolachlor, Pretilachlor, Propachlor, Propisochlor, Thenylchlor oder Pethoamid (CAS RN 106700-29-2)
Herbizide aus der Klasse der Phenoxyessigsäuren wie z.B. 2,4-D, Fluroxypyr, MCPA, MCPP, MCPB, Trichlorpyr oder Mecropop-P;
Herbizide aus der Klasse der Triazinone wie z.B. Hexazinon, Metamitron oder Metribuzin;
Herbizide aus der Klasse der Dinitroaniline wie z.B. Oryzalin, Pendimethalin oder Trifluralin;
Herbizide aus der Klasse der Azinone wie z.B. Chloridazon oder Norflurazon;
Herbizide aus der Klasse der Carbamate, wie z.B. Chlorpropham, Desmedipham, Phenmedipham oder Propham;
Herbizide aus der Klasse der Oxyacetamide wie z.B. Mefenacet oder Fluthiacet;
Herbizide aus der Klasse der Thiolcarbamate wie z.B. Butylate, Cycloate, Diallate, EPTC, Esprocarb, Molinate, Prosulfocarb, Thiobencarb oder Triallate;
Herbizide aus der Klasse der Azoloharnstoffe wie z.B. Fentrazamide (CAS RN158237-07-1) oder Cafenstrole;
Herbizide aus der Klasse der Benzoesäuren wie z.B. Dicamba oder Picloram;
Herbizide aus der Klasse der Anilide wie z.B. Diflufenican, oder Propanil;
Herbizide aus der Klasse der Nitrile wie z.B. Bromoxynil, Dichlobenil oder loxynil;
Herbizide aus der Klasse der Trione wie z.B. Sulcotrione, Mesotrione (bekannt aus US-A-5,006,158), Isoxaflutole oder Isoxachlortole;
Herbizide aus der Klasse der Sulfonamide wie z.B. Flucarbazone (CAS RN 181274-17-9), Procarbazone (CAS RN 145026-81-9), Chlorasulam, Diclosulam (CAS RN 145701-21-9), Florasulam, Flumetsulam oder Metosulam;
sowie Amitrol, Benfuresate, Bentazone, Cinmethylin, Clomazone, Chlopyralid, Difenzoquat, Dithiopyr, Ethofumesate, Flurochloridone, Indanofane, Isoxaben, Oxaziclomefone, Pyridate, Pyridafol (CAS RN. 40020-01-7), Quinchlorac, Quinmerac, Tridiphane oder Flamprop.

Wenn nicht anders angegeben, sind die oben angegebenen Mischungspartner der Verbindung der Formel I aus The Pesticide Manual, Eleventh Edition, 1997, BCPC bekannt. Die Mischungspartner der Verbindung der Formel I können gegebenenfalls auch in Form von Estern oder Salzen vorliegen, wie sie z. B. in The Pesticide Manual, Eleventh Edition, 1997, BCPC, genannt sind.

In den folgenden Tabellen sind die Schmelzpunkte in °C angegeben. Me bedeutet die Methylgruppe. Ist für die Substituenten G₁₉ und G₁₀ eine Formel dargestellt, so ist die linke Seite dieser Formel der Verknüpfungspunkt mit dem Sauerstoffatom des Heterocyclus Q₉ oder Q₁₀. Die übrigen endständigen Valenzen stellen Methylgruppen dar.

In den folgenden Tabellen bedeutet " LC/MS: M+" das positiv geladene molekulare Ion in daltons exprimiert, das aus dem Massenspektrum eruiert wurde bei der Analyse des Produktes mit gekoppelten HPLC (High Performance Liquid Chromatography) und MS (Mass Spectrometry) Geräten.

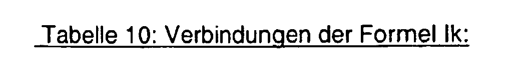

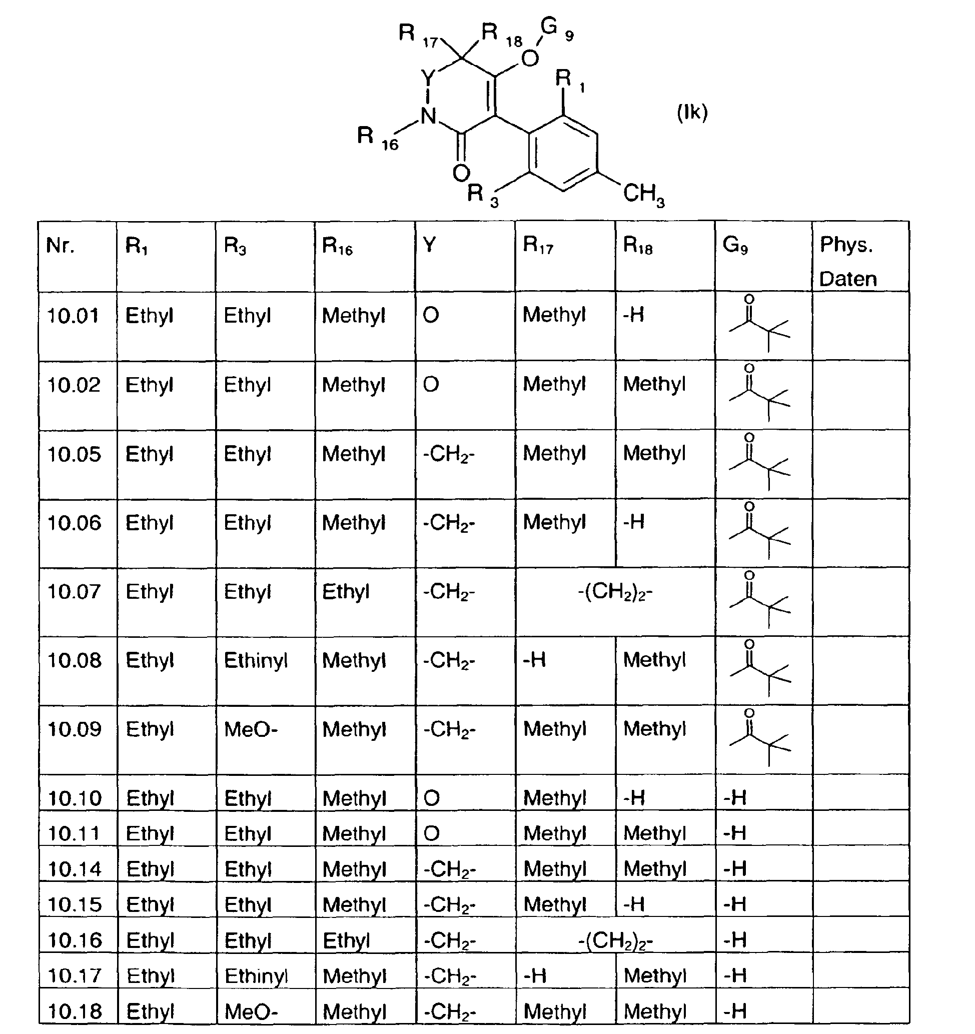

In der folgenden Tabelle 21 steht Me für Methyl, Et für Ethyl, Pr für Propyl und Bu für Butyl:

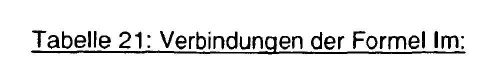

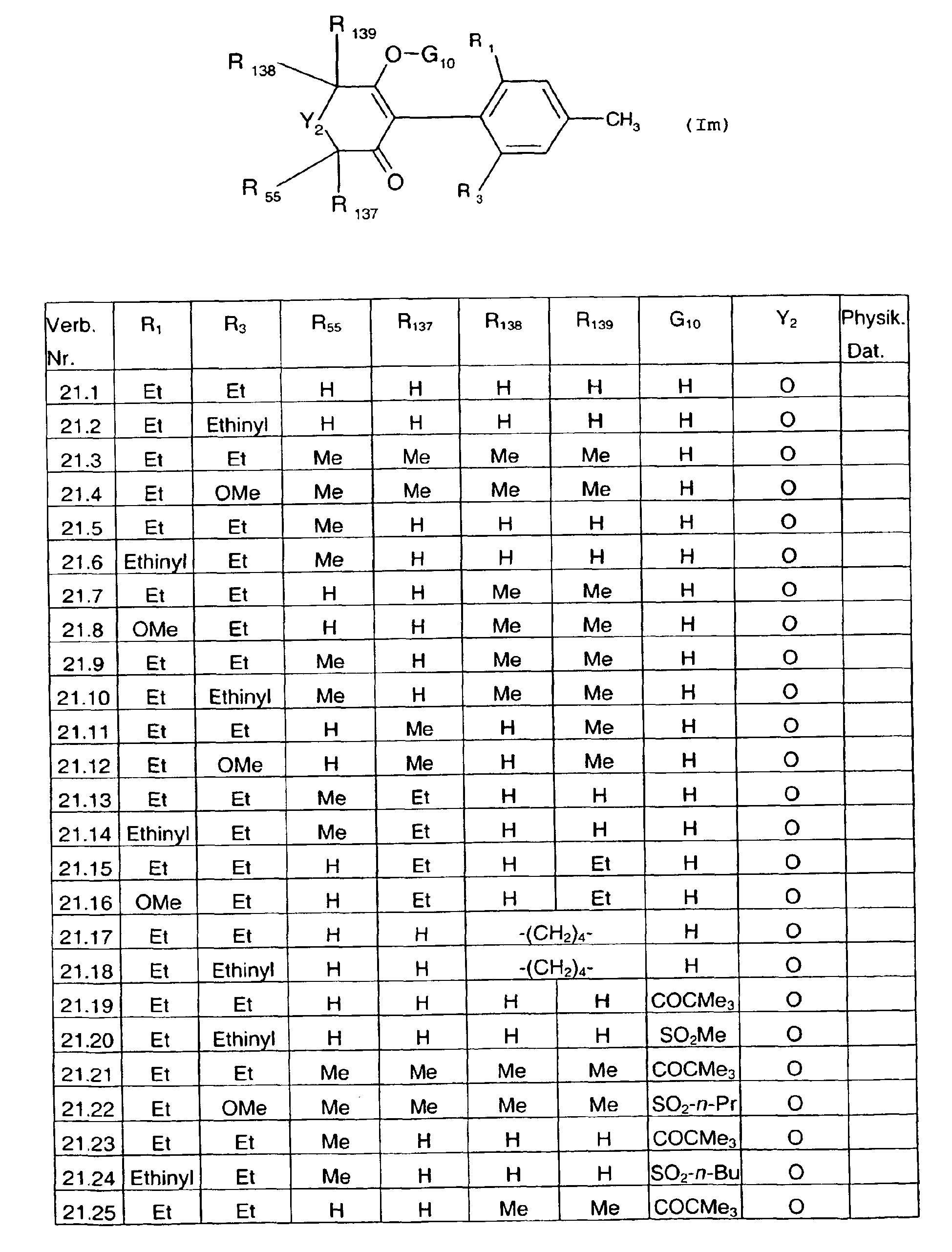

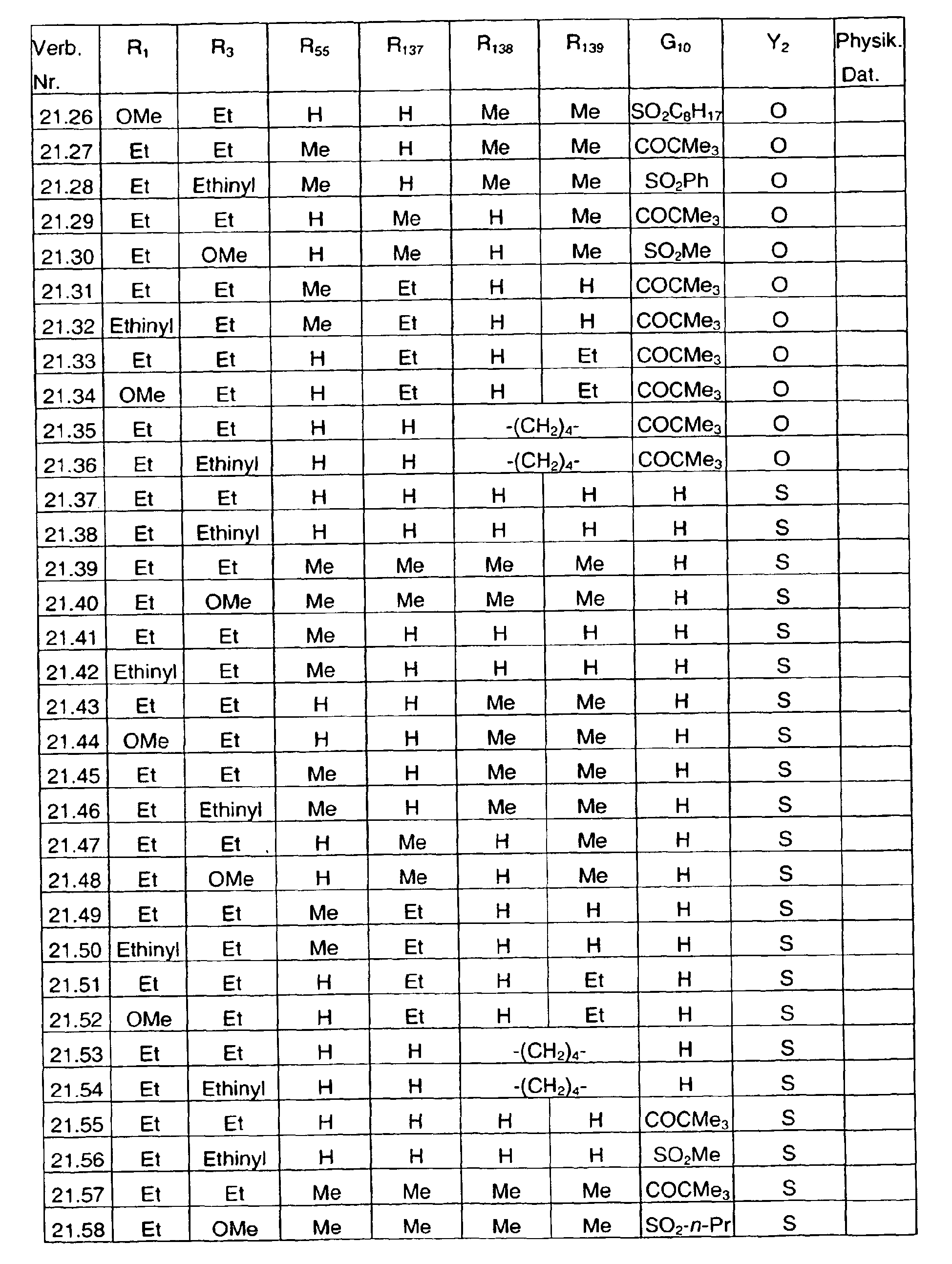

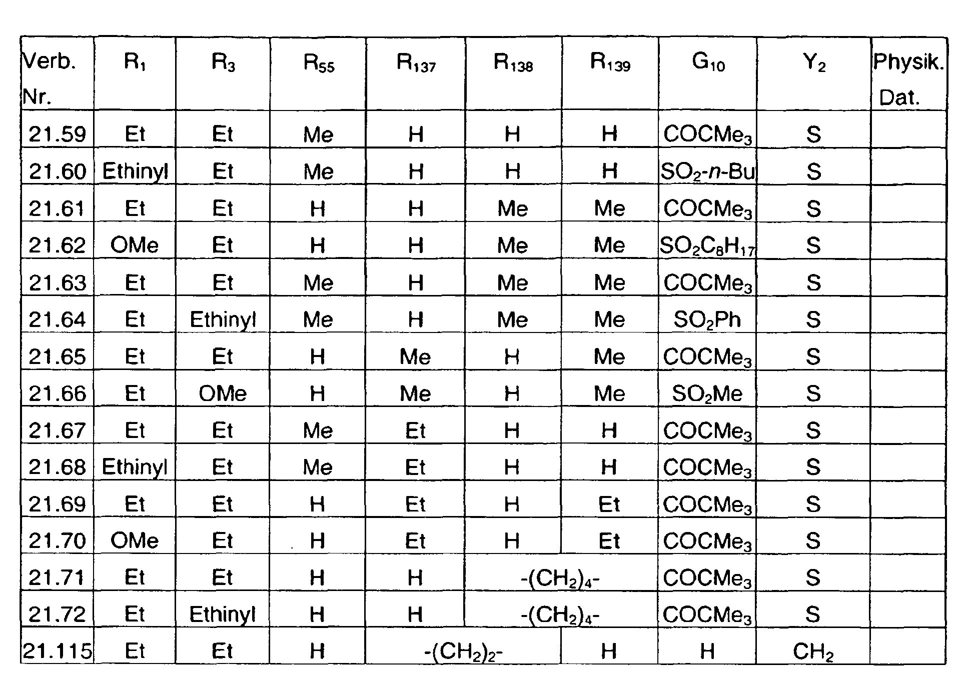

### Biologische Beispiele

### Beispiel B1: Herbizidwirkung vor dem Auflaufen der Pflanzen (pre-emergente Wirkung)

Monokotyle und dikotyle Unkräuter werden in Kunststofftöpfen in Standarderde ausgesät. Unmittelbar nach der Saat werden die Prüfsubstanzen als wäßrige Suspension (hergestellt aus einem 25 %igen Spritzpulver (Beispiel F3, b)) oder als Emulsion (hergestellt aus einem 25 %igen Emulsionskonzentrat (Beispiel F1, c)) appliziert (500 I Wasser/ha). Die Aufwandmenge beträgt 500 g/ha Aktivsubstanz. Anschließend werden die Testpflanzen im Gewächshaus unter Optimalbedingungen angezogen. Die Auswertung erfolgt 3 Wochen nach Applikation mit einer neunstufigen Boniturskala (1 = vollständige Schädigung, 9 = keine Wirkung). Boniturnoten von 1 bis 4 (insbesondere 1 bis 3) bedeuten eine gute bis sehr gute Herbizidwirkung.
Testpflanzen: Alopecurus (Alo), Avena (Ave), Lolium (Lol), Setaria (Set), Panicum (Pan), Sorghum (Sor), Digitaria (Dig), Echinocloa (Ech) und Brachiaria (Bra).

### Beispiel B2: Herbizide Wirkung nach dem Auflaufen der Pflanzen (post-emergente Wirkung):

Monokotyle und dikotyle Unkräuter werden unter Gewächshausbedingungen in Kunststofftöpfen in Standarderde angezogen. Die Applikation der Prüfsubstanzen verfolgt im 3- bis 6-Blattstadium der Testpflanzen. Die Prüfsubstanzen werden als wäßrige Suspension (hergestellt aus einem 25 %igen Spritzpulver (Beispiel F3, b)) oder als Emulsion (hergestellt aus einem 25 %igen Emulsionskonzentrat (Beispiel F1, c)) (500 l Wasser/ha) mit einer Aufwandmenge von 500 g/ha Aktivsubstanz appliziert. Die Auswertung erfolgt 3 Wochen nach Applikation mit einer neunstufigen Boniturskala (1 =vollständige Schädigung, 9 = keine Wirkung). Boniturnoten von 1 bis 4 (insbesondere 1 bis 3) bedeuten eine gute bis sehr gute Herbizidwirkung.
Testpflanzen: Alopecurus (Alo), Avena (Ave), Lolium (Lol), Setaria (Set), Panicum (Pan), Sorghum (Sor), Digitaria (Dig), Echinocloa (Ech) und Brachiaria (Bra).

### Beispiel B3: Herbizidwirkung vor dem Auflaufen der Pflanzen (pre-emergente Wirkung) von Verbindungen der vorliegenden Erfindung:

Monokotyle und dikotyle Unkräuter werden in Kunststofftöpfen in Standarderde ausgesät. Unmittelbar nach der Saat werden die Prüfsubstanzen als wäßrige Suspension (hergestellt aus einem 25 %igen Spritzpulver (Beispiel F3, b)) oder als Emulsion (hergestellt aus einem 25 %igen Emulsionskonzentrat (Beispiel F1, c)) appliziert (500 I Wasser/ha). Die Aufwandmenge beträgt 500 g/ha Aktivsubstanz. Anschließend werden die Testpflanzen im Gewächshaus unter Optimalbedingungen angezogen. Die Auswertung erfolgt 3 Wochen nach Applikation mit einer neunstufigen Boniturskala (1 = vollständige Schädigung, 9 = keine Wirkung). Boniturnoten von 1 bis 4 (insbesondere 1 bis 3) bedeuten eine gute bis sehr gute Herbizidwirkung.
Testpflanzen: Avena (Ave), Lolium (Lol), Setaria (Set).

## Patentansprüche

1. Verbindungen der Formel I 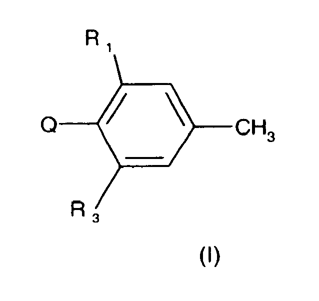 worin
R₁ und R₃ unabhängig voneinander Ethyl, Halogenethyl, Ethinyl, C₁-C₂-Alkoxy, C₁-C₂₋Halogenalkoxy oder C₁-C₂-Alkylcarbonyl bedeuten;
Q eine Gruppe 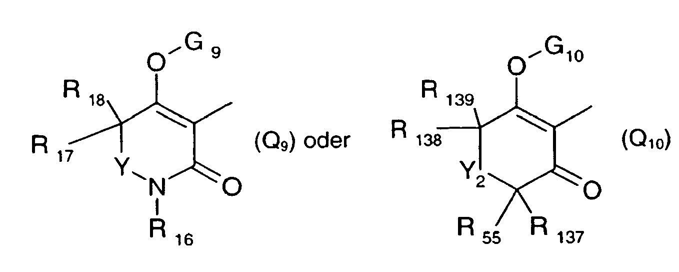 bedeutet;
R₁₆ C₁-C₁₀-Alkyl, C₂-C₁₀-Alkenyl, C₂-C₁₀-Alkinyl, C₁-C₁₀-Halogenalkyl, C₂-C₁₀-Alkoxyalkyl, C₃₋C₁₀-Alkenyloxyalkyl, C₃-C₁₀-Alkinyloxyalkyl, C₂-C₁₀-Alkylthiolkyl, C₂-C₁₀-Alkylsulfinylalkyl, C₂₋C₁₀-Alkylsulfonylalkyl, C₃-C₁₀-Cycloalkyl, Aryl oder Heteroaryl;
R₁₇ C₁-C₁₀-Alkyl, C₂-C₁₀-Alkenyl, C₂-C₁₀-Alkinyl, C₁-C₁₀-Halogenalkyl, C₂-C₁₀-Alkoxyalkyl, C₃₋C₁₀-Alkenyloxyalkyl, C₃-C₁₀-Alkinyloxyalkyl, C₂-C₁₀-Alkylthioalkyl, C₂-C₁₀-Alkylsulfinylalkyl, C₂₋C₁₀-Alkylsulfonylalkyl, C₂-C₁₀-Alkylcarbonyl-alkyl, C₃-C₁₀-Cycloalkyl, Aryl oder Heteroaryl bedeuten;
R₁₈ Wasserstoff, C₂-C₁₀-Alkenyl, C₂-C₁₀-Alkinyl, C₁-C₁₀-Alkyl oder C₁-C₁₀-Alkoxyalkyl bedeutet; oder
R₁₇ und R₁₈ bilden gemeinsam mit den Atomen, an die sie gebunden sind einen 3- bis 7-gliedrigen Cyclus, der ein oder zwei Heteroatome ausgewählt aus Stickstoff, Sauerstoff und Schwefel enthalten kann;
Y Sauerstoff oder C-R₁₉ bedeutet,
R₁₉ C₁-C₁₀-Alkyl, C₂-C₁₀-Alkenyl, C₂-C₁₀-Alkinyl, C₁-C₁₀-Halogenalkyl, Phenyl oder Heteroaryl bedeutet; oder
R₁₈ und R₁₉ bilden gemeinsam mit dem Atom, an das sie gebunden sind, einen gesättigten 5- bis 7- gliedrigen Cyclus, der ein oder zwei Heteroatome ausgewählt aus Stickstoff, Sauerstoff und Schwefel enthalten kann;
G₉ und G₁₀ unabhängig voneinander Wasserstoff, -C(X₁)-R₂₀, -C(X₂)-X₃-R₂₁, -C(X₄)-N(R₂₂)-R₂₃. -SO₂-R₂₄, ein Alkali-, Erdalkali-, Sulfonium- oder Ammoniumkation, -P(X₅)(R₂₅)-R₂₆ oder -CH₂-X₆-R₂₇ bedeuten;
X₁, X₂, X₃, X₄ , X₅ und X₆ unabhängig voneinander Sauerstoff oder Schwefel bedeuten;
R₂₀, R₂₁, R₂₂ und R₂₃ unabhängig voneinander Wasserstoff, C₁-C₁₀-Alkyl, C₂-C₁₀-Alkenyl, C₂-C₁₀-Alkinyl, C₁-C₁₀-Halogenalkyl, C₁-C₁₀-Cyanoalkyl, C₁-C₁₀-Nitroalkyl, C₁-C₁₀-Aminoalkyl, C₁-C₅-Alkylamino-C₁-C₅-alkyl, C₂-C₈-Dialkylamino-C₁-C₅-alkyl, C₃-C₇-Cycloalkyl-C₁-C₅-alkyl, C₂-C₁₀-Alkoxy-alkyl, C₄-C₁₀-Alkenyloxy-alkyl, C₄-C₁₀-Alkinyloxy-alkyl, C₂-C₁₀-Alkylthio-alkyl, C₁-C₅-Alkysulfoxyl-C₁-C₅-alkyl, C₁-C₅-Alkylsulfonyl-C₁-C₅-alkyl, C₂-C₈-Alkylideneamino-oxy-C₁-C₅-alkyl, C₁-C₅-Alkylcarbonyl-C₁-C₅-alkyl, C₁-C₅-Alkoxycarbonyl-C₁-C₅-alkyl, C₁-C₅-Aminocarbonyl-C₁-C₅-alkyl, C₂-C₈-Dialkylamino-carbonyl-C₁-C₅-alkyl, C₁-C₅-Alkylcarbonylamino-C₁₋C₅-alkyl, C₁-C₅-Alkylcarbonyl-(C₂-C₅-alkyl)-aminoalkyl, C₃-C₆-Trialkylsilyl-C₁-C₅-alkyl, Phenyl-C₁-C₅-alkyl, Heteroaryl-C₁-C₅-alkyl, Phenoxy- C₁-C₅-alkyl, Heteroaryloxy-C₁-C₅-alkyl, C₂-C₅₋Alkenyl, C₂-C₅-Halogenalkenyl, C₃-C₈-Cycloalkyl, Phenyl, oder durch C₁-C₃-Alkyl, C₁-C₃₋Halogenalkyl, C₁-C₃-Alkoxy, C₁-C₃-Halogenalkoxy, Halogen, Cyano oder Nitro substituiertes Phenyl, oder Heteroaryl oder Heteroarylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Heteroaryl oder Heteroarylamino, Diheteroarylamino oder durch C₁-C₃-Alkyl, C₁-C₃-Halogenalkyl, C₁₋C₃-Alkoxy, C₁-C₃-Halogenalkoxy, Halogen, Cyano oder Nitro substituiertes Diheteroarylamino, Phenylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenylamino, Diphenylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Diphenylamino, oder C₃-C₇-Cycloalkylamino, Di-C₃-C₇₋cycloalkylamino oder C₃-C₇-Cycloalkoxy bedeuten;
R₂₄, R₂₅ und R₂₆ Wasserstoff, C₁-C₁₀-Alkyl, C₂-C₁₀-Alkenyl, C₂-C₁₀-Alkinyl, C₁-C₁₀₋Halogenalkyl, C₁-C₁₀-Cyanoalkyl, C₁-C₁₀-Nitroalkyl, C₁- C₁₀-Aminoalkyl, C₁-C₅-Alkylamino-C₁₋C₅-alkyl, C₂-C₈-Dialkylamino- C₁-C₅-alkyl, C₃-C₇Cycloalkyl-C₁-C₅-alkyl, C₂-C₁₀-Alkoxy-alkyl, C₄-C₁₀-Alkenyloxy-alkyl, C₄-C₁₀-Alkinyloxy-alkyl, C₂- C₁₀-Alkylthio-alkyl, C₁-C₅-Alkysulfoxyl-C₁-C₅-alkyl, C₁-C₅-Alkylsulfonyl-C₁-C₅-alkyl, C₂-C₈-Alkylideneamino-oxy-C₁-C₅-alkyl, C₁-C₅₋Alkylcarbonyl-C₁-C₅-alkyl, C₁-C₅-Alkoxycarbonyl-C₁-C₅-alkyl, C₁-C₅-Amino-carbonyl-C₁-C₅₋alkyl, C₁-C₈-Dialkylamino-carbonyl-C₁-C₅-alkyl, C₁-C₅-Alkylcarbonylamino-C₁-C₅-alkyl, C₁-C₅₋Alkylcarbonyl-(C₂-C₅-alkyl)-aminoalkyl, C₃-C₆-Trialkylsilyl-C₁-C₅-alkyl, Phenyl-C₁-C₅-alkyl, Heteroaryl-C₁-C₅-alkyl, Phenoxy- C₁-C₅-alkyl, Heteroaryloxy- C₁-C₅-alkyl, C₂-C₅-Alkenyl, C₂₋C₅-Halogenalkenyl, C₃-C₈-Cycloalkyl, Phenyl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Phenyl, oder Heteroaryl oder Heteroarylamino, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Heteroaryl oder Heteroarylamino, Diheteroarylamino, oder durch C₁-C₃-Alkyl, C₁-C₃-Halogenalkyl, C₁-C₃₋Alkoxy, C₁-C₃-Halogenalkoxy, Halogen, Cyano oder Nitro substituiertes Diheteroarylamino, Phenylamino, oder durch C₁-C₃-Alkyl, C₁-C₃-Halogenalkyl, C₁-C₃-Alkoxy, C₁-C₃₋Halogenalkoxy, Halogen, Cyano oder Nitro substituiertes Phenylamino, Diphenylamino oder durch C₁-C₃-Alkyl, C₁-C₃-Halogenalkyl, C₁-C₃-Alkoxy, C₁-C₃-Halogenalkoxy, Halogen, Cyano oder Nitro substituiertes Diphenylamino, oder C₃-C₇-Cycloalkylamino, Di-C₃-C₇₋cycloalkylamino, C₃-C₇-Cycloalkoxy, C₁-C₁₀-Alkoxy, C₁-C₁₀-Halogenalkoxy, C₁-C₅₋Alkylamino, C₂-C₈-Dialkylamino, Benzyloxy oder Phenoxy, wobei die Benzyl- und Phenylgruppen ihrerseits durch C₁-C₃-Alkyl, C₁-C₃-Halogenalkyl, C₁-C₃-Alkoxy, C₁-C₃₋Halogenalkoxy, Halogen, Cyano oder Nitro substituiert sein können, bedeuten;
R₂₇ C₁-C₁₀-Alkyl, C₂-C₁₀-Alkenyl, C₂-C₁₀-Alkinyl, C₁-C₁₀-Halogenalkyl, C₁-C₁₀-Cyanoalkyl, C₁₋C₁₀-Nitroalkyl, C₁-C₁₀-Aminoalkyl, C₁-C₅-Alkylamino-C₁-C₅-alkyl, C₂-C₈-Dialkylamino-C₁-C₅₋alkyl, C₃-C₇-Cycloalkyl-C₁-C₅-alkyl, C₂-C₁₀-Alkoxy-alkyl, C₄-C₁₀-Alkenyloxy-alkyl, C₄-C₁₀₋Alkinyloxy-alkyl, C₂-C₁₀-Alkylthio-alkyl, C₁-C₅-Alkylsulfoxyl- C₁-C₅-alkyl, C₁-C₅-Alkylsulfonyl-C₁-C₅-alkyl, C₂-C₈-Alkylideneamino-oxy-C₁-C₅-alkyl, C₁-C₅-Alkylcarbonyl-C₁-C₅-alkyl, C₁-C₅₋Alkoxycarbonyl-C₁-C₅-alkyl, C₁-C₅-Amino-carbonyl-C₁-C₅-alkyl, C₂-C₈-Dialkylamino-carbonyl-C₁-C₅-alkyl, C₁-C₅-Alkylcarbonylamino-C₁-C₅-alkyl, C₁-C₅-Alkylcarbonyl-(C₂-C₅-alkyl)-aminoalkyl, C₃-C₆-Trialkylsilyl-C₁-C₅-alkyl, Phenyl-C₁-C₅-alkyl, Heteroaryl-C₁-C₅-alkyl, Phenoxy-C₁-C₅-alkyl, Heteroaryloxy-C₁-C₅-alkyl, C₂-C₅-Alkenyl, C₂-C₅-Halogenalkenyl, C₃-C₈₋Cycloalkyl, Phenyl, oder durch C₁-C₃-Alkyl, C₁-C₃-Halogenalkyl, C₁-C₃-Alkoxy, C₁-C₃₋Halogenalkoxy, Halogen, Cyano oder Nitro substituiertes Phenyl, oder Heteroaryl, oder Heteroarylamino, oder durch C₁-C₃-Alkyl, C₁-C₃-Halogenalkyl, C₁-C₃-Alkoxy, C₁-C₃₋Halogenalkoxy, Halogen, Cyano oder Nitro substituiertes Heteroaryl oder Heteroarylamino, Diheteroarylamino, durch C₁-C₃-Alkyl, C₁-C₃-Halogenalkyl, C₁-C₃-Alkoxy, C₁-C₃₋Halogenalkoxy, Halogen, Cyano oder Nitro substituiertes Diheteroarylamino, oder Phenylamino, durch C₁-C₃-Alkyl, C₁-C₃-Halogenalkyl, C₁-C₃-Alkoxy, C₁-C₃-Halogenalkoxy, Halogen, Cyano oder Nitro substituiertes Phenylamino, Diphenylamino, durch C₁-C₃-Alkyl, C₁-C₃-Halogenalkyl, C₁-C₃-Alkoxy, C₁-C₃-Halogenalkoxy, Halogen, Cyano oder Nitro substituiertes Diphenylamino, C₃-C₇-Cycloalkylamino, Di-C₃-C₇-cycloalkylamino, C₃-C₇₋Cycloalkoxy oder C₁-C₁₀-Alkylcarbonyl bedeutet;
Y₂ Sauerstoff oder Schwefel bedeutet,
R₅₅ C₁-C₁₀-Alkyl, C₂-C₁₀-Alkenyl, C₂-C₁₀-Alkinyl, C₁-C₁₀-Halogenalkyl, C₂-C₁₀-Alkoxyalkyl, C₃₋C₁₀-Alkenyloxyalkyl, C₃-C₁₀-Alkinyloxyalkyl, C₂-C₁₀-Alkylthioalkyl, C₂-C₁₀-Alkylsulfinylalkyl, C₂₋C₁₀-Alkylsulfonylalkyl, C₂-C₁₀-Alkylcarbonyl-alkyl, C₃-C₁₀-Cycloalkyl, Aryl oder Heteroaryl bedeutet;
R₁₃₇ Wasserstoff, C₁-C₁₀-Alkyl C₂-C₁₀-Alkenyl, C₂-C₁₀-Alkinyl, oder C₁-C₁₀-Alkoxyalkyl bedeutet; oder
R₅₅ und R₁₃₇ bilden gemeinsam mit den Atomen, an die sie gebunden sind, einen 3- bis 7-gliedrigen Cyclus, der ein oder zwei Heteroatome ausgewählt aus Stickstoff, Sauerstoff und Schwefel enthalten kann; und
R₁₃₈ und R₁₃₉ unabhängig voneinander Wasserstoff, C₁-C₁₀-Alkyl, C₂-C₁₀-Alkenyl, C₂-C₁₀₋Alkinyl, C₂-C₁₀-Alkoxyalkyl sind;
sowie die Verbindung Nr. 21.115,
sowie agronomisch verträgliche Salze, Isomere und Enantiomere dieser Verbindungen.

2. Verfahren zur Herstellung von Verbindungen der Formel gemäß Anspruch 1, **dadurch gekennzeichnet, daß** man eine Verbindung der Formel XXX
Q-H (XXX)
worin Q für Q₉ oder Q₁₀ steht, wobei deren Substituenten mit Ausnahme von G₉ und G₁₀ die oben angegebene Bedeutung haben und G₉ und G₁₀ Wasserstoff bedeuten, mit einer Verbindung der Formel XXXI 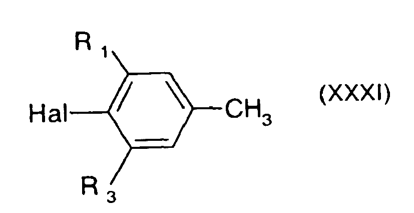 worin R₁ und R₃ die unter Formel I angegebene Bedeutung haben und Hal für Chlor, Brom oder Jod steht, in Gegenwart eines inerten Lösungsmittels, einer Base und eines Palladiumkatalysators bei Temperaturen von 30 bis 250 °C umsetzt.

3. Herbizides und den Pflanzenwuchs hemmendes Mittel, **dadurch gekennzeichnet, daß** es auf einem inerten Träger einen herbizid wirksamen Gehalt an Verbindung der Formel I aufweist.

4. Verfahren zur Bekämpfung unerwünschten Pflanzenwachstums, **dadurch gekennzeichnet, daß** man einen Wirkstoff der Formel I, oder ein diesen Wirkstoff enthaltendes Mittel in einer herbizid wirksamen Menge auf die Pflanzen oder deren Lebensraum appliziert.

5. Verfahren zur Hemmung des Pflanzenwachstums, **dadurch gekennzeichnet, daß** man einen Wirkstoff der Formel I, oder ein diesen Wirkstoff enthaltendes Mittel in einer herbizid wirksamen Menge auf die Pflanzen ode deren Lebensraum appliziert.

6. Selektiv-herbizides Mittel, **dadurch gekennzeichnet, daß** es neben üblichen inerten Formulierungshilfsmitteln als Wirkstoff eine Mischung aus
a) einer herbizid-wirksamen Menge einer Verbindung der Formel I gemäß Anspruch 1 und
b) einer herbizid-antagonistisch wirksamen Menge entweder einer Verbindung der Formel X 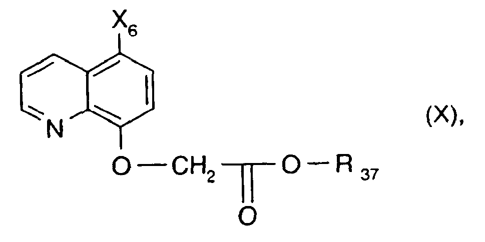
worin
R₃₇ Wasserstoff, C₁-C₈-Alkyl oder durch C₁-C₆-Alkoxy oder C₃-C₆-Alkenyloxy substituiertes C₁-C₈-Alkyl; und X₆ Wasserstoff oder Chlor bedeutet; oder einer Verbindung der Formel XI 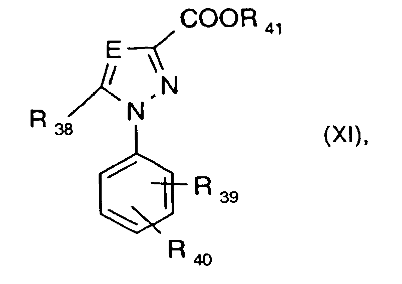 worin
E Stickstoff oder Methin; R₃₈ -CCl₃, Phenyl oder durch Halogen substituiertes Phenyl;
R₃₉ und R₄₀ unabhängig voneinander Wasserstoff oder Halogen; und
R₄₁ C₁-C₄-Alkyl bedeuten; oder einer Verbindung der Formel XII 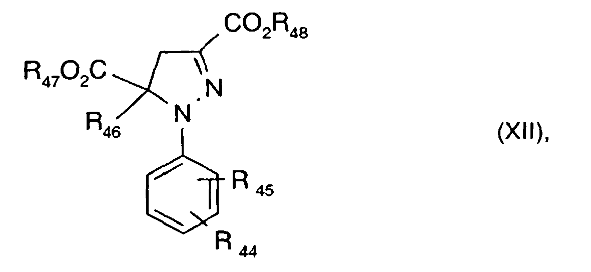 worin R₄₄ und R₄₅ unabhängig voneinander Wasserstoff oder Halogen und
R₄₆, R₄₇ und R₄₈ unabhängig voneinander C₁-C₄-Alkyl bedeuten, oder einer Verbindung der Formel XIII 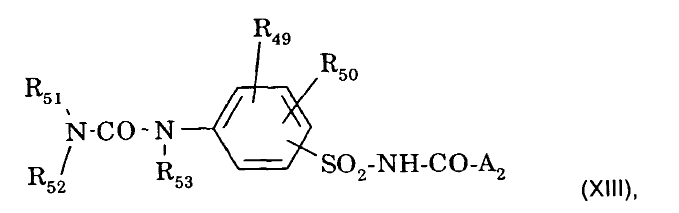 worin A₂ für eine Gruppe 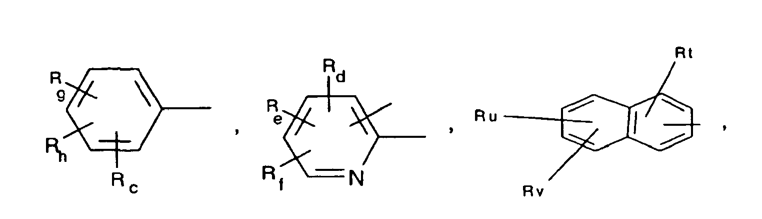 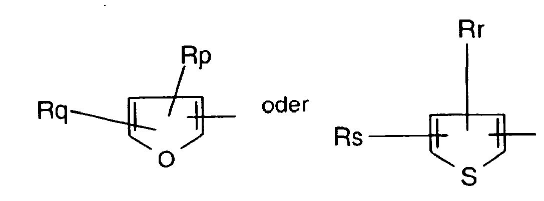 steht,
R₅₁ und R₅₂ unabhängig voneinander Wasserstoff, C₁-C₈-Alkyl, C₃-C₈-Cycloalkyl, C₃-C₆₋Alkenyl, C₃-C₆-Alkinyl, 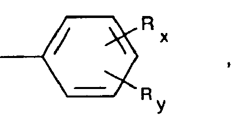 oder durch C₁-C₄-Alkoxy oder 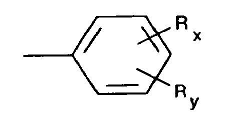 substituiertes C₁-C₄-Alkyl bedeutet; oder R₅₁ und R₅₂ bilden zusammen eine C₄-C₆-Alkylenbrücke, die durch Sauerstoff, Schwefel, SO, SO₂, NH oder -N(C₁-C₄-Alkyl)- unterbrochen sein kann,
R₅₃ für Wasserstoff oder C₁-C₄-Alkyl;
R₄₉ für Wasserstoff, Halogen, Cyano, Trifluoromethyl, Nitro, C₁-C₄-Alkyl, C₁-C₄-Alkoxy, C₁₋C₄-Alkylthio, C₁-C₄-Alkylsulfinyl, C₁-C₄-Alkylsulfonyl, -COORⱼ, -CONRₖRₘ, -CORₙ, -SO₂NRₖRₘ oder -OSO₂-C₁-C₄-Alkyl;
R_{g} für Wasserstoff, Halogen, Cyano, Nitro, C₁-C₄-Alkyl, C₁-C₄-Halogenalkyl, C₁-C₄-Alkylthio, C₁-C₄-Alkylsutfinyl, C₁-C₄-Alkylsulfonyl, -COORⱼ, -CONRₖRₘ, -CORₙ, -SO₂NRₖRₘ, -OSO₂-C₁₋C₄-Alkyl, C₁-C₆-Alkoxy, oder C₁-C₆alkoxy substituiert durch C₁-C₄-Alkoxy oder Halogen, C₃₋C₆-Alkenyloxy, oder C₃-C₆-Alkenyloxy substituiert durch Halogen, oder C₃-C₆-Alkinyloxy, oder R₄₉ und R₅₀ zusammen bilden eine C₃-C₄-Alkylenbrücke, die durch Halogen oder C₁-C₄-Alkyl substituiert sein kann, oder bilden eine C₃-C₄-Alkenylenbrücke, die durch Halogen oder C₁₋C₄-Alkyl substituiert sein kann, oder bilden eine C₄-Alkadienylenbrücke, die durch Halogen oder C₁-C₄-Alkyl substituiert sein kann;
R₅₀ und Rₕ unabhängig voneinander für Wasserstoff, Halogen, C₁-C₄-Alkyl, Trifluormethyl, C₁-C₆-Alkoxy, C₁-C₆-Alkylthio oder -COORⱼ;
R_{c} für Wasserstoff, Halogen, Nitro, C₁-C₄-Alkyl oder Methoxy; R_{d} für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ;
Rₑ für Wasserstoff, Halogen, C₁-C₄-Alkyl, -COORⱼ, Trifluormethyl or Methoxy, oder R_{d} und Rₑ bilden zusammen eine C₃-C₄-Alkylenbrücke;
Rp für Wasserstoff, Halogen, C₁-C₄-Alkyl, -COORⱼ, Trifluormethyl or Methoxy; Rq für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -CO0Rⱼ oder CONRₖRₘ; , oder Rp und Rq bilden zusammen eine C₃-C₄₋Alkylenbrücke;
Rr für Wasserstoff, Halogen, C₁-C₄-Alkyl, -COORⱼ, Trifluormethyl or Methoxy; Rs für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ; , oder Rr und Rs bilden zusammen eine C₃-C₄₋Alkylenbrücke;
Rt für Wasserstoff, Halogen, C₁-C₄-Alkyl, -COORⱼ, Trifluormethyl or Methoxy; Ru für Wasserstoff, Halogen, Nitro, C₁-C₄-Alkyl, C₁-C₄-Alkoxy, C₁-C₄Alkylthio, C₁-C₄-Alkylsulfinyl, C₁-C₄-Alkylsulfonyl, -COORⱼ oder CONRₖRₘ; , oder Rv und Ru bilden zusammen eine C₃-C₄₋Alkylenbrücke;
R_{f} und Rv für Wasserstoff, Halogen oder C₁-C₄-Alkyl;
Rₓ und R_{y} unabhängig voneinander für Wasserstoff, Halogen, C₁-C₄-Alkyl, C₁-C₄-Alkoxy, C₁₋C₄-Alkylthio, -COOR₅₄, Trifluoromethyl, Nitro oder Cyano;
Rⱼ, Rₖ und Rₘ unabhängig voneinander für Wasserstoff oder C₁-C₄-Alkyl; oder
Rₖ und Rₘ bilden zusammen eine C₄-C₆-Alkylenbrücke, die durch Sauerstoff, NH oder -N(C₁₋C₄-Alkyl)- unterbrochen sein kann;
Rₙ für C₁-C₄-Alkyl, Phenyl, oder durch Halogen, C₁-C₄-Alkyl, Methoxy, Nitro oder Trifluormethyl substituiertes Phenyl;
R₅₄ für Wasserstoff, C₁-C₁₀-Alkyl, C₁-C₄-Alkoxy-C₁-C₄-alkyl, C₁-C₄-Alkylthio-C₁-C₄-alkyl, Di-C₁-C₄-alkylamino-C₁-C₄-alkyl, Halogen-C₁-C₈-alkyl, C₂-C₈-Alkenyl, Halogen-C₂-C₈-alkenyl, C₃-C₈-Alkinyl, C₃-C₇-Cycloalkyl, Halogen-C₃-C₇-cycloalkyl, C₁-C₈-Alkylcarbonyl, Allylcarbonyl, C₃-C₇-Cycloalkylcarbonyl, Benzoyl, das unsubstituiert oder am Phenylring gleich oder verschieden bis zu dreifach durch Halogen, C₁-C₄-Alkyl, Halogen-C₁-C₄-alkyl, Halogen-C₁₋C₄-alkoxy oder C₁-C₄-Alkoxy substituiert ist; oder Furoyl, Thienyl; oder C₁-C₄-Alkyl substituiert durch Phenyl, Halogenphenyl, C₁-C₄-Alkylphenyl, C₁-C₄-Alkoxyphenyl, Halogen-C₁-C₄-alkylphenyl, Halogen-C₁-C₄-alkoxyphenyl, C₁-C₆-Alkoxycarbonyl, C₁-C₄-Alkoxy-C₁-C₈₋alkoxycarbonyl, C₃-C₈-Alkenyloxycarbonyl, C₃-C₈-Alkinyloxycarbonyl, C₁-C₈₋Alkylthiocarbonyl, C₃-C₈-Alkenylthiocarbonyl, C₃-C₈-Alkinylthiocarbonyl, Carbamoyl, Mono-C₁-C₄-alkylaminocarbonyl, Di-C₁-C₄-alkylaminocarbonyl; oder Phenylaminocarbonyl, das unsubstituiert oder am Phenyl gleich oder verschieden bis zu dreifach durch Halogen, C₁₋C₄-Alkyl, Halogen-C₁-C₄-alkyl, Halogen-C₁-C₄-alkoxy oder C₁-C₄-Alkoxy oder einfach durch Cyano oder Nitro substituiert ist, oder Dioxolan-2-yl, das unsubstituiert ist oder durch ein oder zwei C₁-C₄-Alkylreste substituiert ist, oder Dioxan-2-yl, das unsubstituiert ist oder durch ein oder zwei C₁-C₄-Alkylreste substituiert ist, oder C₁-C₄-Alkyl, das durch Cyano, Nitro, Carboxyl oder C₁-C₈-Alkylthio-C₁-C₈-alkoxycarbonyl substituiert ist, bedeutet;
oder einer Verbindung der Formel XIV 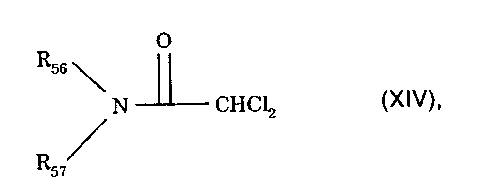 worin R₅₆ und R₅₇ unabhängig voneinander für C₁-C₆-Alkyl oder C₂-C₆-Alkenyl; oder R₅₆ und R₅₇ zusammen für 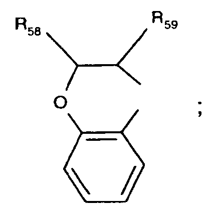 R₅₈ und R₅₉ unabhängig voneinander für Wasserstoff oder C₁-C₆-Alkyl; oder R₅₆ und R₅₇ zusammen für 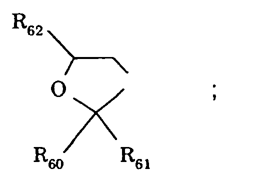 R₆₀ und R₆₁ unabhängig voneinander für C₁-C₄-Alkyl, oder R₆₀ und R₆₁ zusammen -(CH₂)₅- ; R₆₂ für Wasserstoff, C₁-C₄-Alkyl oder 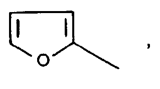 oder R₅₆ und R₅₇ zusammen für 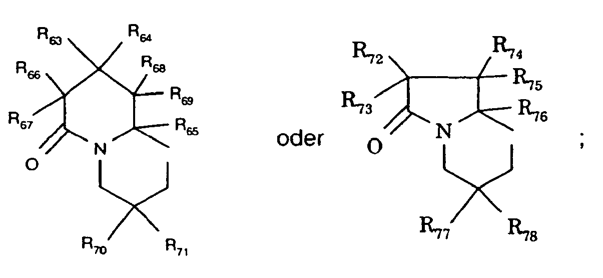 R₆₃, R₆₄, R₆₅, R₆₆, R₆₇, R₆₈, R₆₉, R₇₀, R₇₁, R₇₂, R₇₃, R₇₄, R₇₅, R₇₆, R₇₇ und R₇₈ unabhängig voneinander für Wasserstoff oder C₁-C₄-Alkyl stehen;
oder einer Verbindung der Formel XV 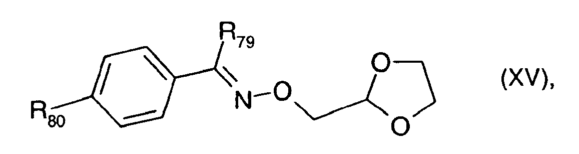 worin R₈₀ Wasserstoff oder Chlor und R₇₉ Cyano oder Trifluormethyl bedeutet,
oder eine Verbindung der Formel XVI 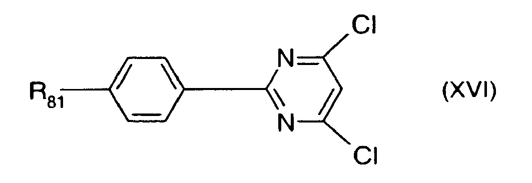 worin R₈₁ Wasserstoff oder Methyl bedeutet,
oder einer Verbindung der Formel XVII 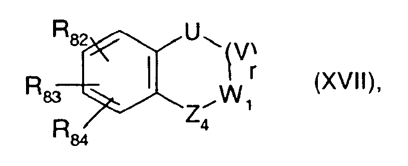 worin
R₈₂ Wasserstoff, C₁-C₄-Alkyl oder C₁-C₄-Alkyl substituiert durch C₁-C₄-Alkyl-X₂- oder C₁-C₄₋Halogenalkyl-X₂- bedeutet, oder für C₁-C₄-Halogenalkyl, Nitro, Cyano, -COOR₈₅, -NR₈₆R₈₇, -SO₂NR₈₈R₈₉ oder -CONR₉₀R₉₁ steht;
R₈₃ Wasserstoff, Halogen, C₁-C₄-Alkyl, Trifluormethyl, C₁-C₄-Alkoxy oder C₁-C₄₋Halogenalkoxy bedeutet;
R₈₄ Wasserstoff, Halogen oder C₁-C₄-Alkyl bedeutet;
U, V, W₁ und Z₄ unabhängig voneinander Sauerstoff, Schwefel, C(R₉₂)R₉₃, Carbonyl, NR₉₄, eine Gruppe 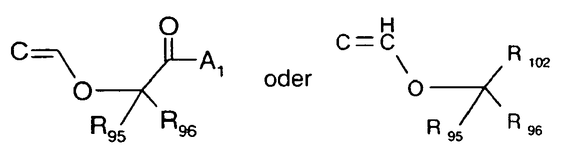 bedeuten, worin R₁₀₂ C₂-C₄-Alkenyl oder C₂-C₄-Alkinyl bedeutet; mit den Maßgaben, daß
a) mindestens eines der Ringglieder U, V, W₁ oder Z₄ Carbonyl ist, und ein zu diesem bzw. diesen Ringgliedern benachbartes Ringglied die Gruppe 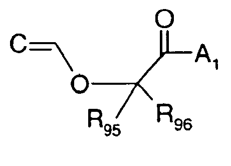 oder 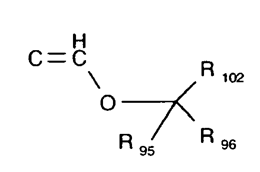 bedeutet, wobei diese Gruppe nur einmal vorkommt; und
b) zwei benachbarte Ringglieder U und V, V und W₁ und W₁ und Z₄ nicht gleichzeitig Sauerstoff bedeuten können;
R₉₅ und R₉₆ unabhängig voneinander Wasserstoff oder C₁-C₈-Alkyl bedeuten; oder
R₉₅ und R₉₆ zusammen eine C₂-C₆-Alkylengruppe bilden;
A₁ R₉₉-Y₁- oder -NR₉₇R₉₈;
X₂ Sauerstoff oder -S(O)ₛ;
Y₁ Sauerstoff oder Schwefel bedeuten;
R₉₉ Wasserstoff, C₁-C₈-Alkyl, C₁-C₈-Halogenalkyl, C₁-C₄-Alkoxy-C₁-C₈-alkyl, C₃-C₆₋Alkenyloxy-C₁-C₈-alkyl oder Phenyl-C₁-C₈-alkyl bedeutet, wobei der Phenylring durch Halogen, C₁-C₄-Alkyl, Trifluormethyl, Methoxy oder Methyl-S(O)ₛ- substituiert sein kann, oder C₃-C₆-Alkenyl, C₃-C₆-Halogenalkenyl, Phenyl-C₃-C₆-alkenyl, C₃-C₆-Alkinyl, Phenyl-C₃₋C₆-alkinyl, Oxetanyl, Furyl oder Tetrahydrofuryl bedeutet;
R₈₅ Wasserstoff oder C₁-C₄-Alkyl;
R₈₆ Wasserstoff, C₁-C₄-Alkyl oder C₁-C₄-Alkylcarbonyl bedeuten;
R₈₇ Wasserstoff oder C₁-C₄-Alkyl bedeutet; oder
R₈₆ und R₈₇ zusammen eine C₄- oder C₅-Alkylengruppe bilden;
R₈₈, R₈₉, R₉₀ und R₉₁ unabhängig voneinander Wasserstoff oder C₁-C₄-Alkyl bedeuten; oder R₈₈ zusammen mit R₈₉ oder R₉₀ zusammen mit R₉₁ unabhängig voneinander C₄- oder C₅₋Alkylen sind, wobei ein Kohlenstoffatom durch Sauerstoff oder Schwefel, oder ein oder zwei Kohlenstoffatome durch -NR₁₀₀- ersetzt sein können;
R₉₂, R₁₀₀ und R₉₃ unabhängig voneinander Wasserstoff oder C₁-C₈-Alkyl sind; oder
R₉₂ und R₉₃ zusammen C₂-C₆-Alkylen sind;
R₉₄ Wasserstoff oder C₁-C₈-Alkyl bedeutet;
R₉₇ Wasserstoff, C₁-C₈-Alkyl, Phenyl oder Phenyl-C₁-C₈-alkyl bedeutet, wobei die Phenylringe durch Fluor, Chlor, Brom, Nitro, Cyano, -OCH₃, C₁-C₄-Alkyl oder CH₃SO₂₋substituiert sein können, oder für C₁-C₄-Alkoxy-C₁-C₈-alkyl, C₃-C₆-Alkenyl oder C₃-C₆-Alkinyl;
R₉₈ Wasserstoff, C₁-C₈-Alkyl, C₃-C₆-Alkenyl oder C₃-C₆-Alkinyl steht; oder
R₉₇ und R₉₈ zusammen C₄- oder C₅-Alkylen bedeuten, wobei ein Kohlenstoffatom durch Sauerstoff oder Schwefel, oder ein oder zwei Kohlenstoffatome durch
-NR₁₀₁- ersetzt sein können;
R₁₀₁ Wasserstoff oder C₁-C₄-Alkyl bedeutet;
r 0 oder 1 ist; und
s 0, 1 oder 2 bedeutet,
oder eine Verbindung der Formel XVIII 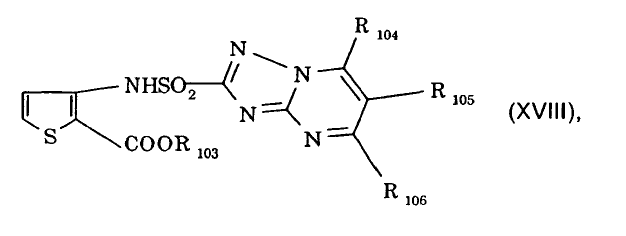 worin R₁₀₃ Wasserstoff, C₁-C₆-Alkyl, C₃-C₆-Cycloalkyl, C₃-C₆-Alkenyl oder C₃-C₆-Alkinyl; und R₁₀₄, R₁₀₅ und R₁₀₆ unabhängig voneinander Wasserstoff, C₁-C₆-Alkyl, C₃-C₆-Cycloalkyl oder C₁-C₆-Alkoxy bedeuten, mit der Maßgabe, daß einer der Substituenten R₁₀₄, R₁₀₅ und R₁₀₆ verschieden von Wasserstoff ist;
oder eine Verbindung der Formel XIX 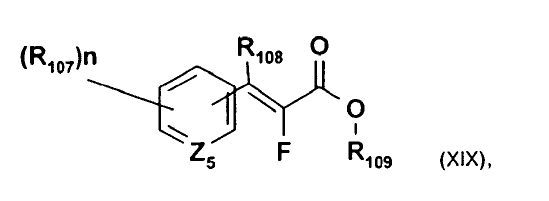 worin Z₅ N oder CH, n für den Fall, daß Z₅ gleich N ist, 0.1, 2 oder 3 und für den Fall, daß Z₅ CH ist, 0, 1, 2, 3 oder 4 bedeutet, R₁₀₇ Halogen, C₁-C₄-Alkyl, C₁-C₄-Halogenalkyl, C₁-C₄₋Alkoxy, C₁-C₄-Halogenalkoxy, Nitro, C₁-C₄-Alkylthio, C₁-C₄-Alkylsulfonyl, C₁-C₄₋Alkoxycarbonyl, Phenyl oder Phenoxy, oder durch C₁-C₃-Alkyl, C₁-C₃-Halogenalkyl, C₁-C₃₋Alkoxy, C₁-C₃-Halogenalkoxy, Halogen, Cyano oder Nitro substituiertes Phenyl oder Phenoxy bedeutet;
R₁₀₈ Wasserstoff oder C₁-C₄-Alkyl bedeutet, R₁₀₉ Wasserstoff, C₁-C₄-Alkyl, C₃-C₆-Cycloalkyl, C₂-C₆-Alkenyl, C₂-C₆-Alkinyl, C₁-C₄-Halogenalkyl, C₂-C₆-Halogenalkenyl, C₂-C₆₋Halogenalkinyl, C₁-C₄-Alkylthio-C₁-C₄-alkyl, C₁-C₄-Alkylsulfonyl- C₁-C₄-alkyl, C₁-C₄-Alkoxy-C₁-C₄-alkyl, C₁-C₄-Alkenyloxy- C₁-C₄-alkyl oder C₁-C₄-Alkinyloxy-C₁-C₄-alkyl ist;
oder eine Verbindung der Formel XX 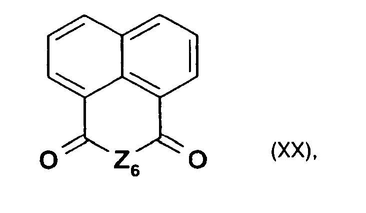 worin Z₆ Sauerstoff oder N-R₁₁₀ und R₁₁₀ eine Gruppe der Formel 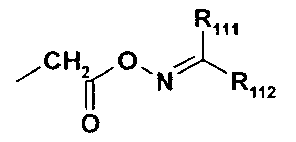 bedeutet, worin R₁₁₁, und R₁₁₂ unabhängig voneinander Cyano, Wasserstoff, C₁-C₄-Alkyl, C₃₋C₆-Cycloalkyl, C₂-C₆-Alkenyl, Aryl, Phenyl oder Heteroaryl, oder durch C₁-C₃-Alkyl, C₁-C₃₋Halogenalkyl, C₁-C₃-Alkoxy, C₁-C₃-Halogenalkoxy, Halogen, Cyano oder Nitro substituiertes Phenyl, Aryl oder Heteroaryl bedeuten;
oder eine Verbindung der Formel XXI 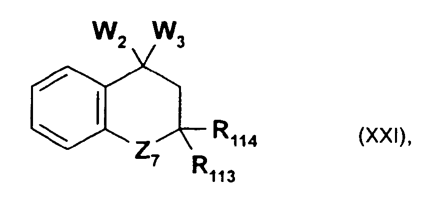 worin Z₇ Sauerstoff, Schwefel, S=O, SO₂ oder CH₂ bedeutet, R₁₁₃ und R₁₁₄ unabhängig voneinander Wasserstoff, Halogen oder C₁-C₄-Alkyl bedeuten, W₂ und W₃ unabhängig voneinander CH₂COOR₁₁₅ oder COOR₀₁₁₅ oder zusammen eine Gruppe der Formel -(CH₂)C(O)-O-C(O)-(CH₂)- bedeuten, und R₁₁₅ und R₀₁₁₅ unabhängig voneinander Wasserstoff, C₁-C₄-Alkyl, C₂-C₄-Alkenyl, C₂-C₆-Alkinyl, C₃-C₆-Cycloalkyl, C₁-C₄-Halogenalkyl, ein Metall- oder ein Ammonium- Kation bedeuten;
oder eine Verbindung der Formel XXII 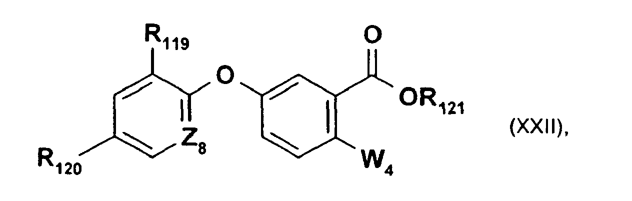 worin R₁₁₉ und R₁₂₀ unabhängig voneinander Wasserstoff, Halogen oder C₁-C₄-Halogenalkyl sind, R₁₂₁ Wasserstoff, C₁-C₄-Alkyl, C₃-C₄-Alkenyl, C₃-C₄-Alkinyl, C₁-C₄-Halogenalkyl, C₃-C₆₋Cycloalkyl, ein Metalkation oder ein Ammoniumkation bedeuten, Z₈ N, CH, C-F oder C-Cl bedeuten und W₄ eine Gruppe der Formel 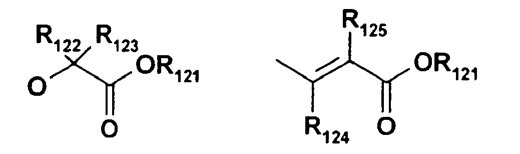 bedeutet, worin R₁₂₂ und R₁₂₃ unabhängig voneinander Wasserstoff oder C₁-C₄-Alkyl und R₁₂₄ und R₁₂₅ unabhängig voneinander Wasserstoff oder C₁-C₄-Alkyl bedeuten;
oder eine Verbindung der Formel XXIII 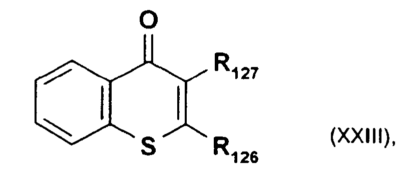 worin R₁₂₆ Wasserstoff, Cyano, Halogen, C₁-C₄-Alkyl, C₃-C₆-Cycloalkyl, C₁-C₄-Alkoxy, C₁-C₄₋Alkoxycarbonyl, C₁-C₄-Alkylthiocarbonyl, -NH-R_{128.} -C(O)NH-R₀₁₂₈, Aryl oder Heteroaryl, oder durch C₁-C₃-Alkyl, C₁-C₃-Halogenalkyl, C₁-C₃-Alkoxy, C₁-C₃-Halogenalkoxy, Halogen, Cyano oder Nitro substituiertes Aryl oder Heteroaryl bedeutet;
R₁₂₇ Wasserstoff, Cyano, Nitro, Halogen, C₁-C₄-Alkyl, C₁-C₄-Halogenalkyl, C₁-C₄-Alkoxy, C₁₋C₄-Thioalkyl bedeuten, und
R₁₂₈ und R₀₁₂₈ unabhängig voneinander C₁-C₄-Alkyl, C₁-C₄-Halogenalkyl, C₃-C₄-Alkenyl, C₃₋C₄-Alkinyl, C₃-C₄-Cycloalkyl, Aryl oder Heteroaryl, oder durch C₁-C₃-Alkyl, C₁-C₃₋Halogenalkyl, C₁-C₃-Alkoxy, C₁-C₃-Halogenalkoxy, Halogen, Cyano oder Nitro substituiertes Aryl oder Heteroaryl, Formyl, C₁-C₄-Alkylcarbonyl, C₁-C₄-Alkylsufonyl bedeuten;
oder eine Verbindung der Formel XXIV 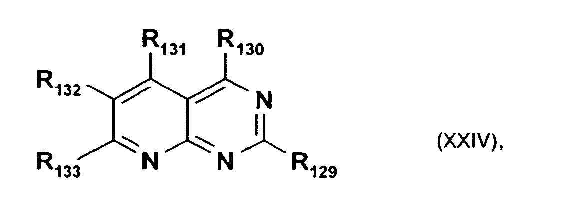 worin R₁₂₉ und R₁₃₀ unabhängig voneinander Wasserstoff, C₁-C₄-Alkyl, C₁-C₄-Halogenalkyl, C₁-C₄-Alkoxy, Mono-C₁-C₈- oder Di-C₁-C₈-Alkylamino, C₃-C₆-Cycloalkyl, C₁-C₄-Thioalkyl, Phenyl oder Heteroaryl sind, R₁₃₁ die Bedeutung von R₁₂₉ hat und zusätzlich OH, NH₂, Halogen, Di- C₁-C₄-Aminoalkyl, C₁-C₄-Alkylthio, C₁-C₄-Alkylsulfonyl oder C₁-C₄₋Alkoxycarbonyl ist, R₁₃₂ die Bedeutung von R₁₂₉ hat und zusätzlich Cyano, Nitro, Carboxyl, C₁-C₄-Alkoxycarbonyl, Di- C₁-C₄-Aminoalkyl, C₁-C₄-Alkylthio, C₁-C₄- Alkylsulfonyl, SO₂-OH, i- C₁-C₄-Aminoalkylsulfonyl oder C₁-C₄-Alkoxysulfonyl ist, R₁₃₃ die Bedeutung von R₁₂₉ hat und zusätzlich OH, NH₂, Halogen, Di- C₁-C₄-Aminoalkyl, Pyrrolidin1-yl, Piperidin-1-yl, Morpholin-1-yl, C₁-C₄-Alkylthio, C₁-C₄-Alkylsulfonyl, C₁-C₄-Alkoxycarbonyl, Phenoxy, Naphtoxy, Phenylamino, Benzoyloxy oder Phenylsulfonyloxy ist;
oder eine Verbindung der Formel XXV 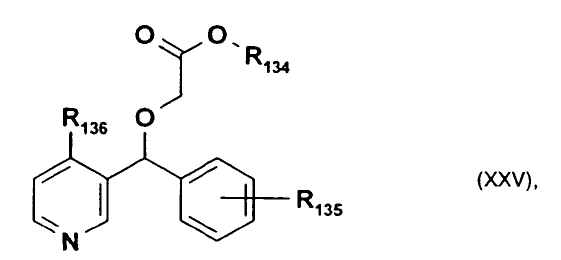 worin R₁₃₄ Wasserstoff, C₄-Alkyl, C₁-C₄-Halogenalkyl, C₂-C₄-Alkenyl, C₂-C₄-Alkinyl oder C₁₋C₄-Alkoxy-C₁-C₄-Alkyl bedeutet, R₁₃₅ Wasserstoff, Halogen, C₁-C₄-Alkyl, C₁-C₄-Halogenalkyl oder C₁-C₄-Alkoxy und R₁₃₆ Wasserstoff, Halogen, C₁-C₄-Alkyl, C₁-C₄-Halogenalkyl oder C₁₋C₄-Alkoxy bedeuten, mit der Maßgabe, daß R₁₃₅ und R₁₃₆ nicht gleichzeitig Wasserstoff bedeuten,
oder der Formel XXVI 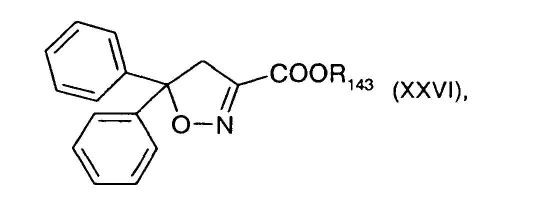 worin
R₁₄₃ Wasserstoff, ein Alkali-, Erdalkali-, Sulfonium- oder Ammonium-Kation oder Ethyl bedeutet;
oder der Formel XXVII 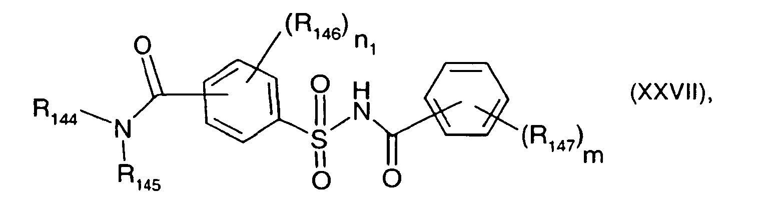 worin R₁₄₄ und R₁₄₅ unabhängig voneinander Wasserstoff, C₁-C₆-Alkyl, C₂-C₆-Alkenyl, C₂-C₆₋Alkinyl oder C₃-C₆-Cycloalkyl bedeuten;
R₁₄₆ Wasserstoff, Halogen, C₁-C₄-Alkyl, C₁-C₆-Halogenalkyl oder C₁-C₆-Halogenalkoxy bedeutet;
R₁₄₇Wasserstoff, Halogen, C₁-C₄-Alkyl, C₁-C₄-Halogenalkyl, C₁-C₄-Alkoxy, C₁-C₄₋Halogenalkoxy, C₁-C₄-Alkylthio, C₁-C₄-Alkoxycarbonyl oder Nitro bedeutet;
n, 0, 1, 2 oder 3; und
m 1 oder 2 bedeutet;
oder der Formel XXVIII 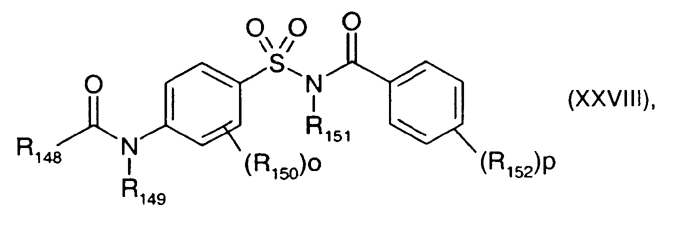 worin
R₁₄₈ Wasserstoff, C₁-C₆-Alkyl, C₁-C₆-Alkoxy, C₁-C₆-Alkylthio, C₃-C₈-Cycloalkyl, Phenyl, Phenyl-C₁-C₆-alkyl oder Heteroaryl bedeutet; wobei die genannten Gruppen durch Halogen, Cyano, Nitro, Amino, Hydroxy, Carbonyl, Carboxyl, Formyl, Carbonamid oder Sulfonamid substituiert sein können;
R₁₄₉ Wasserstoff, C₁-C₆-Alkyl oder C₁-C₄-Halogenalkyl bedeutet;
jedes R₁₅₀ unabhängig Wasserstoff, Halogen, C₁-C₄-Alkyl, C₁-C₄-Halogenalkyl, C₁-C₄-Alkoxy, C₁-C₄-Alkylthio, C₁-C₄-Alkylsulfonyl, Cyano, Nitro, Formyl oder Carboxyl bedeutet;
R₁₅₁ Wasserstoff, C₁-C₆-Alkyl oder C₁-C₄-Halogenalkyl bedeutet;
jedes R₁₅₂ unabhängig Wasserstoff, Halogen, C₁-C₄-Alkyl, C₁-C₄-Halogenalkyl, C₁-C₄-Alkoxy, C₁-C₄-Alkylthio, C₁-C₄-Alkylsulfonyl, Cyano, Nitro, Formyl oder Carboxyl bedeutet;
O für 0, 1, oder 2 steht, und
p 0, 1 oder 2 bedeutet;
oder der Formel XXIX 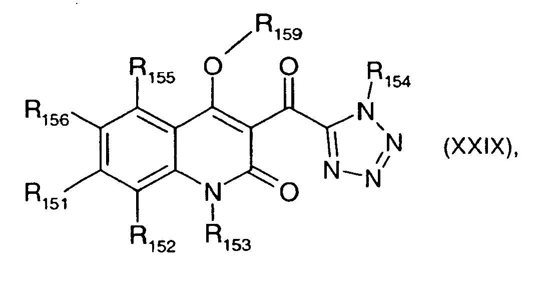 worin
R₁₅₉ Wasserstoff, Formyl, C₁₋₆-Alkylcarbonyl, C₁₋₆-Alkenylcarbonyl, C₁₋₆-Alkinylcarbonyl, C₁₋₆₋Alkoxycarbonyl, C₁₋₆-Alkylthiocarbonyl, C₃₋₈-Cycloalkylcarbonyl, Phenyl-C₁₋₆-alkylcarbonyl, Phenylcarbonyl. C₁₋₆-Alkylsulfonyl, C₁₋₆-Alkenylsulfonyl oder Phenylsulfonyl bedeutet, wobei die vorstehend genannten Kohlenwasserstoffgruppen durch ein oder mehrere Halogenatome, Cyano, Nitro, Amino, Methoxy, Ethoxy oder Phenyl substituiert sein können; R₁₅₃ Wasserstoff, C₁₋₆-Alkyl, C₁₋₆-Alkenyl, C₁₋₆-Alkinyl, C₃₋₈-Cycloalkyl, Formyl, C₁₋₆ Alkylcarbonyl, C₁₋₆-Alkenylcarbonyl, C₁₋₆-Alkinylcarbonyl, C₁₋₆-Alkoxycarbonyl, C₁₋₆₋Alkylthiocarbonyl, C₃₋₈-Cycloalkylcarbonyl, C₁₋₆-Alkylsulfonyl, C₁₋₆-Alkenylsulfonyl oder Phenylsulfonyl bedeutet, wobei die vorstehend genannten Kohlenwasserstoffgruppen durch ein oder mehrere Halogenatome, Cyano, Nitro, Amino, Methoxy, Ethoxy oder Phenyl substituiert sein können;
R₁₅₄ Wasserstoff, C₁₋₆-Alkyl, C₁₋₆-Alkenyl, C₁₋₆-Alkinyl, C₃₋₈-Cycloalkyl, Formyl, C₁₋₆₋Alkylcarbonyl, C₁₋₆-Alkenylcarbonyl, C₁₋₆-Alkinylcarbonyl, C₁₋₆-Alkoxycarbonyl, C₁₋₆₋Alkylthiocarbonyl, C₃₋₈-Cycloalkylcarbonyl, C₁₋₆-Alkylsulfonyl, C₁₋₆-Alkenylsulfonyl oder Phenylsulfonyl bedeutet, wobei die vorstehend genannten Kohlenwasserstoffgruppen durch ein oder mehrere Halogenatome, Cyano, Nitro, Amino, Methoxy, Ethoxy oder Phenyl substituiert sein können;
R₁₅₅, R₁₅₆, R₁₅₇, und R₁₅₈ unabhängig voneinander Wasserstoff, Halogen, Amino, C₁₋₃₋Alkylamino, C₁₋₆-Dialkylamino, Hydroxy, Cyano, Nitro, Formyl, Carboxyl, C₁₋₆-Alkoxy, C₁₋₆-Halogenalkoxy, C₁₋₆-Alkylcarbonyl, C₁₋₆-Alkoxycarboxyl, C₁₋₆-Alkyl, C₁₋₆-Halogenalkyl, C₁₋₆₋Alkenyl oder C₁₋₆-Alkinyl bedeuten;
oder R₁₅₃ und R₁₅₈ bilden gemeinsam mit den Ringatomen, an die sie gebunden sind, einen fünf- oder sechsgliedrigen teilgesättigten oder ungesättigten Ring, der bis zu 2 gleiche oder verschiedene Heteroatome aus der Gruppe Sauerstoff, Schwefel und Stickstoff enthalten kann, wobei dieser Ring durch einen Rest Oxo substituiert sein kann;
enthält.

7. Mittel nach Anspruch 6, **dadurch gekennzeichnet, daß** es eine herbizid-antagonistisch wirksamen Menge eines Safeners der Formel X, XI, XII, XIII, XIV, XV, XVI, XVII, XVIII, XIX, XX, XXI, XXII, XXIII, XXIV oder XXV enthält.

8. Verfahren zum selektiven Bekämpfen von Unkräutern und Gräsern in Nutzpflanzenkulturen, **dadurch gekennzeichnet, daß** man die Nutzpflanzen, deren Samen oder Stecklinge oder deren Anbaufläche mit einer herbizid wirksamen Menge eines Herbizids der Formel I und einer herbizid-antagonistisch wirksamen Menge eines Safeners der Formel X, XI, XII, XIII, XIV, XV, XVI, XVII, XVIII, XIX, XX, XXI, XXII, XXIII, XXIV, XXV, XXVI, XXVII, XXVIII oder XXIX behandelt.

9. Verfahren nach Anspruch 8, **dadurch gekennzeichnet, daß** man die Nutzpflanzen, deren Samen oder Stecklinge oder deren Anbaufläche mit einer herbizid-antagonistisch wirksamen Menge eines Safeners der Formel X, XI, XII, XIII, XIV, XV, XVI, XVII, XVIII, XIX, XX, XXI, XXII, XXIII, XXIV oder XXV behandelt.

10. Mittel nach Anspruch 3, **dadurch gekennzeichnet, daß** es Sprühtank-Adjuvantien enthält.

11. Mittel nach Anspruch 6, **dadurch gekennzeichnet, daß** es Sprühtank-Adjuvantien enthält.

## Claims

1. A compound of formula I 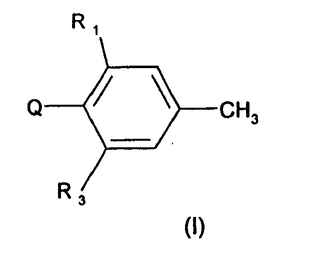 wherein
R₁ and R₃ are each independently of the other ethyl, haloethyl, ethynyl, C₁-C₂alkoxy, C₁-C₂haloalkoxy or C₁-C₂alkylcarbonyl;
Q is a group 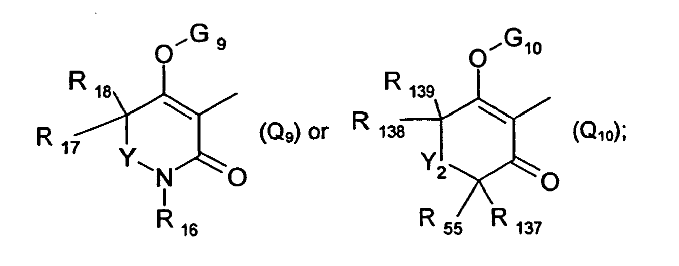 R₁₆ is C₁-C₁₀alkyl, C₂-C₁₀alkenyl, C₂-C₁₀alkynyl, C₁-C₁₀haloalkyl, C₂-C₁₀alkoxyalkyl, C₃-C₁₀₋alkenyloxyalkyl, C₃-C₁₀alkynyloxyalkyl, C₂-C₁₀alkylthioalkyl, C₂-C₁₀alkylsulfinylalkyl, C₂-C₁₀₋alkylsulfonylalkyl, C₃-C₁₀cycloalkyl, aryl or heteroaryl;
R₁₇ is C₁-C₁₀alkyl, C₂-C₁₀alkenyl, C₂-C₁₀alkynyl, C₁-C₁₀haloalkyl, C₂-C₁₀alkoxyalkyl, C₃-C₁₀₋alkenyloxyalkyl, C₃-C₁₀alkynyloxyalkyl, C₂-C₁₀alkylthioalkyl, C₂-C₁₀alkylsulfinylalkyl, C₂-C₁₀₋alkylsulfonylalkyl, C₂-C₁₀alkylcarbonylalkyl, C₃-C₁₀cycloalkyl, aryl or heteroaryl;
R₁₈ is hydrogen, C₂-C₁₀alkenyl, C₂-C₁₀alkynyl, C₁-C₁₀alkyl or C₁-C₁₀alkoxyalkyl; or
R₁₇ and R₁₈, together with the atoms to which they are bonded, form a 3- to 7-membered cyclic group that may contain one or two hetero atoms selected from nitrogen, oxygen and sulfur;
Y is oxygen or C-R₁₉;
R₁₉ is C₁-C₁₀alkyl, C₂-C₁₀alkenyl, C₂-C₁₀alkynyl, C₁-C₁₀haloalkyl, phenyl or heteroaryl; or R₁₈ and R₁₉, together with the atom to which they are bonded, form a saturated 5- to 7-membered cyclic group that may contain one or two hetero atoms selected from nitrogen, oxygen and sulfur,
G₉ and G₁₀ are each independently of the other hydrogen, -C(X₁)-R₂₀, -C(X₂)-X₃-R₂₁, -C(X₄)-N(R₂₂)-R₂₃, -SO₂-R₂₄, an alkali metal cation, alkaline earth metal cation, sulfonium cation or ammonium cation, -P(X₅)(R₂₅)-R₂₆ or -CH₂-X₆-R₂₇;
X₁, X₂, X₃, X₄, X₅ and X₆ are each independently of the others oxygen or sulfur;
R₂₀, R₂₁, R₂₂ and R₂₃ are each independently of the others hydrogen, C₁-C₁₀alkyl, C₂-C₁₀₋alkenyl, C₂-C₁₀alkynyl, C₁-C₁₀haloalkyl, C₁-C₁₀cyanoalkyl, C₁-C₁₀nitroalkyl, C₁-C₁₀aminoalkyl, C₁-C₅alkylamino-C₁-C₅alkyl, C₂-C₈dialkylamino-C₁-C₅alkyl, C₃-C₇cycloalkyl-C₁-C₅alkyl, C₂-C₁₀alkoxyalkyl, C₄-C₁₀alkenyloxyalkyl, C₄-C₁₀alkynyloxyalkyl, C₂-C₁₀alkylthioalkyl, C₁-C₅₋alkylsulfoxyl-C₁-C₅alkyl, C₁-C₅alkylsulfonyl-C₁-C₅alkyl, C₂-C₈alkylideneaminooxy-C₁-C₅alkyl, C₁-C₅alkylcarbonyl-C₁-C₅alkyl, C₁-C₅alkoxycarbonyl-C₁-C₅alkyl, C₁-C₅aminocarbonyl-C₁-C₅₋alkyl, C₂-C₈dialkylaminocarbonyl-C₁-C₅alkyl, C₁-C₅alkylcarbonylamino-C₁-C₅alkyl, C₁-C₅alkylcarbonyl-(C₂-C₅alkyl)-aminoalkyl, C₃-C₆trialkylsilyl-C₁-C₅alkyl, phenyl-C₁-C₅alkyl, heteroaryl-C₁-C₅alkyl, phenoxy-C₁-C₅alkyl, heteroaryloxy-C₁-C₅alkyl, C₂-C₅alkenyl, C₂-C₅haloalkenyl, C₃-C₈cycloalkyl, phenyl, or phenyl substituted by C₁-C₃alkyl, C₁-C₃haloalkyl, C₁-C₃alkoxy, C₁-C₃haloalkoxy, halogen, cyano or by nitro, or heteroaryl or heteroarylamino, or heteroaryl or heteroarylamino substituted by C₁-C₃alkyl, C₁-C₃haloalkyl, C₁-C₃alkoxy, C₁-C₃haloalkoxy, halogen, cyano or by nitro, diheteroarylamino, or diheteroarylamino substituted by C₁-C₃alkyl, C₁-C₃haloalkyl, C₁-C₃alkoxy, C₁-C₃haloalkoxy, halogen, cyano or by nitro, phenylamino, or phenylamino substituted by C₁-C₃alkyl, C₁-C₃haloalkyl, C₁-C₃alkoxy, C₁-C₃haloalkoxy, halogen, cyano or by nitro, diphenylamino, or diphenylamino substituted by C₁-C₃alkyl, C₁-C₃haloalkyl, C₁-C₃alkoxy, C₁-C₃haloalkoxy, halogen, cyano or by nitro, or C₃-C₇₋cycloalkylamino, di-C₃-C₇cycloalkylamino or C₃-C₇cycloalkoxy;
R₂₄, R₂₅ and R₂₆ are hydrogen, C₁-C₁₀alkyl, C₂-C₁₀alkenyl, C₂-C₁₀alkynyl, C₁-C₁₀haloalkyl, C₁-C₁₀cyanoalkyl, C₁-C₁₀nitroalkyl, C₁-C₁₀aminoalkyl, C₁-C₅alkylamino-C₁-C₅alkyl, C₂-C₈₋dialkylamino-C₁-C₅alkyl, C₃-C₇cycloalkyl-C₁-C₅alkyl, C₂-C₁₀alkoxyalkyl, C₄-C₁₀alkenyloxyalkyl, C₄-C₁₀alkynyloxyalkyl, C₂-C₁₀alkylthioalkyl, C₁-C₅alkylsulfoxyl-C₁-C₅alkyl, C₁-C₅alkylsulfonyl-C₁-C₅alkyl, C₂-C₈alkylideneaminooxy-C₁-C₅alkyl, C₁-C₅alkylcarbonyl-C₁-C₅alkyl, C₁-C₅alkoxycarbonyl-C₁-C₅alkyl, C₁-C₅aminocarbonyl-C₁-C₅alkyl, C₂-C₈dialkylaminocarbonyl-C₁-C₅alkyl, C₁-C₅alkylcarbonylamino-C₁-C₅alkyl, C₁-C₅alkylcarbonyl-(C₂-C₅alkyl)-aminoalkyl, C₃-C₆₋trialkylsilyl-C₁-C₅alkyl, phenyl-C₁-C₅alkyl, heteroaryl-C₁-C₅alkyl, phenoxy-C₁-C₅alkyl, heteroaryloxy-C₁-C₅alkyl, C₂-C₅alkenyl, C₂-C₅haloalkenyl, C₃-C₈cycloalkyl, phenyl, or phenyl substituted by C₁-C₃alkyl, C₁-C₃haloalkyl, C₁-C₃alkoxy, C₁-C₃haloalkoxy, halogen, cyano or by nitro, or heteroaryl or heteroarylamino, or heteroaryl or heteroarylamino substituted by C₁-C₃alkyl, C₁-C₃haloalkyl, C₁-C₃alkoxy, C₁-C₃haloalkoxy, halogen, cyano or by nitro, diheteroarylamino, or diheteroarylamino substituted by C₁-C₃alkyl, C₁-C₃haloalkyl, C₁-C₃₋alkoxy, C₁-C₃haloalkoxy, halogen, cyano or by nitro, phenylamino, or phenylamino substituted by C₁-C₃alkyl, C₁-C₃haloalkyl, C₁-C₃alkoxy, C₁-C₃haloalkoxy, halogen, cyano or by nitro, diphenylamino, or diphenylamino substituted by C₁-C₃alkyl, C₁-C₃haloalkyl, C₁-C₃₋alkoxy, C₁-C₃haloalkoxy, halogen, cyano or by nitro, or C₃-C₇cycloalkylamino, di-C₃-C₇cycloalkylamino, C₃-C₇cycloalkoxy, C₁-C₁₀alkoxy, C₁-C₁₀haloalkoxy, C₁-C₅alkylamino, C₂-C₈dialkylamino, benzyloxy or phenoxy, wherein the benzyl and phenyl groups may in turn be substituted by C₁-C₃alkyl, C₁-C₃haloalkyl, C₁-C₃alkoxy, C₁-C₃haloalkoxy, halogen, cyano or by nitro;
R₂₇ is C₁-C₁₀alkyl, C₂-C₁₀alkenyl, C₂-C₁₀alkynyl, C₁-C₁₀haloalkyl, C₁-C₁₀cyanoalkyl, C₁-C₁₀₋nitroalkyl, C₁-C₁₀aminoalkyl, C₁-C₅alkylamino-C₁-C₅alkyl, C₂-C₈dialkylamino-C₁-C₅alkyl, C₃-C₇cycloalkyl-C₁-C₅alkyl, C₂-C₁₀alkoxyalkyl, C₄-C₁₀alkenyloxyalkyl, C₄-C₁₀alkynyloxyalkyl, C₂-C₁₀alkylthioalkyl, C₁-C₅alkylsulfoxyl-C₁-C₅alkyl, C₁-C₅alkylsulfonyl-C₁-C₅alkyl, C₂-C₈alkyl-ideneaminooxy-C₁-C₅alkyl, C₁-C₅alkylcarbonyl-C₁-C₅alkyl, C₁-C₅alkoxycarbonyl-C₁-C₅alkyl, C₁-C₅aminocarbonyl-C₁-C₅alkyl, C₂-C₈dialkylaminocarbonyl-C₁-C₅alkyl, C₁-C₅alkylcarbonylamino-C₁-C₅alkyl, C₁-C₅alkylcarbonyl-(C₂-C₅alkyl)-aminoalkyl, C₃-C₆trialkylsilyl-C₁-C₅alkyl, phenyl-C₁-C₅alkyl, heteroaryl-C₁-C₅alkyl, phenoxy-C₁-C₅alkyl, heteroaryloxy-C₁-C₅alkyl, C₂-C₅alkenyl, C₂-C₅haloalkenyl, C₃-C₈cycloalkyl, phenyl, or phenyl substituted by C₁-C₃alkyl, C₁-C₃haloalkyl, C₁-C₃alkoxy, C₁-C₃haloalkoxy, halogen, cyano or by nitro, or heteroaryl or heteroarylamino, or heteroaryl or heteroarylamino substituted by C₁-C₃alkyl, C₁-C₃haloalkyl, C₁-C₃alkoxy, C₁-C₃haloalkoxy, halogen, cyano or by nitro, diheteroarylamino, diheteroarylamino substituted by C₁-C₃alkyl, C₁-C₃haloalkyl, C₁-C₃alkoxy, C₁-C₃haloalkoxy, halogen, cyano or by nitro, or phenylamino, phenylamino substituted by C₁-C₃alkyl, C₁-C₃haloalkyl, C₁-C₃alkoxy, C₁-C₃haloalkoxy, halogen, cyano or by nitro, diphenylamino, diphenylamino substituted by C₁-C₃alkyl, C₁-C₃haloalkyl, C₁-C₃alkoxy, C₁-C₃haloalkoxy, halogen, cyano or by nitro, or C₃-C₇cycloalkylamino, di-C₃-C₇cycloalkylamino, C₃-C₇cycloalkoxy or C₁-C₁₀alkylcarbonyl;
Y₂ is oxygen or sulfur,
R₅₅ is C₁-C₁₀alkyl, C₂-C₁₀alkenyl, C₂-C₁₀alkynyl, C₁-C₁₀haloalkyl, C₂-C₁₀alkoxyalkyl, C₃-C₁₀₋alkenyloxyalkyl, C₃-C₁₀alkynyloxyalkyl, C₂-C₁₀alkylthioalkyl, C₂-C₁₀alkylsulfinylalkyl, C₂-C₁₀₋alkylsulfonylalkyl, C₂-C₁₀alkylcarbonylalkyl, C₃-C₁₀cycloalkyl, aryl or heteroaryl;
R₁₃₇ is hydrogen, C₁-C₁₀alkyl, C₂-C₁₀alkenyl, C₂-C₁₀alkynyl or C₁-C₁₀alkoxyalkyl; or R₅₅ and R₁₃₇, together with the atoms to which they are bonded, form a 3- to 7-membered cyclic group that may contain one or two hetero atoms selected from nitrogen, oxygen and sulfur; and
R₁₃₈ and R₁₃₉ are each independently of the other hydrogen, C₁-C₁₀alkyl, C₂-C₁₀alkenyl, C₂-C₁₀alkynyl or C₂-C₁₀alkoxyalkyl;
or compound No. 21.115,
or an agronomically tolerable salt, isomer or enantiomer of such a compound.

2. A process for the preparation of a compound of formula I according to claim 1, wherein a compound of formula XXX
Q-H (XXX)
wherein Q is Q₉ or Q₁₀, the substituents of which, with the exception of G₉ and G₁₀, have the meanings given above, and G₉ and G₁₀ are hydrogen, is reacted with a compound of formula XXXI 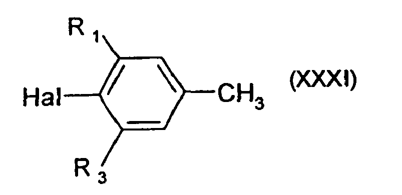 wherein R₁ and R₃ are as defined for formula I and Hal is chlorine, bromine or iodine, in the presence of an inert solvent, a base and a palladium catalyst, at temperatures of from 30 to 250°C.

3. A herbicidal and plant growth-inhibiting composition that comprises a herbicidally effective amount of a compound of formula I on an inert carrier.

4. A method of controlling undesired plant growth that comprises applying a herbicidally effective amount of an active ingredient of formula I, or of a composition comprising such an active ingredient, to the plants or to the locus thereof.

5. A method of inhibiting plant growth that comprises applying a herbicidally effective amount of an active ingredient of formula I, or of a composition comprising such an active ingredient, to the plants or to the locus thereof.

6. A selective-herbicidal composition that comprises as active ingredient, in addition to customary inert formulation adjuvants, a mixture of
a) a herbicidally effective amount of a compound of formula I according to claim 1 and
b) a herbicide-antagonistically effective amount either of a compound of formula X 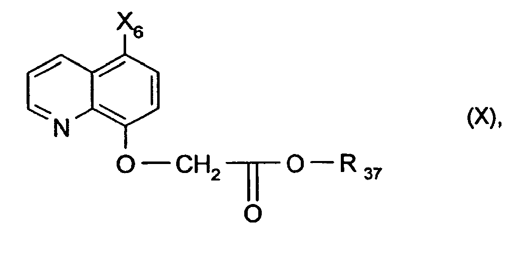
wherein
R₃₇ is hydrogen, C₁-C₈alkyl, or C₁-C₈alkyl substituted by C₁-C₆alkoxy or by C₃-C₆alkenyloxy; and X₆ is hydrogen or chlorine; or of a compound of formula XI 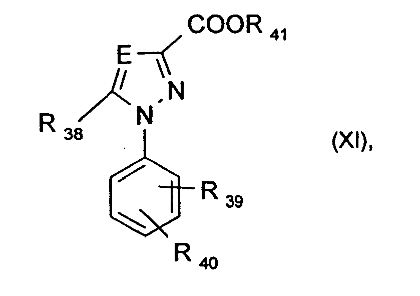 wherein
E is nitrogen or methine;
R₃₈ is -CCl₃, phenyl or phenyl substituted by halogen;
R₃₉ and R₄₀ are each independently of the other hydrogen or halogen; and
R₄₁ is C₁-C₄alkyl; or of a compound of formula XII 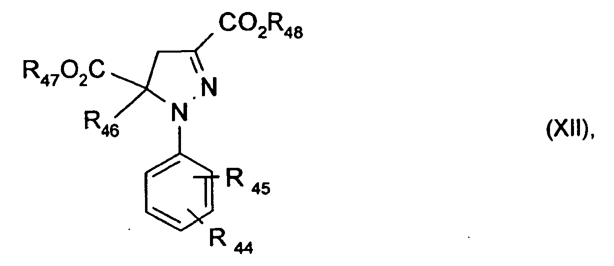 wherein R₄₄ and R₄₅ are each independently of the other hydrogen or halogen, and
R₄₆, R₄₇ and R₄₈ are each independently of the others C₁-C₄alkyl, or of a compound of formula XIII 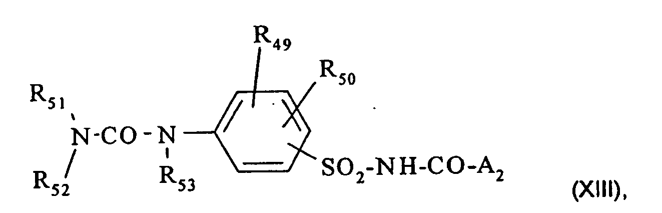 wherein A₂ is a group 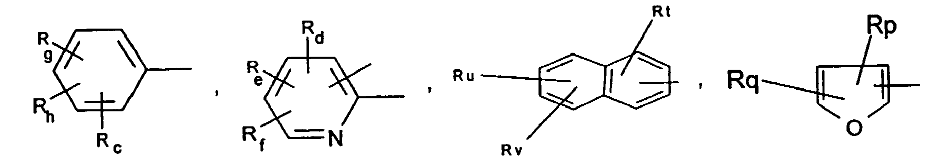 or 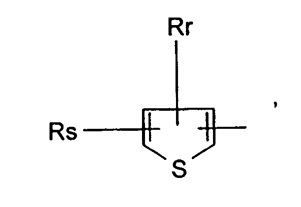 R₅₁ and R₅₂ are each independently of the other hydrogen, C₁-C₈alkyl, C₃-C₈cycloalkyl, C₃-C₆alkenyl, C₃-C₆alkynyl, 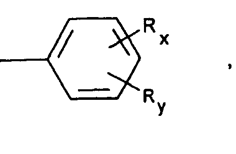 or C₁-C₄alkyl substituted by C₁-C₄alkoxy or by 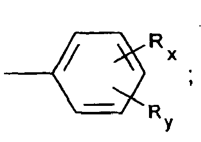 or R₅₁ and R₅₂ together form a C₄-C₆alkylene bridge that may be interrupted by oxygen, sulfur, SO, SO₂, NH or by -N(C₁-C₄alkyl)-;
R₅₃ is hydrogen or C₁-C₄alkyl;
R₄₉ is hydrogen, halogen, cyano, trifluoromethyl, nitro, C₁-C₄alkyl, C₁-C₄alkoxy, C₁-C₄alkylthio, C₁-C₄alkylsulfinyl, C₁-C₄alkylsulfonyl, -COORⱼ. -CONRₖRₘ, -CORₙ, -SO₂NRₖRₘ or -OSO₂-C₁-C₄alkyl;
R₉ is hydrogen, halogen, cyano, nitro, C₁-C₄alkyl, C₁-C₄haloalkyl, C₁-C₄alkylthio, C₁-C₄alkylsulfinyl, C₁-C₄alkylsulfonyl, -COORⱼ. -CONRₖRₘ, -CORₙ, -SO₂NRₖRₘ, -OSO₂-C₁-C₄alkyl, C₁-C₆alkoxy, or C₁-C₆alkoxy substituted by C₁-C₄alkoxy or by halogen, C₃-C₆alkenyloxy, or C₃-C₆alkenyloxy substituted by halogen, or C₃-C₆alkynyloxy, or R₄₉ and R₅₀ together form a C₃-C₄alkylene bridge that may be substituted by halogen or by C₁-C₄alkyl, or together form a C₃-C₄alkenylene bridge that may be substituted by halogen or by C₁-C₄alkyl, or together form a C₄alkadienylene bridge that may be substituted by halogen or by C₁-C₄alkyl;
R₅₀ and Rₕ are each independently of the other hydrogen, halogen, C₁-C₄alkyl, trifluoromethyl, C₁-C₆alkoxy, C₁-C₆alkylthio or -COORⱼ;
R_{c} is hydrogen, halogen, nitro, C₁-C₄alkyl or methoxy; R_{d} is hydrogen, halogen, nitro, C₁-C₄alkyl, C₁-C₄alkoxy, C₁-C₄alkylthio, C₁-C₄alkylsulfinyl, C₁-C₄alkylsulfonyl, -COORⱼ or CONRₖRₘ;
Rₑ is hydrogen, halogen, C₁-C₄alkyl, -COORⱼ, trifluoromethyl or methoxy, or R_{d} and Rₑ together form a C₃-C₄alkylene bridge;
Rp is hydrogen, halogen, C₁-C₄alkyl, -COORⱼ, trifluoromethyl or methoxy; Rq is hydrogen, halogen, nitro, C₁-C₄alkyl, C₁-C₄alkoxy, C₁-C₄alkylthio, C₁-C₄alkylsulfinyl, C₁-C₄alkylsulfonyl, -COORⱼ or CONRₖRₘ; or Rp and Rq together form a C₃-C₄alkylene bridge;
Rr is hydrogen, halogen, C₁-C₄alkyl, -COORⱼ, trifluoromethyl or methoxy; Rs is hydrogen, halogen, nitro, C₁-C₄alkyl, C₁-C₄alkoxy, C₁-C₄alkylthio, C₁-C₄alkylsulfinyl, C₁-C₄alkylsulfonyl, -COORⱼ or CONRₖRₘ; or Rr and Rs together form a C₃-C₄alkylene bridge;
Rt is hydrogen, halogen, C₁-C₄alkyl, -COORⱼ, trifluoromethyl or methoxy; Ru is hydrogen, halogen, nitro, C₁-C₄alkyl, C₁-C₄alkoxy, C₁-C₄alkylthio, C₁-C₄alkylsulfinyl, C₁-C₄alkylsulfonyl, -COORⱼ or CONRₖRₘ; or Rv and Ru together form a C₃-C₄alkylene bridge;
R_{f} and Rv are hydrogen, halogen or C₁-C₄alkyl;
Rₓ and R_{y} are each independently of the other hydrogen, halogen, C₁-C₄alkyl, C₁-C₄alkoxy, C₁-C₄alkylthio, -COOR₅₄, trifluoromethyl, nitro or cyano;
Rⱼ, Rₖ and Rₘ are each independently of the others hydrogen or C₁-C₄alkyl; or Rₖ and Rₘ together form a C₄-C₆alkylene bridge that may be interrupted by oxygen, NH or by -N(C₁-C₄alkyl)-;
Rₙ is C₁-C₄alkyl, phenyl, or phenyl substituted by halogen, C₁-C₄alkyl, methoxy, nitro or by trifluoromethyl;
R₅₄ is hydrogen, C₁-C₁₀alkyl, C₁-C₄alkoxy-C₁-C₄alkyl, C₁-C₄alkylthio-C₁-C₄alkyl, di-C₁-C₄₋alkylamino-C₁-C₄alkyl, halo-C₁-C₈alkyl, C₂-C₈alkenyl, halo-C₂-C₈alkenyl, C₃-C₈alkynyl, C₃-C₇cycloalkyl, halo-C₃-C₇cycloalkyl, C₁-C₈alkylcarbonyl, allylcarbonyl, C₃-C₇cycloalkylcarbonyl, benzoyl which is unsubstituted or substituted on the phenyl ring identically or differently up to three times by halogen, C₁-C₄alkyl, halo-C₁-C₄alkyl, halo-C₁-C₄alkoxy or by C₁-C₄alkoxy; or furoyl, thienyl; or C₁-C₄alkyl substituted by phenyl, halophenyl, C₁-C₄alkylphenyl, C₁-C₄alkoxyphenyl, halo-C₁-C₄alkylphenyl, halo-C₁-C₄alkoxyphenyl, C₁-C₆alkoxycarbonyl, C₁-C₄alkoxy-C₁-C₈alkoxycarbonyl, C₃-C₈alkenyloxycarbonyl, C₃-C₈alkynyloxycarbonyl, C₁-C₈alkylthiocarbonyl, C₃-C₈alkenylthiocarbonyl, C₃-C₈alkynylthiocarbonyl, carbamoyl, mono-C₁-C₄alkylaminocarbonyl, di-C₁-C₄alkylaminocarbonyl; or phenylaminocarbonyl, which is unsubstituted or substituted on the phenyl identically or differently up to three times by halogen, C₁-C₄alkyl, halo-C₁-C₄alkyl, halo-C₁-C₄alkoxy or C₁-C₄alkoxy or once by cyano or nitro; or dioxolan-2-yl, which is unsubstituted or substituted by one or two C₁-C₄alkyl radicals, or dioxan-2-yl, which is unsubstituted or substituted by one or two C₁-C₄alkyl radicals, or C₁-C₄alkyl, which is substituted by cyano, nitro, carboxyl or by C₁-C₈₋alkylthio-C₁-C₈alkoxycarbonyl;
or of a compound of formula XIV 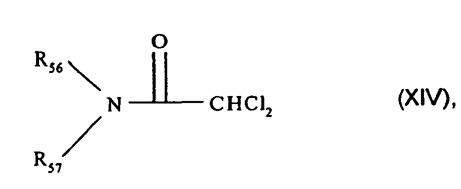 wherein R₅₆ and R₅₇ are each independently of the other C₁-C₆alkyl or C₂-C₆alkenyl; or R₅₆ and R₅₇ together are 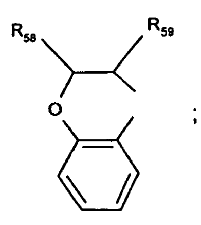 R₅₈ and
R₅₉ are each independently of the other hydrogen or C₁-C₆alkyl; or R₅₆ and R₅₇ together are 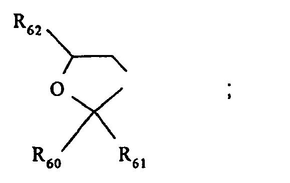 R₆₀ and R₆₁ are each independently of the other C₁-C₄alkyl, or R₆₀ and R₆₁ together are -(CH₂)₅-;
R₆₂ is hydrogen, C₁-C₄alkyl or 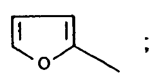 or R₅₆ and R₅₇ together are 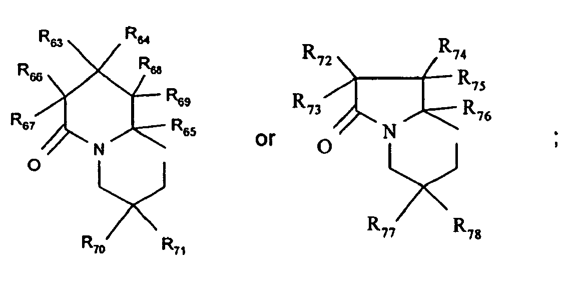 R₆₃, R₆₄, R₆₅, R₆₆, R₆₇, R₆₈, R₆₉, R₇₀, R₇₁, R₇₂, R₇₃, R₇₄, R₇₅, R₇₆, R₇₇ and R₇₈ are each independently of the others hydrogen or C₁-C₄alkyl;
or of a compound of formula XV 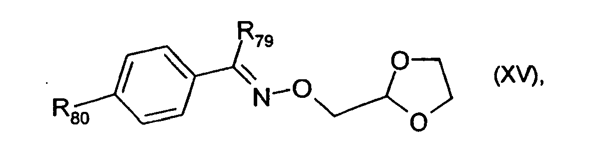 wherein R₈₀ is hydrogen or chlorine and R₇₉ is cyano or trifluoromethyl;
or of a compound of formula XVI 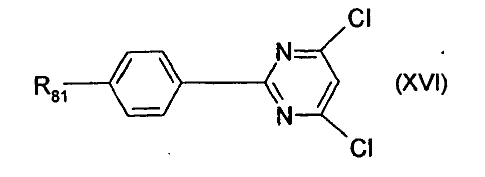 wherein R₈₁ is hydrogen or methyl;
or of a compound of formula XVII 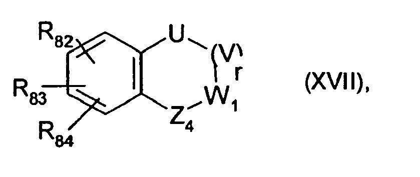 wherein
R₈₂ is hydrogen, C₁-C₄alkyl, or C₁-C₄alkyl substituted by C₁-C₄alkyl-X₂- or by C₁-C₄haloalkyl-X₂-, or is C₁-C₄haloalkyl, nitro, cyano, -COOR₈₅, -NR₈₆R₈₇, -SO₂NR₈₈R₈₉ or -CONR₉₀R₉₁ ; R₈₃ is hydrogen, halogen, C₁-C₄alkyl, trifluoromethyl, C₁-C₄alkoxy or C₁-C₄haloalkoxy;
R₈₄ is hydrogen, halogen or C₁-C₄alkyl;
U, V, W₁ and Z₄ are each independently of the others oxygen, sulfur, C(R₉₂)R₉₃, carbonyl,
NR₉₄, or a group 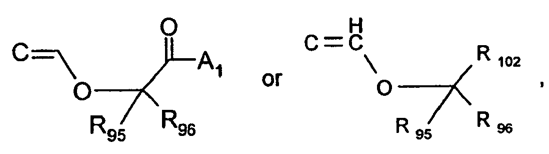 wherein R₁₀₂ is C₂-C₄alkenyl or C₂-C₄alkynyl; with the provisos that
a) at least one of the ring members U, V, W₁ or Z₄ is carbonyl, and a ring member adjacent to that ring member or to those ring members is the group 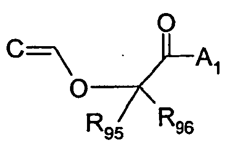 or 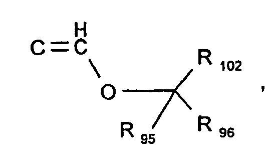 that group appearing only once; and
b) two adjacent ring members U and V, V and W₁ and W₁ and Z₄ cannot simultaneously be oxygen;
R₉₅ and R₉₆ are each independently of the other hydrogen or C₁-C₈alkyl; or
R₉₅ and R₉₆ together form a C₂-C₆alkylene group;
A₁ is R₉₉-Y₁- or -NR₉₇R₉₈;
X₂ is oxygen or -S(O)ₛ;
Y₁ is oxygen or sulfur;
R₉₉ is hydrogen, C₁-C₈alkyl, C₁-C₈haloalkyl, C₁-C₄alkoxy-C₁-C₈alkyl, C₃-C₆alkenyloxy-C₁-C₈₋alkyl, or phenyl-C₁-C₈alkyl in which the phenyl ring may be substituted by halogen, C₁-C₄₋alkyl, trifluoromethyl, methoxy or by methyl-S(O)ₛ-, or is C₃-C₆alkenyl, C₃-C₆haloalkenyl, phenyl-C₃-C₆alkenyl, C₃-C₆alkynyl, phenyl-C₃-C₆alkynyl, oxetanyl, furyl or tetrahydrofuryl;
R₈₅ is hydrogen or C₁-C₄alkyl;
R₈₆ is hydrogen, C₁-C₄alkyl or C₁-C₄alkylcarbonyl;
R₈₇ is hydrogen or C₁-C₄alkyl; or
R₈₆ and R₈₇ together form a C₄- or C₅-alkylene group;
R₈₈, R₈₉, R₉₀ and R₉₁, are each independently of the others hydrogen or C₁-C₄alkyl; or R₈₈ together with R₈₉, or R₉₀ together with R₉₁, are each independently of the other C₄- or C₅₋alkylene in which one carbon atom may have been replaced by oxygen or by sulfur, or one or two carbon atoms may have been replaced by -NR₁₀₀-;
R₉₂, R₁₀₀ and R₉₃ are each independently of the others hydrogen or C₁-C₈alkyl; or
R₉₂ and R₉₃ together are C₂-C₆alkylene;
R₉₄ is hydrogen or C₁-C₈alkyl;
R₉₇ is hydrogen, C₁-C₈alkyl, phenyl or phenyl-C₁-C₈alkyl, wherein the phenyl rings may be substituted by fluorine, chlorine, bromine, nitro, cyano, -OCH₃, C₁-C₄alkyl or by CH₃SO₂-, or is C₁-C₄alkoxy-C₁-C₈alkyl, C₃-C₈alkenyl or C₃-C₆alkynyl;
R₉₈ is hydrogen, C₁-C₈alkyl, C₃-C₆alkenyl or C₃-C₆alkynyl; or
R₉₇ and R₉₈ together are C₄- or C₅-alkylene in which one carbon atom may have been replaced by oxygen or by sulfur, or one or two carbon atoms may have been replaced by -NR₁₀₁-;
R₁₀₁ is hydrogen or C₁-C₄alkyl;
r is 0 or 1; and
s is 0, 1 or 2,
or of a compound of formula XVIII 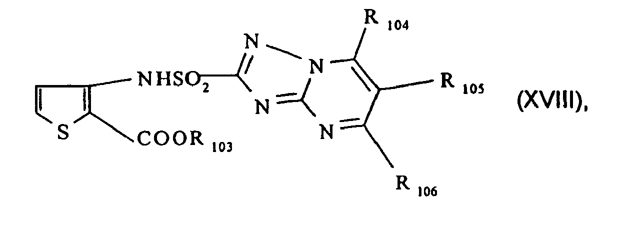 wherein R₁₀₃ is hydrogen, C₁-C₆alkyl, C₃-C₆cycloalkyl, C₃-C₆alkenyl or C₃-C₆alkynyl; and R₁₀₄, R₁₀₅ and R₁₀₆ are each independently of the others hydrogen, C₁-C₆alkyl, C₃-C₆cycloalkyl or
C₁-C₆alkoxy, with the proviso that one of the substituents R₁₀₄, R₁₀₅ and R₁₀₆ is other than hydrogen;
or of a compound of formula XIX 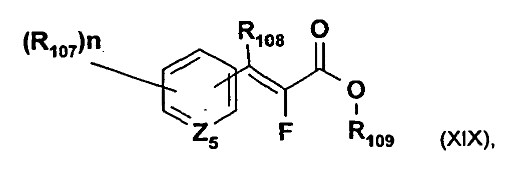 wherein Z₅ is N or CH, n is 0, 1, 2 or 3 when Z₅ is N, and n is 0, 1, 2, 3 or 4 when Z₅ is CH, R₁₀₇ is halogen, C₁-C₄alkyl, C₁-C₄haloalkyl, C₁-C₄alkoxy, C₁-C₄haloalkoxy, nitro, C₁-C₄alkylthio, C₁-C₄alkylsulfonyl, C₁-C₄alkoxycarbonyl, phenyl or phenoxy, or phenyl or phenoxy substituted by C₁-C₃alkyl, C₁-C₃haloalkyl, C₁-C₃alkoxy, C₁-C₃haloalkoxy, halogen, cyano or by nitro;
R₁₀₈ is hydrogen or C₁-C₄alkyl, R₁₀₉ is hydrogen, C₁-C₄alkyl, C₃-C₆cycloalkyl, C₂-C₆alkenyl, C₂-C₆,alkynyl, C₁-C₄haloalkyl, C₂-C₆haloalkenyl, C₂-C₆haloalkynyl, C₁-C₄alkylthio-C₁-C₄alkyl, C₁-C₄alkylsulfonyl-C₁-C₄alkyl, C₁-C₄alkoxy-C₁-C₄alkyl, C₁-C₄alkenyloxy-C₁-C₄alkyl or C₁-C₄₋alkynyloxy-C₁-C₄alkyl;
or of a compound of formula XX 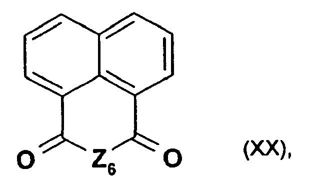 wherein Z₆ is oxygen or N-R₁₁₀ and R₁₁₀ is a group of formula 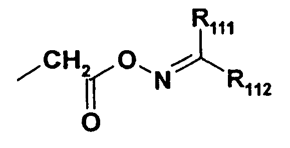 wherein R₁₁₁ and R₁₁₂ are each independently of the other cyano, hydrogen, C₁-C₄alkyl, C₃-C₆cycloalkyl, C₂-C₆alkenyl, aryl, phenyl or heteroaryl, or phenyl, aryl or heteroaryl substituted by C₁-C₃alkyl, C₁-C₃haloalkyl, C₁-C₃alkoxy, C₁-C₃haloalkoxy, halogen, cyano or by nitro;
or of a compound of formula XXI 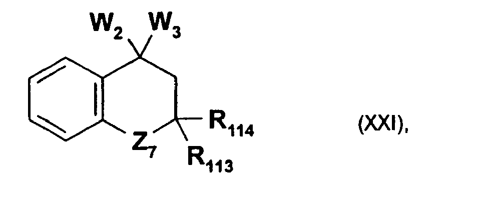 wherein Z₇ is oxygen, sulfur, S=O, SO₂ or CH₂, R₁₁₃ and R₁₁₄ are each independently of the other hydrogen, halogen or C₁-C₄alkyl, W₂ and W₃ are each independently of the other CH₂COOR₁₁₅ or COOR₀₁₁₅ or together are a group of formula -(CH₂)C(O)-O-C(O)-(CH₂)-, and R₁₁₅ and R₀₁₁₅ are each independently of the other hydrogen, C₁-C₄alkyl, C₂-C₄alkenyl, C₂-C₆₋alkynyl, C₃-C₆cycloalkyl, C₁-C₄haloalkyl, or a metal cation or an ammonium cation;
or of a compound of formula XXII 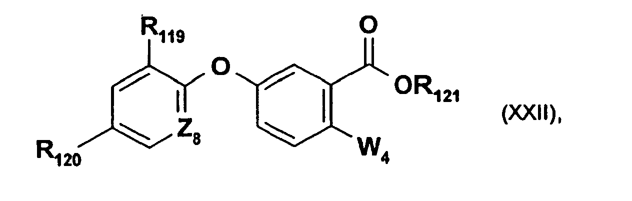 wherein R₁₁₉ and R₁₂₀ are each independently of the other hydrogen, halogen or C₁-C₄haloalkyl, R₁₂₁ is hydrogen, C₁-C₄alkyl, C₃-C₄alkenyl, C₃-C₄alkynyl, C₁-C₄haloalkyl, C₃-C₆cycloalkyl, a metal cation or an ammonium cation, Z₈ is N, CH, C-F or C-Cl and W₄ is a group of formula 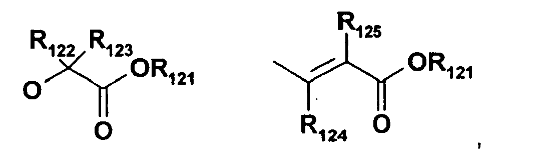 wherein R₁₂₂ and R₁₂₃ are each independently of the other hydrogen or C₁-C₄alkyl and R₁₂₄ and R₁₂₅ are each independently of the other hydrogen or C₁-C₄alkyl;
or of a compound of formula XXIII 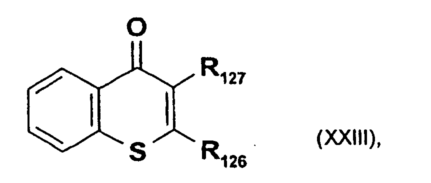 wherein R₁₂₆ is hydrogen, cyano, halogen, C₁-C₄alkyl, C₃-C₆cycloalkyl, C₁-C₄alkoxy, C₁-C₄₋alkoxycarbonyl, C₁-C₄alkylthiocarbonyl, -NH-R₁₂₈, -C(O)NH-R₀₁₂₈, aryl or heteroaryl, or aryl or heteroaryl substituted by C₁-C₃alkyl, C₁-C₃haloalkyl, C₁-C₃alkoxy, C₁-C₃haloalkoxy, halogen, cyano or by nitro;
R₁₂₇ is hydrogen, cyano, nitro, halogen, C₁-C₄alkyl, C₁-C₄haloalkyl, C₁-C₄alkoxy or C₁-C₄thioalkyl; and
R₁₂₈ and R₀₁₂₈ are each independently of the other C₁-C₄alkyl, C₁-C₄haloalkyl, C₃-C₄alkenyl, C₃-C₄alkynyl, C₃-C₄cycloalkyl, aryl or heteroaryl, or aryl or heteroaryl substituted by C₁-C₃₋alkyl, C₁-C₃haloalkyl, C₁-C₃alkoxy, C₁-C₃haloalkoxy, halogen, cyano or by nitro, or formyl, C₁-C₄alkylcarbonyl or C₁-C₄alkylsufonyl;
or of a compound of formula XXIV 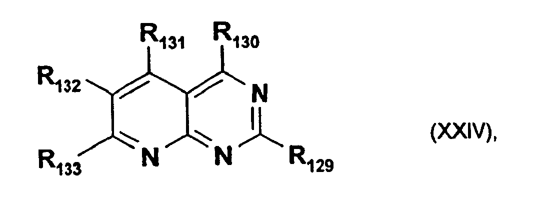 wherein R₁₂₉ and R₁₃₀ are each independently of the other hydrogen, C₁-C₄alkyl, C₁-C₄haloalkyl, C₁-C₄alkoxy, mono-C₁-C₈- or di-C₁-C₈-alkylamino, C₃-C₆cycloalkyl, C₁-C₄thioalkyl, phenyl or heteroaryl, R₁₃₁ has the meanings of R₁₂₉ and in addition is OH, NH₂, halogen, di-C₁-C₄aminoalkyl, C₁-C₄alkylthio, C₁-C₄alkylsulfonyl or C₁-C₄alkoxycarbonyl, R₁₃₂ has the meanings of R₁₂₉ and in addition is cyano, nitro, carboxyl, C₁-C₄alkoxycarbonyl, di-C₁-C₄₋aminoalkyl, C₁-C₄alkylthio, C₁-C₄alkylsulfonyl, SO₂-OH, i-C₁-C₄aminoalkylsulfonyl or C₁-C₄₋alkoxysulfonyl, R₁₃₃ has the meanings of R₁₂₉ and in addition is OH, NH₂, halogen, di-C₁-C₄₋aminoalkyl, pyrrolidin-1-yl, piperidin-1-yl, morpholin-1-yl, C₁-C₄alkylthio, C₁-C₄alkylsulfonyl, C₁-C₄alkoxycarbonyl, phenoxy, naphthyloxy, phenylamino, benzoyloxy or phenylsulfonyloxy;
or of a compound of formula XXV 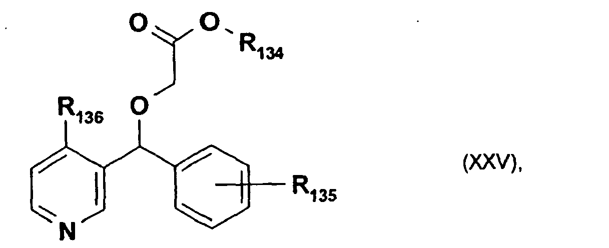 wherein R₁₃₄ is hydrogen, C₄alkyl, C₁-C₄haloalkyl, C₂-C₄alkenyl, C₂-C₄alkynyl or C₁-C₄alkoxy-C₁-C₄alkyl, R₁₃₅ is hydrogen, halogen, C₁-C₄alkyl, C₁-C₄haloalkyl or C₁-C₄alkoxy and R₁₃₆ is hydrogen, halogen, C₁-C₄alkyl, C₁-C₄haloalkyl or C₁-C₄alkoxy, with the proviso that R₁₃₅ and
R₁₃₆ are not simultaneously hydrogen,
or of formula XXVI 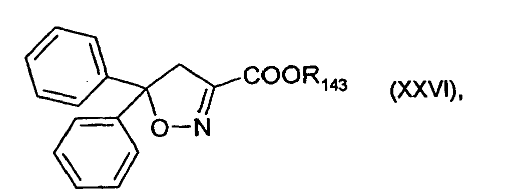 wherein
R₁₄₃ is hydrogen, an alkali metal cation, alkaline earth metal cation, sulfonium cation or ammonium cation or ethyl;
or of formula XXVII 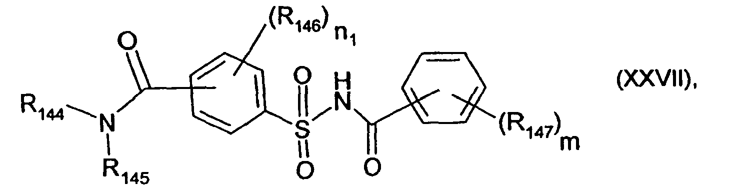 wherein R₁₄₄ and R₁₄₅ are each independently of the other hydrogen, C₁-C₆alkyl, C₂-C₆₋alkenyl, C₂-C₆alkynyl or C₃-C₆cycloalkyl;
R₁₄₆ is hydrogen, halogen, C₁-C₄alkyl, C₁-C₆haloalkyl or C₁-C₆haloalkoxy;
R₁₄₇ is hydrogen, halogen, C₁-C₄alkyl, C₁-C₄haloalkyl, C₁-C₄alkoxy, C₁-C₄haloalkoxy, C₁-C₄₋alkylthio, C₁-C₄alkoxycarbonyl or nitro;
n₁ is 0, 1, 2 or 3; and
m is 1 or 2;
or of formula XXVIII 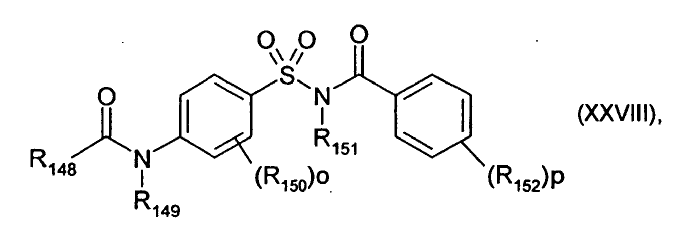 wherein
R₁₄₈ is hydrogen, C₁-C₆alkyl, C₁-C₆alkoxy, C₁-C₆alkylthio, C₃-C₈cycloalkyl, phenyl, phenyl-C₁-C₆alkyl or heteroaryl; wherein the said groups may be substituted by halogen, cyano, nitro, amino, hydroxy, carbonyl, carboxyl, formyl, carbonamide or by sulfonamide;
R₁₄₉ is hydrogen, C₁-C₆alkyl or C₁-C₄haloalkyl;
each R₁₅₀ is independently of any other(s) hydrogen, halogen, C₁-C₄alkyl, C₁-C₄haloalkyl, C₁-C₄alkoxy, C₁-C₄alkylthio, C₁-C₄alkylsulfonyl, cyano, nitro, formyl or carboxyl;
R₁₅₁ is hydrogen, C₁-C₆alkyl or C₁-C₄haloalkyl;
each R₁₅₂ is independently of any other(s) hydrogen, halogen, C₁-C₄alkyl, C₁-C₄haloalkyl, C₁-C₄alkoxy, C₁-C₄alkylthio, C₁-C₄alkylsulfonyl, cyano, nitro, formyl or carboxyl;
o is 0, 1, or 2, and
p is 0, 1 or 2;
or of formula XXIX 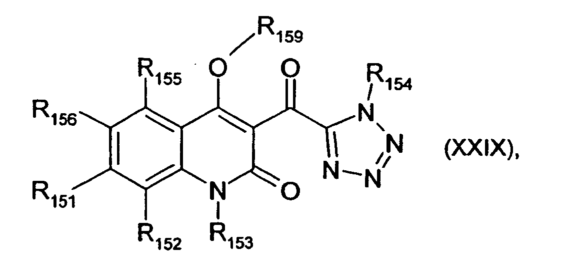 wherein
R₁₅₉ is hydrogen, formyl, C₁₋₆alkylcarbonyl, C₁₋₆alkenylcarbonyl, C₁₋₆alkynylcarbonyl, C₁₋₆alkoxycarbonyl, C₁₋₆alkylthiocarbonyl, C₃₋₈cycloalkylcarbonyl, phenyl-C₁₋₆alkylcarbonyl, phenylcarbonyl, C₁₋₆alkylsulfonyl, C₁₋₆alkenylsulfonyl or phenylsulfonyl, wherein the aforementioned hydrocarbon groups may be substituted by one or more halogen atoms, cyano, nitro, amino, methoxy, ethoxy or phenyl;
R₁₅₃ is hydrogen, C₁₋₆alkyl, C₁₋₆alkenyl, C₁₋₆alkynyl, C₃₋₈cycloalkyl, formyl, C₁₋₆alkylcarbonyl, C₁₋₆alkenylcarbonyl, C₁₋₆alkynylcarbonyl, C₁₋₆alkoxycarbonyl, C₁₋₆alkylthiocarbonyl, C₃₋₈cycloalkylcarbonyl, C₁₋₆alkylsulfonyl, C₁₋₆alkenylsulfonyl or phenylsulfonyl, wherein the aforementioned hydrocarbon groups may be substituted by one or more halogen atoms, cyano, nitro, amino, methoxy, ethoxy or phenyl;
R₁₅₄ is hydrogen, C₁₋₆alkyl, C₁₋₆alkenyl, C₁₋₆alkynyl, C₃₋₈cycloalkyl, formyl, C₁₋₆alkylcarbonyl, C₁₋₆alkenylcarbonyl, C₁₋₆alkynylcarbonyl, C₁₋₆alkoxycarbonyl, C₁₋₆alkylthiocarbonyl, C₃₋₈cycloalkylcarbonyl, C₁₋₆alkylsulfonyl, C₁₋₆alkenylsulfonyl or phenylsulfonyl, wherein the aforementioned hydrocarbon groups may be substituted by one or more halogen atoms, cyano, nitro, amino, methoxy, ethoxy or phenyl;
R₁₅₅, R₁₅₆, R₁₅₇ and R₁₅₈ are each independently of the others hydrogen, halogen, amino, C₁₋₃alkylamino, C₁₋₆dialkylamino, hydroxy, cyano, nitro, formyl, carboxyl, C₁₋₆alkoxy, C₁₋₆haloalkoxy, C₁₋₆alkylcarbonyl, C₁₋₆alkoxycarboxyl, C₁₋₆alkyl, C₁₋₆haloalkyl, C₁₋₆alkenyl or C₁₋₆alkynyl;
or R₁₅₃ and R₁₅₈, together with the ring atoms to which they are bonded, form a five- or six-membered, partially saturated or unsaturated ring that may contain up to 2 identical or different hetero atoms from the group oxygen, sulfur and nitrogen, it being possible for that ring to be substituted by an oxo radical.

7. A composition according to claim 6 that comprises a herbicide-antagonistically effective amount of a safener of formula X, XI, XII, XIII, XIV, XV, XVI, XVII, XVIII, XIX, XX, XXI, XXII, XXIII, XXIV or XXV.

8. A method of selectively controlling weeds and grasses in crops of useful plants that comprises treating the useful plants, the seeds or the cuttings thereof or the area of cultivation thereof with a herbicidally effective amount of a herbicide of formula and of a herbicide-antagonistically effective amount of a safener of formula X, XI, XII, XIII, XIV, XV, XVI, XVII, XVIII, XIX, XX, XXI, XXII, XXIII, XXIV, XXV, XXVI, XXVII, XXVIII or XXIX.

9. A method according to claim 8 that comprises treating the useful plants, the seeds or cuttings thereof or the area of cultivation thereof with a herbicide-antagonistically effective amount of a safener of formula X, XI, XII, XIII, XIV, XV, XVI, XVII, XVIII, XIX, XX, XXI, XXII, XXIII, XXIV or XXV.

10. A composition according to claim 3 that includes spray tank adjuvants.

11. A composition according to claim 6 that includes spray tank adjuvants.

## Revendications

1. Composés de formule I 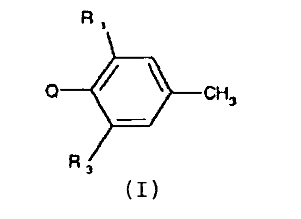 dans laquelle
R₁ et R₃ représentent indépendamment l'un de l'autre un éthyle, halogénoéthyle, éthynyle, alcoxy en C₁ à C₂, halogénoalcoxy en C₁ à C₂ ou (alkyle en C₁ à
C₂)carbonyle ;
Q représente un groupe 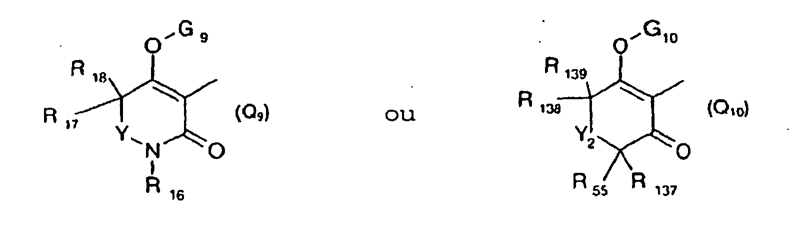 R₁₆ représente un alkyle en C₁ à C₁₀, alcényle en C₂ à C₁₀, alcynyle en C₂ à C₁₀, halogénoalkyle en C₁ à C₁₀, alcoxyalkyle en C₂ à C₁₀, alcényloxyalkyle en C₃ à C₁₀, alcynyloxyalkyle en C₃ à C₁₀, alkylthioalkyle en C₂ à C₁₀, alkylsulfinylalkyle en C₂ à C₁₀, alkylsulfonylalkyle en C₂ à C₁₀, cycloalkyle en C₃ à C₁₀, aryle ou hétéroaryle ;
R₁₇ représente un alkyle en C₁ à C₁₀, alcényle en C₂ à C₁₀, alcynyle en C₂ à C₁₀, halogénoalkyle en C₁ à C₁₀, alcoxyalkyle en C₂ à C₁₀, alcényloxyalkyle en C₃ à C₁₀, alcynyloxyalkyle en C₃ à C₁₀, alkylthioalkyle en C₂ à C₁₀, alkylsulfinylalkyle en C₂ à C₁₀, alkylsulfonylalkyle en C₂ à C₁₀, (alkylcarbonyle en C₂ à C₁₀)-alkyle, cycloalkyle en C₃ à C₁₀, aryle ou hétéroaryle ;
R₁₈ représente un hydrogène, alcényle en C₂ à C₁₀, alcynyle en C₂ à C₁₀, alkyle en C₁ à C₁₀ ou (alcoxy en C₁ à C₁₀)alkyle ; ou
R₁₇ et R₁₈ forment ensemble, avec les atomes auxquels ils sont liés, un cycle à 3 à 7 chaînons, qui peut contenir un ou deux hétéroatomes choisis parmi l'azote, l'oxygène et le soufre ;
Y représente un oxygène ou C-R₁₉,
R₁₉ représente un alkyle en C₁ à C₁₀, alcényle en C₂ à C₁₀, alcynyle en C₂ à C₁₀, halogénoalkyle en C₁ à C₁₀, phényle ou hétéroaryle ; ou
R₁₈ et R₁₉ forment ensemble avec l'atome auquel ils sont liés, un cycle saturé à 5 à 7 chaînons, qui peut contenir un ou deux hétéroatomes choisis parmi l'azote, l'oxygène et le soufre ;
G₉ et G₁₀ représentent indépendamment l'un de l'autre un hydrogène, -C(X₁)R₂₀, -C(X₂)-X₃-R₂₁, -C(X₄)-N(R₂₂)-R₂₃, -SO₂-R₂₄, un cation de métal alcalin, de métal alcalino-terreux, de sulfonium ou d'ammonium, -P(X₅) (R₂₅)-R₂₆ ou -CH₂-X₆-R₂₇ ;
X₁, X₂, X₃, X₄, X₅ et X₆ représentent indépendamment l'un de l'autre un oxygène ou un soufre ;
R₂₀, R₂₁, R₂₂ et R₂₃ représentent indépendamment l'un de l'autre un hydrogène, alkyle en C₁ à C₁₀, alcényle en C₂ à C₁₀, alcynyle en C₂ à C₁₀, halogénoalkyle en C₁ à C₁₀, cyanoalkyle en C₁ à C₁₀, nitroalkyle en C₁ à C₁₀, aminoalkyle en C₁ à C₁₀, (alkylamino en C₁ à C₅) - (alkyle en C₁ à C₅), (dialkylamino en C₂ à C₈) - (alkyle en C₁ à C₅), (cycloalkyle en C₃ à C₇) - (alkyle en C₁ à C₅), alcoxyalkyle en C₂ à C₁₀, alcényloxyalkyle en C₄ à C₁₀, alcynyloxyalkyle en C₄ à C₁₀, alkylthioalkyle en C₂ à C₁₀, (alkylsulfoxyle en C₁ à C₅)-(alkyle en C₁ à C₅), (alkylsulfonyle en C₁ à C₅) - (alkyle en C₁ à C₅₎, (alkylidèneamino-oxy en C₂ à C₈)-(alkyle en C₁ à C₅), (alkyle en C₁ à C₅)carbonyl-(alkyle en C₁ à C₅), (alcoxy en C₁ à C₅) carbonyl-(alkyle en C₁ à C₅), (alkylamino en C₁ à C₅) carbonyle-(alkyle en C₁ à C₅), (dialkylamino en C₂ à C₈)-carbonyl-(alkyle en C₁ à C₅), (alkyle en C₁ à C₅)carbonylamino-(alkyle en C₁ à C₅), (alkyle en C₁ à C₅) carbonyl- (alkyle en C₂ à C₅)-aminoalkyle, (trialkylsilyle en C₃ à C₆)-(alkyle en C₁ à C₅), phényl- (alkyle en C₁ à C₅), hétéroaryl- (alkyle en C₁ à C₅), phénoxy-(alkyle en C₁ à C₅), hétéroaryloxy-(alkyle en C₁ à C₅), alcényle en C₂ à C₅, halogénoalcényle en C₂ à C₅, cycloalkyle en C₃ à C₈, phényle ou phényle substitué par un alkyle en C₁ à C₃, halogénoalkyle en C₁ à C₃, alcoxy en C₁ à C₃, halogénoalcoxy en C₁ à C₃, halogène, cyano ou nitro, ou hétéroaryle ou hétéroarylamino, ou hétéroaryle ou hétéroarylamino substitué par un alkyle en C₁ à C₃, halogéno-alkyle en C₁ à C₃, alcoxy en C₁ à C₃, halogénoalcoxy en C₁ à C₃, halogène, cyano ou nitro, dihétéroarylamino, ou dihétéroarylamino substitué par un alkyle en C₁ à C₃, halogénoalkyle en C₁ à C₃, alcoxy en C₁ à C₃, halogénoalcoxy en C₁ à C₃, halogène, cyano ou nitro, phénylamino, ou phénylamino substitué par un alkyle en C₁ à C₃, halogénoalkyle en C₁ à C₃, alcoxy en C₁ à C₃, halogénoalcoxy en C₁ à C₃, halogène, cyano ou nitro, diphénylamino ou diphénylamino substitué par un alkyle en C₁ à C₃, halogénoalkyle en C₁ à C₃, alcoxy en C₁ à C₃, halogénoalcoxy en C₁ à C₃, halogène, cyano ou nitro, ou cycloalkylamino en C₃ à C₇, di-(cycloalkylamino en C₃ à C₇) ou cycloalcoxy en C₃ à C₇ ;
R₂₄, R₂₅ et R₂₆ représentent un hydrogène, alkyle en C₁ à C₁₀, alcényle en C₂ à C₁₀, alcynyle en C₂ à C₁₀, halogénoalkyle en C₁ à C₁₀, cyanoalkyle en C₁ à C₁₀, nitroalkyle en C₁ à C₁₀, aminoalkyle en C₁ à C₁₀, (alkylamino en C₁ à C₅) - (alkyle en C₁ à C₅), (dialkylamino en C₂ à C₈) - (alkyle en C₁ à C₅), (cycloalkyle en C₃ à C₇) - (alkyle en C₁ à C₅), alcoxyalkyle en C₂ à C₁₀, alcényloxyalkyle en C₄ à C₁₀, alcynyloxyalkyle en C₄ à C₁₀, alkylthioalkyle en C₂ à C₁₀, (alkylsulfoxyle en C₁ à C₅)- (alkyle en C₁ à C₅), (alkylsulfonyle en C₁ à C₅) - (alkyle en C₁ à C₅), (alkylidèneamino-oxy en C₂ à C₈) - (alkyle en C₁ à C₅), (alkyle en C₁ à C₅) carbonyl-(alkyle en C₁ à C₅), (alcoxy en C₁ à C₅)carbonyl-(alkyle en C₁ à C₅), (aminocarbonyle en C₁ à C₅)-(alkyle en C₁ à C₅), (dialkylamino en C₂ à C₈)-carbonyl-(alkyle en C₁ à C₅), (alkyle en C₁ à C₅)carbonylamino-(alkyle en C₁ à C₅), (alkyle en C₁ à C₅) carbonyl-(alkyle en C₂ à C₅) -aminoalkyle, (trialkylsilyle en C₃ à C₆) - (alkyle en C₁ à C₅), phényl- (alkyle en C₁ à C₅), hétéroaryl- (alkyle en C₁ à C₅), phénoxy-(alkyle en C₁ à C₅), hétéroaryloxy- (alkyle en C₁ à C₅), alcényle en C₂ à C₅, halogénoalcényle en C₂ à C₅, cycloalkyle en C₃ à C₈, phényle, ou phényle substitué par un alkyle en C₁ à C₃, halogénoalkyle en C₁ à C₃, alcoxy en C₁ à C₃, halogénoalcoxy en C₁ à C₃, halogène, cyano ou nitro, ou hétéroaryle ou hétéroarylamino, ou hétéroaryle ou hétéroarylamino substitué par un alkyle en C₁ à C₃, halogénoalkyle en C₁ à C₃, alcoxy en C₁ à C₃, halogénoalcoxy en C₁ à C₃, halogène, cyano ou nitro, dihétéroarylamino ou dihétéroarylamino substitué par un alkyle en C₁ à C₃, halogénoalkyle en C₁ à C₃, alcoxy en C₁ à C₃, halogénoalcoxy en C₁ à C₃, halogène, cyano ou nitro, phénylamino ou phénylamino substitué par un alkyle en C₁ à C₃, halogénoalkyle en C₁ à C₃, alcoxy en C₁ à C₃, halogénoalcoxy en C₁ à C₃, halogène, cyano ou nitro, diphénylamino ou diphénylamino substitué par un alkyle en C₁ à C₃, halogénoalkyle en C₁ à C₃, alcoxy en C₁ à C₃, halogénoalcoxy en C₁ à C₃, halogène, cyano ou nitro, ou cycloalkylamino en C₃ à C₇, di-(cycloalkylamino en C₃ à C₇), cycloalcoxy en C₃ à C₇, alcoxy en C₁ à C₁₀, halogénoalcoxy en C₁ à C₁₀, alkylamino en C₁ à C₅, dialkylamino en C₂ à C₈, benzyloxy ou phénoxy, les groupes benzyle et phényle pouvant être substitués par un alkyle en C₁ à C₃, halogénoalkyle en C₁ à C₃, alcoxy en C₁ à C₃, halogénoalcoxy en C₁ à C₃, halogène, cyano ou nitro ;
R₂₇ représente un alkyle en C₁ à C₁₀, alcényle en C₂ à C₁₀, alcynyle en C₂ à C₁₀, halogénoalkyle en C₁ à C₁₀, cyanoalkyle en C₁ à C₁₀, nitroalkyle en C₁ à C₁₀, aminoalkyle en C₁ à C₁₀, (alkylamino en C₁ à C₅)-(alkyle en C₁ à C₅), (dialkylamino en C₂ à C₈)-(alkyle en C₁ à C₅), (cycloalkyle en C₃ à C₇)-(alkyle en C₁ à C₅), alcoxyalkyle en C₂ à C₁₀, alcényloxyalkyle en C₄ à C₁₀, alcynyloxyalkyle en C₄ à C₁₀, alkylthioalkyle en C₂ à C₁₀, (alkylsulfoxyle en C₁ à C₅) - (alkyle en C₁ à C₅), (alkylsulfonyle en C₁ à C₅) - (alkyle en C₁ à C₅), (alkylidèneamino-oxy en C₂ à C₈)-(alkyle en C₁ à C₅), (alkyle en C₁ à C₅)carbonyl-(alkyle en C₁ à C₅), (alcoxy en C₁ à C₅)carbonyl- (alkyle en C₁ à C₅), (alkylamino en C₁ à C₅)carbonyle-(alkyle en C₁ à C₅), (dialkylamino en C₂ à C₈) -carbonyl- (alkyle en C₁ à C₅), (alkyle en C₁ à C₅)carbonylamino-(alkyle en C₁ à C₅), (alkyle en C₁ à C₅)carbonyl- (alkyle en C₂ à C₅)-aminoalkyle, (trialkylsilyle en C₃ à C₆) - (alkyle en C₁ à C₅), phényl- (alkyle en C₁ à C₅), hétéroaryl- (alkyle en C₁ à C₅), phénoxy-(alkyle en C₁ à C₅), hétéroaryloxy- (alkyle en C₁ à C₅), alcényle en C₂ à C₅, halogénoalcényle en C₂ à C₅, cycloalkyle en C₃ à C₈, phényle, ou phényle substitué par un alkyle en C₁ à C₃, halogénoalkyle en C₁ à C₃, alcoxy en C₁ à C₃, halogénoalcoxy en C₁ à C₃, halogène, cyano ou nitro, hétéroaryle ou hétéroarylamino, ou hététoaryle ou hétéroarylamino substitué par un alkyle en C₁ à C₃, halogénoalkyle en C₁ à C₃, alcoxy en C₁ à C₃, halogénoalcoxy en C₁ à C₃, halogène, cyano ou nitro, dihétéroarylamino, dihétéroarylamino substitué par un alkyle en C₁ à C₃, halogénoalkyle en C₁ à C₃, alcoxy en C₁ à C₃, halogénoalcoxy en C₁ à C₃, halogène, cyano ou nitro, phénylamino, phénylamino substitué par un alkyle en C₁ à C₃, halogénoalkyle en C₁ à C₃, alcoxy en C₁ à C₃, halogénoalcoxy en C₁ à C₃, halogène, cyano ou nitro, diphénylamino, diphénylamino substitué par un alkyle en C₁ à C₃, halogénoalkyle en C₁ à C₃, alcoxy en C₁ à C₃, halogénoalcoxy en C₁ à C₃, halogène, cyano ou nitro, cycloalkylamino en C₃ à C₇, di-(cycloalkylamino en C₃ à C₇), cycloalcoxy en C₃ à C₇ ou (alkyle en C₁ à C₁₀) carbonyle ;
Y₂ représente un oxygène ou un soufre,
R₅₅ représente un alkyle en C₁ à C₁₀, alcényle en C₂ à C₁₀, alcynyle en C₂ à C₁₀, halogénoalkyle en C₁ à C₁₀, alcoxyalkyle en C₂ à C₁₀, alcényloxyalkyle en C₃ à C₁₀ alcynyloxyalkyle en C₃ à C₁₀, alkylthioalkyle en C₂ à C₁₀, alkylsulfinylalkyle en C₂ à C₁₀, alkylsulfonylalkyle en C₂ à C₁₀, alkylcarbonylalkyle en C₂ à C₁₀, cycloalkyle en C₃ à C₁₀, aryle ou hétéroaryle ;
R₁₃₇ représente un hydrogène, alkyle en C₁ à C₁₀, alcényle en C₂ à C₁₀, alcynyle en C₂ à C₁₀ ou (alcoxy en C₁ à C₁₀) alkyle ; ou
R₅₅ et R₁₃₇ forment ensemble, avec les atomes auxquels ils sont liés, un cycle à 3 à 7 chaînons, qui peut contenir un ou deux hétéroatomes choisis parmi l'azote, l'oxygène et le soufre ; et
R₁₃₈ et R₁₃₉ sont indépendamment l'un de l'autre un hydrogène, alkyle en C₁ à C₁₀, alcényle en C₂ à C₁₀, alcynyle en C₂ à C₁₀, alcoxyalkyle en C₂ à C₁₀;
et le composé n° 21.115,
ainsi que des sels, des isomères et énantiomères agronomiquement acceptables de ces composés.

2. Procédé pour la préparation de composés de formule I selon la revendication 1, **caractérisé en ce que** l'on met à réagir un composé de formule XXX
Q-H (XXX)
dans laquelle Q représente Q₉ ou Q₁₀, dans lesquels leurs substituants à l'exception de G₉ et de G₁₀ ont la signification indiquée ci-dessus et G₉ et G₁₀ représentent un hydrogène, avec un composé de formule XXXI 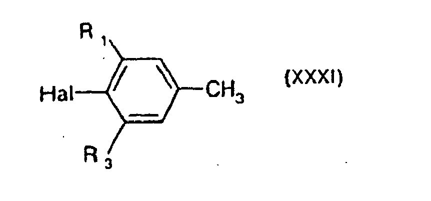 dans laquelle R₁ et R₃ ont la signification indiquée à la formule I et Hal représente le chlore, le brome ou l'iode, en présence d'un solvant inerte, d'une base et d'un catalyseur de palladium à des températures de 30 à 250 °C.

3. Agent herbicide et inhibant la croissance végétale, **caractérisé en ce qu'**il présente sur un support inerte une teneur herbicide efficace en composé de formule I.

4. Procédé pour la lutte contre la croissance végétale non souhaitée, **caractérisé en ce que** l'on applique une substance active de formule I, ou un agent contenant cette substance active en une quantité herbicide efficace sur les plantes ou leur espace vital.

5. Procédé pour l'inhibition de la croissance , végétale, **caractérisé en ce que** l'on applique une substance active de formule I, ou un agent contenant cette substance active en une quantité herbicide efficace sur les plantes ou leur espace vital.

6. Agent herbicide sélectif, **caractérisé en ce qu'**il contient en plus d'adjuvants de formulation inertes usuels comme substance active, un mélange de
a) une quantité herbicide efficace d'un composé de formule I selon la revendication 1 et
b) une quantité efficace comme antagoniste d'herbicide soit d'un composé de formule X 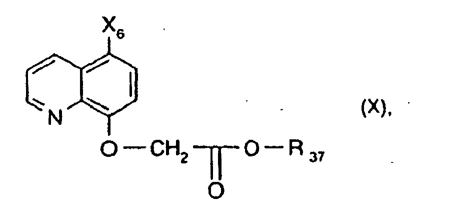
dans laquelle
R₃₇ représente un hydrogène, alkyle en C₁ à C₈ ou alkyle en C₁ à C₈ substitué par un alcoxy en C₁ à C₆ ou alcényloxy en C₃ à C₆ ; et X₆ représente un hydrogène
ou un chlore ; ou d'un composé de formule XI 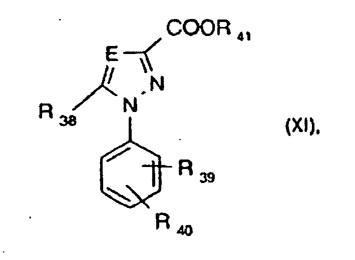 dans laquelle
E représente un azote ou méthyne ;
R₃₈ représente -CCl₃, un phényle ou phényle substitué par un halogène ;
R₃₉ et R₄₀ représentent indépendamment l'un de l'autre un hydrogène ou halogène ; et
R₄₁ représente un alkyle en C₁ à C₄ ; ou d'un composé de formule XII 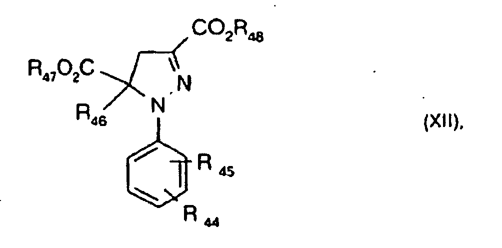 dans laquelle
R₄₄ et R₄₅ représentent indépendamment l'un de l'autre un hydrogène ou halogène et
R₄₆, R₄₇ et R₄₈ représentent indépendamment l'un de l'autre un alkyle en C₁ à C₄, ou d'un composé de formule XIII 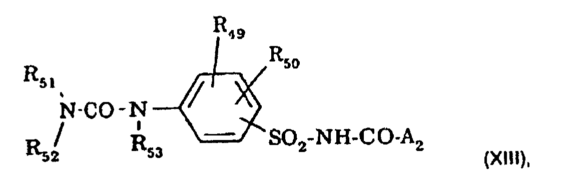 dans laquelle A₂ représente un groupe 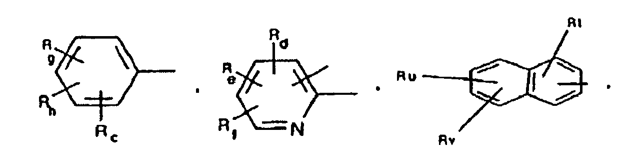 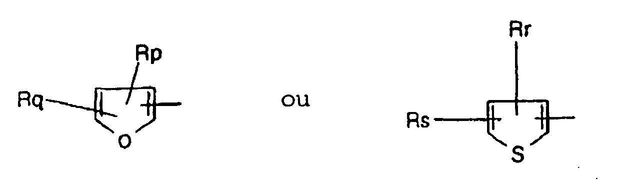 R₅₁ et R₅₂ représentent indépendamment l'un de l'autre un hydrogène, alkyle en C₁ à C₈, cycloalkyle en C₃ à C₈, alcényle en C₃ à C₆, alcynyle en C₃ à C₆, 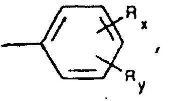 ou alkyle en C₁ à C₄ substitué par un alcoxy en C₁ à C₄ ou 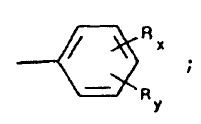 ou R₅₁ et R₅₂ forment ensemble un pont alkylène en C₄ à C₆, qui peut être interrompu par un oxygène, soufre, SO, SO₂, NH ou -N(alkyle en C₁ à C₄), R₅₃ représente un hydrogène ou alkyle en C₁ à C₄ ;
R₄₉ représente un hydrogène, halogène, cyano, trifluorométhyle, nitro, alkyle en C₁ à C₄, alcoxy en C₁ à C₄, alkylthio en C₁ à C₄, alkylsulfinyle en C₁ à C₄, alkylsulfonyle en C₁ à C₄, -COORⱼ, -CONRₖRₘ, -CORₙ, -SO₂NRₖRₘ ou -OSO₂- (alkyle en C₁ à C₄);
R_{g} représente un hydrogène, halogène, cyano, nitro, alkyle en C₁ à C₄, halogénoalkyle en C₁ à C₄, alkylthio en C₁ à C₄, alkylsulfinyle en C₁ à C₄, alkylsulfonyle en C₁ à C₄, -COORⱼ, -CONRₖRₘ, -CORₙ, -SO₂NRₖRₘ, -OSO₂- (alkyle en C₁ à C₄), alcoxy en C₁ à C₆, ou alcoxy en C₁ à C₆ substitué par un alcoxy en C₁ à C₄ ou un halogène, alcényloxy en C₃ à C₆, ou alcényloxy en C₃ à C₆ substitué par un halogène, ou alcynyloxy en C₃ à C₆, ou R₄₉ et R₅₀ forment ensemble un pont alkylène en C₃ à C₄, qui peut être substitué par un halogène ou un alkyle en C₁ à C₄, ou forment un pont alcénylène en C₃ à C₄, qui peut être substitué par un halogène ou un alkyle en C₁ à C₄, ou forment un pont alcadiénylène en C₄, qui peut être substitué par un halogène ou alkyle en C₁ à C₄ ;
R₅₀ et Rₕ représentent indépendamment l'un de l'autre un hydrogène, halogène, alkyle en C₁ à C₄, trifluorométhyle, alcoxy en C₁ à C₆, alkylthio en C₁ à C₆ ou -COORⱼ ;
R_{c} représente un hydrogène, halogène, nitro, alkyle en C₁ à C₄ ou méthoxy ; R_{d} représente un hydrogène, halogène, nitro, alkyle en C₁ à C₄, alcoxy en C₁ à C₄, alkylthio en C₁ à C₄, alkylsulfinyle en C₁ à C₄, alkylsulfonyle en C₁ à C₄, -COORⱼ ou CONRₖRₘ ;
Rₑ représente un hydrogène, halogène, alkyle en C₁ à C₄, -COORⱼ, trifluorométhyle ou méthoxy, ou R_{d} et Rₑ forment ensemble un pont alkylène en C₃ à C₄ ;
Rₚ représente un hydrogène, halogène, alkyle en C₁ à C₄, -COORⱼ, trifluorométhyle ou méthoxy ; R_{q} représente un hydrogène, halogène, nitro, alkyle en C₁ à C₄, alcoxy en C₁ à C₄, alkylthio en C₁ à C₄, alkylsulfinyle en C₁ à C₄ , alkylsulfonyle en C₁ à C₄, -COORⱼ ou CONRₖRₘ ; ou Rₚ et R_{q} forment ensemble un pont alkylène en C₃ à C₄ ;
Rr représente un hydrogène, halogène, alkyle en C₁ à C₄, -COORⱼ, trifluorométhyle ou méthoxy ; Rs représente un hydrogène, halogène, nitro, alkyle en C₁ à C₄, alcoxy en C₁ à C₄, alkylthio en C₁ à C₄, alkylsulfinyle en C₁ à C₄, alkylsulfonyle en C₁ à C₄, -COORⱼ ou CONRₖRₘ ; ou Rr et Rs forment ensemble un pont alkylène en C₃ à C₄ ;
Rt représente un hydrogène, halogène, alkyle en C₁ à C₄, -COORⱼ, trifluorométhyle ou méthoxy ; Ru représente un hydrogène, halogène, nitro, alkyle en C₁ à C₄, alcoxy en C₁ à C₄, alkylthio en C₁ à C₄, alkylsulfinyle en C₁ à C₄, alkylsulfonyle en C₁ à C₄, -COORⱼ ou CONRₖRₘ ; ou Rv et Ru forment ensemble un pont alkylène en C₃ à C₄ ;
R_{f} et Rv représentent ensemble un hydrogène, halogène ou alkyle en C₁ à C₄ ;
Rₓ et R_{y} représentent indépendamment l'un de l'autre un hydrogène, halogène, alkyle en C₁ à C₄, alcoxy en C₁ à C₄, alkylthio en C₁ à C₄, -COOR₅₄, trifluorométhyle, nitro ou cyano ;
Rⱼ, Rₖ et Rₘ représentent indépendamment l'un de l'autre un hydrogène ou alkyle en C₁ à C₄ ; ou
Rₖ et Rₘ forment ensemble un pont alkylène en C₄ à C₆, qui peut être interrompu par un oxygène, NH ou -N (alkyle en C₁ à C₄)-;
Rₙ représente un alkyle en C₁ à C₄, phényle ou phényle substitué par un halogène, alkyle en C₁ à C₄, méthoxy, nitro ou trifluorométhyle ;
R₅₄ représente un hydrogène, alkyle en C₁ à C₁₀, (alcoxy en C₁ à C₄) -(alkyle en C₁ à C₄), (alkylthio en C₁ à C₄)-(alkyle en C₁ à C₄), di-(alkyamino en C₁ à C₄)-(alkyle en C₁ à C₄), halogène- (alkyle en C₁ à C₈), alcényle en C₂ à C₈, halogène- (alcényle en C₂ à C₈), alcynyle en C₃ à C₈, cycloalkyle en C₃ à C₇, halogène-(cycloalkyle en C₃ à C₇), (alkyle en C₁ à C₈) carbonyle, allylcarbonyle, (cycloalkyle en C₃ à C₇)carbonyle, benzoyle, qui est non substitué ou substitué sur le cycle phényle jusqu'à trois fois de manière identique ou différente par un halogène, alkyle en C₁ à C₄, halogéno-(alkyle en C₁ à C₄), halogène- (alcoxy en C₁ à C₄) ou alcoxy en C₁ à C₄ ; ou furoyle, thiényle ; ou alkyle en C₁ à C₄ substitué par un phényle, halogénophényle, (alkyle en C₁ à C₄)phényle, (alcoxy en C₁ à C₄)phényle, halogéno-(alkyle en C₁ à C₄)phényle, halogène-(alcoxy en C₁ à C₄) phényle, (alcoxy en C₁ à C₆)carbonyle, (alcoxy en C₁ à C₄) - (alcoxy en C₁ à C₈) carbonyle, alcényloxycarbonyle en C₃ à C₈, alcynyloxycarbonyle en C₃ à C₈, (alkylthio en C₁ à C₈) carbonyle, alcénylthiocarbonyle en C₃ à C₈, alcynylthiocarbonyle en C₃ à C₈, carbamoyle, mono-(alkylamino en C₁ à C₄) carbonyle, di- (alkylamino en C₁ à C₄) carbonyle ; ou phénylaminocarbonyle, qui est non substitué ou substitué sur le phényle jusqu'à trois fois par des groupes identiques ou différents par un halogène, alkyle en C₁ à C₄, halogène-(alkyle en C₁ à C₄), halogène- (alcoxy en C₁ à C₄) ou alcoxy en C₁ à C₄ ou une fois par un cyano ou nitro, ou dioxolan-2-yle, qui est non substitué ou est substitué par un ou deux radicaux alkyle en C₁ à C₄, ou dioxan-2-yle, qui est non substitué ou est substitué par un ou deux radicaux alkyle en C₁ à C₄, ou alkyle en C₁ à C₄, qui est substitué par un cyano, nitro, carboxyle ou (alkylthio en C₁ à C₈)-(alcoxy en C₁ à C₈) carbonyle ;
ou d'un composé de formule XIV 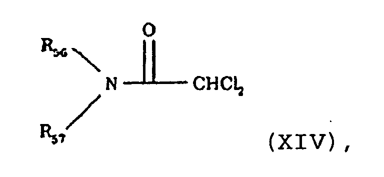 dans laquelle R₅₆ et R₅₇ représentent indépendamment l' un de l'autre un alkyle en C₁ à C₆ ou alcényle en C₂ à C₆ ; ou R₅₆ et R₅₇ représentent ensemble 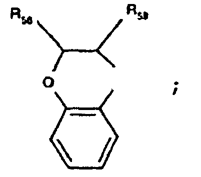 R₅₈ et R₅₉ indépendamment l'un de l'autre représentent un hydrogène ou un alkyle en C₁ à C₆ ; ou R₅₆ et R₅₇ représentent ensemble 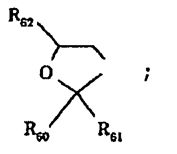 R₆₀ et R₆₁ indépendamment l'un de l'autre représentent un alkyle en C₁ à C₄, ou R₆₀ et R₆₁ représentent ensemble -(CH₂)₅- ;
R₆₂ représente un hydrogène, alkyle en C₁ à C₄ ou 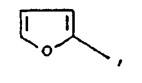 ou R₅₆ et R₅₇ représentent ensemble 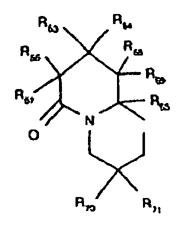 ou 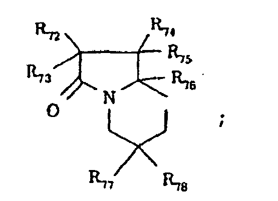 R₆₃, R₆₄, R₆₅, R₆₆, R₆₇, R₆₈, R₆₉, R₇₀, R₇₁, R₇₂, R₇₃, R₇₄, R₇₅, R₇₆, R₇₇ et R₇₈ représentent indépendamment l'un de l'autre un hydrogène ou un alkyle en C₁ à C₄ ;
ou un composé de formule XV 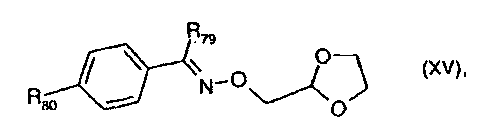 dans laquelle R₈₀ représente un hydrogène ou chlore et R₇₉ représente un cyano ou trifluorométhyle,
ou un composé de formule XVI 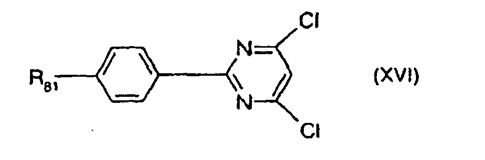 dans laquelle R₈₁ représente un hydrogène ou un méthyle,
ou un composé de formule XVII 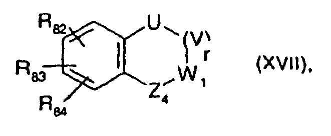 dans laquelle
R₈₂ représente un hydrogène, alkyle en C₁ à C₄ ou alkyle en C₁ à C₄ substitué par un (alkyle en C₁ à C₄) -X₂- ou (halogénoalkyle en C₁ à C₄)-X₂-, ou représente un halogénoalkyle en C₁ à C₄, nitro, cyano, -COOR₈₅, -NR₈₆R₈₇, -SO₂NR₈₈R₈₉ ou -CONR₉₀R₉₁ ;
R₈₃ représente un hydrogène, halogène, alkyle en C₁ à C₄, trifluorométhyle, alcoxy en C₁ à C₄ ou halogénoalcoxy en C₁ à C₄ ;
R₈₄ représente un hydrogène, halogène ou alkyle en C₁ à C₄
U, V, W₁ et Z₄ représentent indépendamment l'un de l'autre un oxygène, soufre, C(R₉₂)R₉₃, carbonyle, NR₉₄, un groupe 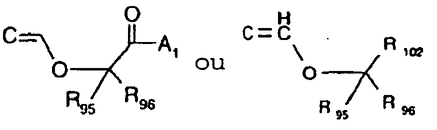 dans lequel
R₁₀₂ représente un alcényle en C₂ à C₄ ou alcynyle en C₂ à C₄ ; sous réserve que
a) au moins un des chaînons du cycle U, V, W₁ ou Z₄ soit un carbonyle, et qu'un chaînon de cycle voisin de ce ou de ces chaînons de cycle représente le groupe 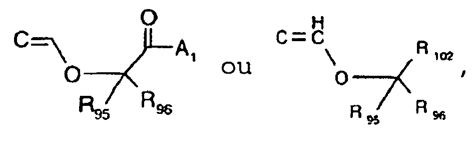 ce groupe apparaissant seulement une fois ; et
b) deux chaînons de cycle voisin U et V, V et W₁ et W₁ et Z₄ ne puissent pas représenter en même temps un oxygène ;
R₉₅ et R₉₆ représentent indépendamment l'un de l'autre un hydrogène ou alkyle en C₁ à C₈ ; ou
R₉₅ et R₉₆ forment ensemble un groupe alkylène en C₂ à C₆ ;
A₁ représente R₉₉-Y₁- ou -NR₉₇R₉₈ ;
X₂ représente un oxygène ou -S(O)ₛ ;
Y₁ représente un oxygène ou soufre ;
R₉₉ représente un hydrogène, alkyle en C₁ à C₈, halogénoalkyle en C₁ à C₈, (alcoxy en C₁ à C₄)-(alkyle en C₁ à C₈), (alcényloxy en C₃ à C₆)-(alkyle en C₁ à C₈) ou phényl-(alkyle en C₁ à C₈), le cycle phényle pouvant être substitué par un halogène, un alkyle en C₁ à C₄, trifluorométhyle, méthoxy ou méthyl-S(O)ₛ-, ou alcényle en C₃ à C₆, halogénoalcényle en C₃ à C₆, phényl-(alcényle en C₃ à C₆), alcynyle en C₃ à C₆, phényl-(alcynyle en C₃ à C₆), oxétanyle, furyle ou tétrahydrofuryle ;
R₈₅ représente un hydrogène ou alkyle en C₁ à C₄ ;
R₈₆ représente un hydrogène, alkyle en C₁ à C₄ ou (alkyle en C₁ à C₄)carbonyle ;
R₈₇ représente un hydrogène ou alkyle en C₁ à C₄ ; ou R₈₆ et R₈₇ forment ensemble un groupe alkylène en C₄ ou C₅ ;
R₈₈, R₈₉, R₉₀ et R₉₁ représentent indépendamment l'un de l'autre un hydrogène ou alkyle en C₁ à C₄ ; ou
R₈₈ ensemble avec R₈₉ ou R₉₀ ensemble avec R₉₁ indépendamment l'un de l'autre sont un alkylène en C₄ ou C₅, un atome de carbone pouvant être remplacé par un oxygène ou un soufre, ou un ou deux atomes de carbone par -NR₁₀₀- ;
R₉₂, R₁₀₀ et R₉₃ sont indépendamment l'un de l'autre un hydrogène ou alkyle en C₁ à C₈ ; ou
R₉₂ et R₉₃ sont ensemble un alkylène en C₂ à C₆ ;
R₉₄ représente un hydrogène ou alkyle en C₁ à C₈ ;
R₉₇ représente un hydrogène, alkyle en C₁ à C₈, phényle ou phényl- (alkyle en C₁ à C₈), dans lequel les cycles phényle peuvent être substitués par un fluor, chlore, brome, nitro, cyano, -OCH₃, alkyle en C₁ à C₄ ou CH₃SO₂-, ou représente un (alcoxy en C₁ à C₄)-(alkyle en C₁ à C₈), alcényle en C₃ à C₆ ou alcynyle en C₃ à C₆ ;
R₉₈ représente un hydrogène, alkyle en C₁ à C₈, alcényle en C₃ à C₆ ou alcynyle en C₃ à C₆ ; ou
R₉₇ et R₉₈ représentent ensemble un alkylène en C₄ ou C₅, un atome de carbone pouvant être remplacé par un oxygène ou un soufre, ou un ou deux atomes de carbone par -NR₁₀₁- ;
R₁₀₁ représente un hydrogène ou alkyle en C₁ à C₄ ;
r est 0 ou 1 ; et
s représente 0, 1 ou 2,
ou un composé de formule XVIII 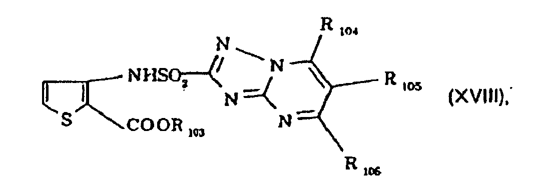 dans laquelle R₁₀₃ représente un hydrogène, alkyle en C₁ à C₆, cycloalkyle en C₃ à C₆, alcényle en C₃ à C₆ ou alcynyle en C₃ à C₆ ; et
R₁₀₄, R₁₀₅ et R₁₀₆ indépendamment l'un de l'autre représentent un hydrogène, alkyle en C₁ à C₆, cycloalkyle en C₃ à C₆ ou alcoxy en C₁ à C₆, sous réserve que l'un des substituants R₁₀₄, R₁₀₅ et R₁₀₆ soit différent d'un hydrogène ;
ou un composé de formule XIX 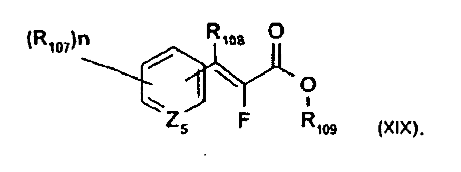 dans laquelle Z₅ représente N ou CH, n dans le cas où Z₅ est égal à N est 0, 1, 2 ou 3 et dans le cas où Z₅ est CH, représente 0, 1, 2, 3 ou 4, R₁₀₇ représente un halogène, alkyle en C₁ à C₄, halogénoalkyle en C₁ à C₄, alcoxy en C₁ à C₄, halogénoalcoxy en C₁ à C₄, nitro, alkylthio en C₁ à C₄, alkylsulfonyle en C₁ à C₄, (alcoxy en C₁ à C₄) carbonyle, phényle ou phénoxy ; ou phényle
ou phénoxy substitué par un alkyle en C₁ à C₃, halogénoalkyle en C₁ à C₃, alcoxy en C₁ à C₃, halogénoalcoxy en C₁ à C₃, halogène, cyano ou nitro ;
R₁₀₈ représente un hydrogène ou alkyle en C₁ à C₄, R₁₀₉ représente un hydrogène, alkyle en C₁ à C₄, cycloalkyle en C₃ à C₆, alcényle en C₂ à C₆, alcynyle en C₂ à C₆, halogénoalkyle en C₁ à C₄, halogénoalcényle en C₂ à C₆, halogénoalcynyle en C₂ à C₆, (alkylthio en C₁ à C₄)-(alkyle en C₁ à C₄), (alkylsulfonyle en C₁ à C₄) - (alkyle en C₁ à C₄), (alcoxy en C₁ à C₄) - (alkyle en C₁ à C₄), (alcényloxy en C₁ à C₄)-(alkyle en C₁ à C₄) ou (alcynyloxy en C₁ à C₄)-(alkyle en C₁ à C₄) ;
ou un composé de formule XX 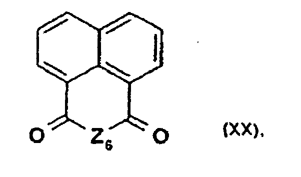 dans laquelle Z₆ représente un oxygène ou N-R₁₁₀ et R₁₁₀ représente un groupe de formule 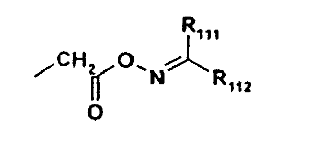 dans laquelle R₁₁₁ et R₁₁₂ indépendamment l'un de l'autre représentent un cyano, hydrogène, alkyle en C₁ à C₄, cycloalkyle en C₃ à C₆, alcényle en C₂ à C₆, aryle, phényle ou hétéroaryle, ou phényle, aryle ou hétéroaryle substitué par un alkyle en C₁ à C₃, halogénoalkyle en C₁ à C₃, alcoxy en C₁ à C₃, halogénoalcoxy en C₁ à C₃, halogène, cyano ou nitro ;
ou un composé de formule XXI 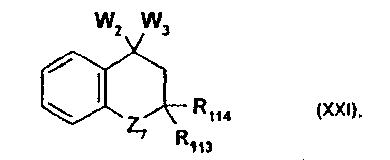 dans laquelle Z₇ représente un oxygène, soufre, S=O, SO₂ ou CH₂, R₁₁₃ et R₁₁₄ représentent indépendamment l'un de l'autre un hydrogène, halogène ou alkyle en C₁ à C₄, W₂ et W₃ représentent indépendamment l'un de l'autre CH₂COOR₁₁₅ ou COOR₀₁₁₅ ou représentent ensemble un groupe de formule - -(CH₂)C(O)-O-C(O)-(CH₂)-, et R₁₁₅ et R₀₁₁₅ représentent indépendamment l'un de l'autre un hydrogène, alkyle en C₁ à C₄, alcényle en C₂ à C₄, alcynyle en C₂ à C₆, cycloalkyle en C₃ à C₆, halogénoalkyle en C₁ à C₄, un cation métallique ou ammonium ;
ou un composé de formule XXII 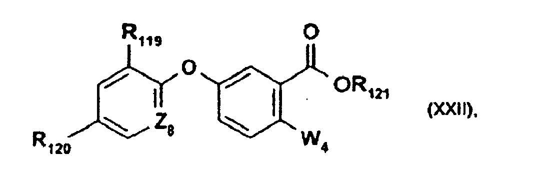 dans laquelle R₁₁₉ et R₁₂₀ représentent indépendamment l'un de l'autre un hydrogène, halogène ou halogéno-alkyle en C₁ à C₄, R₁₂₁ représente un hydrogène, alkyle en C₁ à C₄, alcényle en C₃ à C₄, alcynyle en C₃ à C₄, halogénoalkyle en C₁ à C₄, cycloalkyle en C₃ à C₆, un cation métallique ou un cation ammonium, Z₈ représente N, CH, C-F ou C-Cl et W₄ représente un groupe de formule 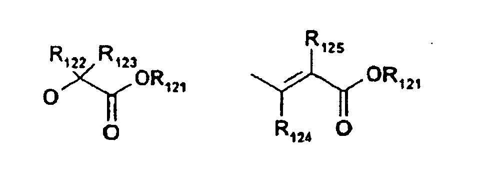 dans laquelle R₁₂₂ et R₁₂₃ représentent indépendamment l'un de l'autre un hydrogène ou alkyle en C₁ à C₄ et R₁₂₄ et R₁₂₅ représentent indépendamment l'un de l'autre un hydrogène ou alkyle en C₁ à C₄;
ou un composé de formule XXIII 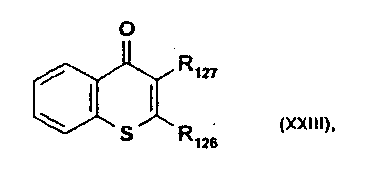 dans laquelle R₁₂₆ représente un hydrogène, cyano, halogène, alkyle en C₁ à C₄, cycloalkyle en C₃ à C₆, alcoxy en C₁ à C₄, (alcoxy en C₁ à C₄)carbonyle, (alkylthio en C₁ à C₄)carbonyle, -NH-R₁₂₈, -C(O)NH-R₀₁₂₈, aryle ou hétéroaryle, ou aryle ou hétéroaryle substitué par un alkyle en C₁ à C₃, halogénoalkyle en C₁ à C₃, alcoxy en C₁ à C₃, halogénoalcoxy en C₁ à C₃, halogène, cyano ou nitro ;
R₁₂₇ représente un hydrogène, cyano, nitro, halogène, alkyle en C₁ à C₄, halogénoalkyle en C₁ à C₄, alcoxy en C₁ à C₄, thioalkyle en C₁ à C₄, et
R₁₂₈ et R₀₁₂₈ représentent indépendamment l'un de l'autre un alkyle en C₁ à C₄, halogénoalkyle en C₁ à C₄, alcényle en C₃ à C₄, alcynyle en C₃ à C₄, cycloalkyle en C₃ à C₄, aryle ou hétéroaryle, ou aryle ou hétéroaryle substitué par un alkyle en C₁ à C₃, halogénoalkyle en C₁ à C₃, alcoxy en C₁ à C₃, halogénoalcoxy en C₁ à C₃, halogène, cyano ou nitro, formyle, (alkyle en C₁ à C₄) carbonyle, alkylsulfonyle en C₁ à C₄ ;
ou un composé de formule XXIV 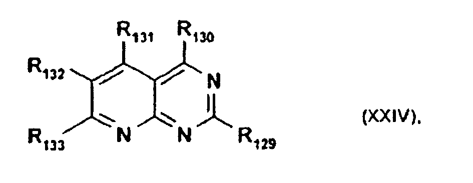 dans laquelle R₁₂₉ et R₁₃₀ représentent indépendamment l'un de l'autre un hydrogène, alkyle en C₁ à C₄, halogénoalkyle en C₁ à C₄, alcoxy en C₁ à C₄, mono-(alkyle en C₁ à C₈) - ou di- (alkyle en C₁ à C₈)-amino, cycloalkyle en C₃ à C₆, thioalkyle en C₁ à C₄, phényle ou hétéroaryle, R₁₃₁ a la signification de R₁₂₉ et est de plus OH, NH₂, un halogène, di-(aminoalkyle en C₁ à C₄), alkylthio en C₁ à C₄, alkylsulfonyle en C₁ à C₄ ou (alcoxy en C₁ à C₄)carbonyle, R₁₃₂ a la signification de R₁₂₉ et représente de plus un cyano, nitro, carboxyle, (alcoxy en C₁ à C₄) carbonyle, di-(aminoalkyle en C₁ à C₄), alkylthio en C₁ à C₄, alkylsulfonyle en C₁ à C₄, SO₂-OH, aminoalkylsulfonyle en C₁ à C₄ ou alcoxysulfonyle en C₁ à C₄, R₁₃₃ a la signification de R₁₂₉ et est de plus OH, NH₂, un halogène, di-(aminoalkyle en C₁ à C₄), pyrrolidin-1-yle, pipéridin-1-yle, morpholin-1-yle, alkylthio en C₁ à C₄, alkylsulfonyle en C₁ à C₄, (alcoxy en C₁ à C₄)carbonyle, phénoxy, naphtoxy, phénylamino, benzoyloxy ou phénylsulfonyloxy ;
ou un composé de formule XXV 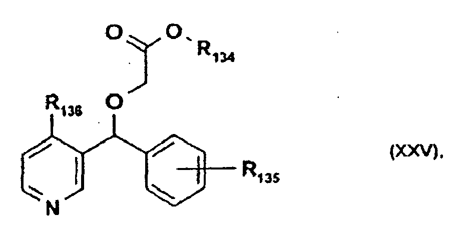 dans laquelle R₁₃₄ représente un hydrogène, alkyle en C₄, halogénoalkyle en C₁ à C₄, alcényle en C₂ à C₄, alcynyle en C₂ à C₄ ou (alcoxy en C₁ à C₄) - (alkyle en C₁ à C₄), R₁₃₅ représente un hydrogène, halogène, alkyle en C₁ à C₄ , halogénoalkyle en C₁ à C₄ ou alcoxy en C₁ à C₄ et R₁₃₆ représente un hydrogène, halogène, alkyle en C₁ à C₄, halogénoalkyle en C₁ à C₄ ou alcoxy en C₁ à C₄, sous réserve que R₁₃₅ et R₁₃₆ ne représentent pas en même temps un hydrogène,
ou de formule XXVI 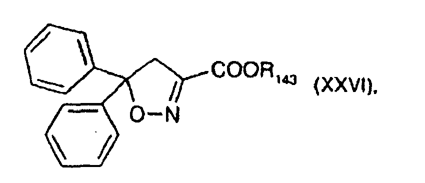 dans laquelle
R₁₄₃ représente un hydrogène, un cation de métal alcalin, alcalino-terreux, de sulfonium ou d'ammonium
ou un éthyle ;
ou de formule XXVII 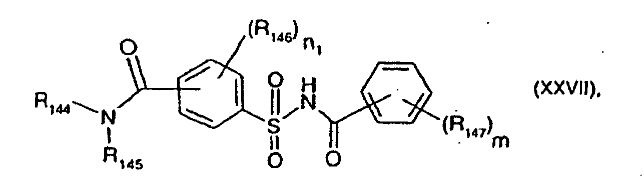 dans laquelle R₁₄₄ et R₁₄₅ représentent indépendamment l'un de l'autre un hydrogène, alkyle en C₁ à C₆, alcényle en C₂ à C₆, alcynyle en C₂ à C₆ ou cycloalkyle en C₃ à C₆ ;
R₁₄₆ représente un hydrogène, halogène, alkyle en C₁ à C₄, halogénoalkyle en C₁ à C₆ ou halogénoalcoxy en C₁ à C₆ ;
R₁₄₇ représente un hydrogène, halogène, alkyle en C₁ à C₄, halogénoalkyle en C₁ à C₄, alcoxy en C₁ à C₄, halogénoalcoxy en C₁ à C₄, alkylthio en C₁ à C₄, (alcoxy en C₁ à C₄)carbonyle ou nitro ;
ni représente 0, 1, 2 ou 3 ; et
m représente 1 ou 2 ;
ou de formule XXVIII 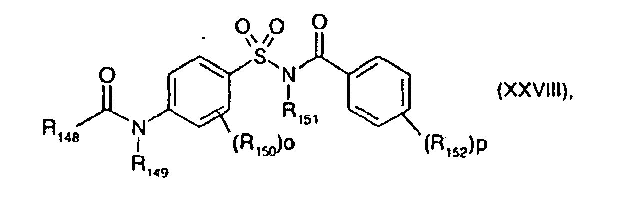 dans laquelle
R₁₄₈ représente un hydrogène, alkyle en C₁ à C₆, alcoxy en C₁ à C₆, alkylthio en C₁ à C₆, cycloalkyle en C₃ à C₈, phényle, phényl- (alkyle en C₁ à C₆) ou hétéroaryle ; les groupes indiqués pouvant être substitués par un halogène, cyano, nitro, amino, hydroxy, carbonyle, carboxyle, formyle, carbonamide ou sulfonamide ;
R₁₄₉ représente un hydrogène, alkyle en C₁ à C₆ ou halogénoalkyle en C₁ à C₄ ;
chaque R₁₅₀ représente indépendamment un hydrogène, halogène, alkyle en C₁ à C₄, halogénoalkyle en C₁ à C₄, alcoxy en C₁ à C₄, alkylthio en C₁ à C₄, alkylsulfonyle en C₁ à C₄, cyano, nitro, formyle ou carboxyle ;
R₁₅₁ représente un hydrogène, alkyle en C₁ à C₆ ou halogénoalkyle en C₁ à C₄ ;
chaque R₁₅₂ représente indépendamment un hydrogène, halogène, alkyle en C₁ à C₄, halogénoalkyle en C₁ à C₄, alcoxy en C₁ à C₄, alkylthio en C₁ à C₄, alkylsulfonyle en C₁ à C₄, cyano, nitro, formyle ou carboxyle ;
O représente 0, 1 ou 2, et
p représente 0, 1 ou 2 ;
ou de formule XXIX 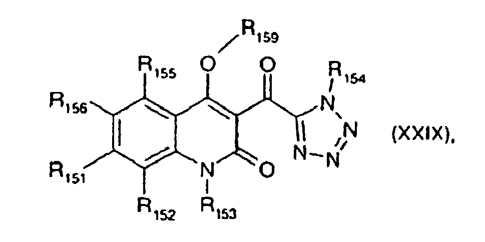 dans laquelle
R₁₅₉ représente un hydrogène, formyle, (alkyle en C₁ à C₆)carbonyle, (alcényle en C₁ à C₆)carbonyle, (alcynyle en C₁ à C₆) carbonyle, (alcoxy en C₁ à C₆)carbonyle, (alkylthio en C₁ à C₆)carbonyle, (cycloalkyle en C₃ à C₈) carbonyle, phényl- (alkyle en C₁ à C₆) carbonyle, phénylcarbonyle, alkylsulfonyle en C₁ à C₆, alcénylsulfonyle en C₁ à C₆ ou phénylsulfonyle, les groupes hydrocarbonés précédemment indiqués pouvant être substitués par un ou plusieurs atomes d'halogène, un cyano, nitro, amino, méthoxy, éthoxy ou phényle ;
R₁₅₃ représente un hydrogène, alkyle en C₁ à C₆, alcényle en C₁ à C₆, alcynyle en C₁ à C₆, cycloalkyle en C₃ à C₈, formyle, (alkyle en C₁ à C₆) carbonyle, (alcényle en C₁ à C₆) carbonyle, (alcynyle en C₁ à C₆) carbonyle, (alcoxy en C₁ à C₆) carbonyle, (alkylthio en C₁ à C₆) carbonyle, (cycloalkyle en C₃ à C₈) carbonyle, alkylsulfonyle en C₁ à C₆, alcénylsulfonyle en C₁ à C₆ ou phénylsulfonyle, les groupes hydrocarbonés précédemment indiqués pouvant être substitués par un ou plusieurs atomes d'halogène, cyano, nitro, amino, méthoxy, éthoxy ou phényle ;
R₁₅₄ représente un hydrogène, alkyle en C₁ à C₆, alcényle en C₁ à C₆, alcynyle en C₁ à C₆, cycloalkyle en C₃ à C₈, formyle, (alkyle en C₁ à C₆)carbonyle, (alcényle en C₁ à C₆)carbonyle, (alcynyle en C₁ à C₆)-carbonyle, (alcoxy en C₁ à C₆)carbonyle, (alkylthio en C₁ à C₆)carbonyle, (cycloalkyle en C₃ à C₈)carbonyle, alkylsulfonyle en C₁ à C₆, alcénylsulfonyle en C₁ à C₆ ou phénylsulfonyle, les groupes hydrocarbonés mentionnés précédemment pouvant être substitués par un ou plusieurs atomes d'halogène, cyano, nitro, amino, méthoxy, éthoxy ou phényle ;
R₁₅₅, R₁₅₆, R₁₅₇ et R₁₅₈ représentent indépendamment l'un de l'autre un hydrogène, halogène, amino, alkylamino en C₁ à C₃, dialkylamino en C₁ à C₆, hydroxy, cyano, nitro, formyle, carboxyle, alcoxy en C₁ à C₆, halogénoalcoxy en C₁ à C₆, (alkyle en C₁ à C₆) carbonyle, (alcoxy en C₁ à C₆)carboxyle, alkyle en C₁ à C₆, halogénoalkyle en C₁ à C₆, alcényle en C₁ à C₆ ou alcynyle en C₁ à C₆ ;
ou R₁₅₃ et R₁₅₈ forment ensemble avec les atomes de cycle auxquels ils sont liés un cycle à cinq ou six chaînons partiellement saturé ou insaturé, qui peut contenir jusqu'à 2 hétéroatomes identiques ou différents choisis dans le groupe de l'oxygène, le soufre et l'azote, ce cycle pouvant être substitué par un radical oxo.

7. Agent selon la revendication 6, **caractérisé en ce qu'**il contient une quantité efficace antagoniste d'herbicide d'un antidote de formule X, XI, XII, XIII, XIV, XV, XVI, XVII, XVIII, XIX, XX, XXI, XXII, XXIII, XXIV ou XXV.

8. Procédé pour la lutte sélective contre les mauvaises herbes et les graminées dans les cultures de plantes utiles, **caractérisé en ce que** l'on traite les plantes utiles, leurs semences ou plants ou leur surface cultivée avec une quantité herbicide efficace d'un herbicide de formule I et une quantité efficace antagoniste d'herbicide d'un antidote de formule X, XI, XII, XIII, XIV, XV, XVI, XVII, XVIII, XIX, XX, XXI, XXII, XXIII, XXIV, XXV, XXVI, XVII, XVIII ou XXIX.

9. Procédé selon la revendication 8, **caractérisé en ce que** l'on traite les plantes utiles, leurs semences ou plants ou leur surface cultivée avec une quantité efficace antagoniste d'herbicide d'un antidote de formule X, XI, XII, XIII, XIV, XV, XVI, XVII, XVIII, XIX, XX, XXI, XXII, XXIII, XXIV ou XXV.

10. Agent selon la revendication 3, **caractérisé en ce qu'**il contient des adjuvants de citerne de pulvérisation.

11. Agent selon la revendication 6, **caractérisé en ce qu'**il contient des adjuvants de citerne de pulvérisation.
